(19) Europäisches Patentamt
European Patent Office
Office européen des brevets

(11) **EP 4 257 166 A1**

(12) **EUROPEAN PATENT APPLICATION**
published in accordance with Art. 153(4) EPC

(43) Date of publication:
**11.10.2023 Bulletin 2023/41**

(21) Application number: **21900467.8**

(22) Date of filing: **24.11.2021**

(51) International Patent Classification (IPC):
*A61M 5/20* (2006.01)       *A61M 5/24* (2006.01)
*A61M 5/28* (2006.01)

(52) Cooperative Patent Classification (CPC):
**A61M 5/20; A61M 5/24; A61M 5/28**

(86) International application number:
**PCT/JP2021/043006**

(87) International publication number:
**WO 2022/118712 (09.06.2022 Gazette 2022/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2020 JP 2020199996**

(71) Applicant: **PHC Holdings Corporation**
**Tokyo 105-8433 (JP)**

(72) Inventors:
• **ENDO Yuri**
  **Ehime 791-0395 (JP)**
• **KATOH Hisanori**
  **Ehime 791-0395 (JP)**
• **HATA Shinsuke**
  **Ehime 791-0395 (JP)**
• **SHIRAKAWA Takashi**
  **Ehime 791-0395 (JP)**
• **OKAMURA Kazumasa**
  **Ehime 791-0395 (JP)**
• **SUZUKI Takuya**
  **Ehime 791-0395 (JP)**
• **MATSUMOTO Tadashi**
  **Ehime 791-0395 (JP)**
• **OMOTE Kazuho**
  **Ehime 791-0395 (JP)**
• **TANI Ryosuke**
  **Ehime 791-0395 (JP)**
• **SHINOHARA Noriyuki**
  **Ehime 791-0395 (JP)**

(74) Representative: **Eisenführ Speiser**
**Patentanwälte Rechtsanwälte PartGmbB**
**Johannes-Brahms-Platz 1**
**20355 Hamburg (DE)**

(54) **DRUG CARTRIDGE, CASSETTE, DRUG INJECTION DEVICE, AND DRUG INJECTION SYSTEM**

(57)     A drug cartridge includes: a cylinder **11** having a cylindrical cylinder columnar space extending in a longitudinal direction; a gasket **13** supported in the space so that the gasket is movable in the longitudinal direction; a drug **14** held in the space and at least including a liquid first component; and a first temperature sensor and an RF tag **16** arranged on a side surface of the cylinder. The RF tag **16** stores drug information that includes at least information indicating a type of the drug, and wirelessly transmits at least the information indicating the type of the drug and first temperature information indicating a temperature detected by the first temperature sensor in response to an instruction from outside.

FIG.7A

EP 4 257 166 A1

**Description**

### TECHNICAL FIELD

The present application relates to a drug cartridge for medical use, a cassette housing the same, a drug injection device, and a drug injection system.

### BACKGROUND ART

Patients suffering from certain diseases may be prescribed, for example, to inject a drug such as insulin or growth hormone several times a day. In order for patients to inject such a drug by themselves (also called self-injection), various drug injection devices have been put into practical use, as disclosed in Patent Document 1, etc.

### CITATION LIST

### PATENT LITERATURE

Patent Document No. 1: Japanese National Phase PCT Laid-open Publication No. 2014-516634

### SUMMARY OF INVENTION

### TECHNICAL PROBLEM

A drug cartridge may contain a quantity of drug that is injected in multiple doses depending on the specifications or the prescription. In this case, it is preferred to properly manage the drug. Performing multiple injections each day may be bothersome to the patient from various perspectives. In view of this, the present application provides a drug cartridge, a cassette, a drug injection device, and a drug injection system, with which it is possible to properly manage the drug and the injection, or reduce the burden on the operator.

### SOLUTION TO PROBLEM

A drug cartridge according to an embodiment of the present disclosure includes: a cylinder having a cylindrical cylinder columnar space extending in a longitudinal direction; a gasket supported in the space so that the gasket is movable in the longitudinal direction; a drug held in the space and at least including a liquid first component; and a first temperature sensor and an RF tag arranged on a side surface of the cylinder, wherein the RF tag stores drug information that includes at least information indicating a type of the drug, and wirelessly transmits at least the information indicating the type of the drug and first temperature information indicating a temperature detected by the first temperature sensor in response to an instruction from outside.

A drug injection device according to an embodiment of the present disclosure includes: a device housing having a housing space that houses at least a portion of a cassette that houses a drug cartridge having a first temperature sensor and an RF tag, and a housing opening that communicates with the housing space; a piston supported so that the piston can move into the housing space; a motor configured to drive the piston; a motor driver configured to generate a drive signal for driving the motor; an antenna arranged adjacent to the housing space; a transmission/reception circuit configured to transmit electromagnetic waves from the antenna and receive electromagnetic waves received by the antenna; a display device configured to output information regarding an injection operation; and a control device configured to control the motor driver, the transmission/reception circuit and the display device, wherein: with the drug cartridge loaded in the housing space, the control device causes the transmission/reception circuit to receive, via the antenna, drug information including information indicating a type of a drug in the drug cartridge transmitted from an RF tag of the drug cartridge and first temperature information detected by the first temperature sensor, and determines a drive power of the motor based on the first temperature information to control the motor driver so as to output the determined drive power.

### ADVANTAGEOUS EFFECTS OF INVENTION

The present disclosure provides a drug injection device that can be properly managed.

**BRIEF DESCRIPTION OF DRAWINGS**

FIG. **1** is a perspective view showing a drug injection system including a cassette, a drug injection device and a charger.

FIG. **2(a),** FIG. **2(b)** and FIG. **2(c)** are perspective views illustrating how a drug cartridge is housed in the cassette.

FIG. **3(a),** FIG. **3(b)** and FIG. **3(c)** are perspective views illustrating how the cassette is housed in the drug injection device.

FIG. **4(a)** and FIG. **4(b)** are perspective views illustrating the state where injection is performed.

FIG. **5** is a block diagram showing an example configuration of an electrical circuit of the drug injection device.

FIG. **6A** is a view illustrating how the drug injection device is set in a charger.

FIG. **6B** is a front view showing the drug injection device set in the charger.

FIG. **7A** is a cross section parallel to the longitudinal direction of the drug cartridge.

FIG. **7B** is a cross section perpendicular to the longitudinal direction of the drug cartridge.

FIG. **8A** is a schematic plan view of an RF tag.

FIG. **8B** is a schematic plan view of another RF tag.

FIG. **9A** shows a schematic cross section of another drug cartridge.

FIG. **9B** shows a schematic cross section of the other drug cartridge taken along line 9B-9B of FIG. **9A.**

FIG. **10(a)** and FIG. **10(b)** are schematic diagrams illustrating the movement of a liquid component.

FIG. **11** is an exploded perspective view of a hood of the cassette.

FIG. **12(a)** and FIG. **12(b)** are views illustrating how a needle unit is attached to the cassette.

FIG. **13** is an exploded perspective view of the drug injection device with the device housing removed.

FIG. **14** is a perspective view of the drug injection device, showing the arrangement of an RF-ID antenna.

FIG. **15** is an exploded perspective view of a piston drive mechanism.

FIG. **16A** is a schematic diagram showing the positional relationship between the RF tag of the drug cartridge and the antenna in the drug injection device.

FIG. **16B** is a schematic diagram showing the positional relationship between the RF tag of the drug cartridge and the antenna in the drug injection device.

FIG. **17** shows an example of drive information by PWM.

FIG. **18A** shows an example of a starting current that occurs when a PWM control is not performed.

FIG. **18B** shows an example of a starting current by a PWM control of the present embodiment.

FIG. **19A** is a flow chart illustrating the operation of the drug injection device during an injection operation.

FIG. **19B** is a flow chart illustrating the operation of the drug injection device during an injection operation.

FIG. **19C** is a flow chart illustrating a remind operation.

FIG. **20** shows an example of a detection result of a touch sensor.

FIG. **21** is a flow chart illustrating the injection operation of the drug injection device using the touch sensor.

FIG. **22** is a schematic diagram illustrating the orientations of the three axes of an acceleration sensor.

FIG. **23** is a schematic diagram illustrating a portion where it is difficult to remove air during air removal.

FIG. **24** is a schematic diagram illustrating the attitude suitable for air removal.

FIG. **25** is a flow chart illustrating an air-removing operation using an acceleration sensor.

FIG. **26** is a flow chart illustrating a mixing operation.

FIG. **27** is a schematic diagram showing zones used in the mixing operation.

FIG. **28** is a schematic diagram illustrating an air-removing operation.

FIG. **29** is an exploded perspective view showing a portion of a piston drive mechanism including a rotary encoder.

FIG. **30** is a plan view of an encoder plate.

FIG. **31** shows an example of a pulse signal.

FIG. **32** is a block diagram showing an example of a fault detector.

FIG. **33** shows an example image showing information stored in an RF tag.

FIG. **34(a)** to FIG. **34(c)** show example images to be displayed in an air-removing operation.

FIG. **35(a)** and FIG. **35(b)** show example images prompting the operator to maintain the same state until completion of an operation of the drug injection device.

FIG. **36(a)** to FIG. **36(f)** show example images prompting to attach a needle unit and to remove a needle case.

FIG. **37(a)** to FIG. **37(f)** show example images to be displayed during injection.

FIG. **38A** is a front view showing how a drug cartridge is loaded in the drug injection device.

FIG. **38B** is a front view showing how a drug cartridge is loaded in the drug injection device.

FIG. **38C** is a front view showing how a drug cartridge is loaded in the drug injection device.

FIG. **39** is views illustrating how a needle unit is attached to, or a used injection needle is removed from, the drug cartridge loaded in the drug injection device.

FIG. **40** is views illustrating how a needle unit is attached to, or a used injection needle is removed from, the drug cartridge loaded in the drug injection device.

FIG. **41** is views illustrating how a needle unit is attached to, or a used injection needle is removed from, the drug cartridge loaded in the drug injection device.

FIG. **42** is views illustrating how an injection is performed using the drug injection device.

## DESCRIPTION OF EMBODIMENTS

When a drug cartridge contains multiple doses of a drug, it is preferred that the drug cartridge or drug injection device that is in the process of use and still has some drug remaining in it is stored in a cold place, such as a refrigerator, to control drug deterioration. However, when a drug is stored in a cold place, the viscosity of the drug increases due to the low temperature. Therefore, if one takes the drug cartridge or drug injection device out of the cold place and immediately uses it for injection, there is a need to eject the highly viscous drug from a small-diameter injection needle, which places a heavy load on the injection motor, etc., making it difficult to appropriately control the drive of the motor. Moreover, some patients feel pain when a low-temperature drug is injected.

For this reason, it is generally recommended that the drug cartridge or drug injection device be left at room temperature for some tens of minutes after being taken out of the cold place, and that the injection be performed after the temperature of the drug has risen to about room temperature. This could solve the problem described above.

However, it can be said that the wait time before injection is substantially part of the time required for a single injection, and it is therefore preferred for the patient that the wait time be short. Moreover, it is preferred that the pain of injection be small. On the other hand, it is preferred that the drug injection device be appropriately controlled in accordance with the viscosity of the drug.

In view of such problems, the present inventors have conceived of a drug cartridge, a cassette, a drug injection device, and a drug injection system that can be properly managed and reduce the burden on the operator. The drug cartridge, the cassette, the drug injection device and the drug injection system of the present disclosure are as follows.

[Item 1]A drug cartridge including:

a cylinder having a cylindrical cylinder columnar space extending in a longitudinal direction;
a gasket supported in the cylinder columnar space so that the gasket is movable in the longitudinal direction;
a drug held in the cylinder columnar space and at least including a liquid first component; and
a first temperature sensor and an RF tag arranged on a side surface of the cylinder, wherein:
the RF tag stores drug information that includes at least information indicating a type of the drug, and wirelessly transmits at least the information indicating the type of the drug and first temperature information indicating a temperature detected by the first temperature sensor in response to an instruction from outside.

[Item 2] The drug cartridge according to item 1, wherein the RF tag is of a passive type.

[Item 3] The drug cartridge according to item 1, wherein the RF tag further stores at least one of information indicating an expiration date of the drug and information indicating an initial amount of the drug.

[Item 4] A cassette housing a drug cartridge that is loaded into a drug injection device, the cassette including:

a cassette body having a cassette columnar space capable of housing at least a portion of the drug cartridge, an injection needle attachment portion that is located at a distal end of the cassette columnar space and to/from which an injection needle can be attached/detached, and a body opening located at a rear end of the cassette columnar space and accessible to the cassette columnar space;
a cassette cap supported in the vicinity of the rear end of the cassette body so as to open/close the body opening; and
a hood including a hood body supported by the cassette body so that the hood body can pivot between a first position at which the injection needle attachment portion of the cassette body is covered and a second position at which the injection needle attachment portion is exposed, a needle covering supported by the hood body so that the needle covering can move between a projecting position at which the needle covering projects relative to the hood body and a stowed position at which the needle covering is at least partially stowed, and a biasing member configured to bias the needle covering in a direction of the projecting position.

[Item 5] The cassette according to item 4, wherein a portion of the needle covering is transparent.

[Item 6] The cassette according to item 5, wherein the needle covering includes a transparent first portion and a semi-transparent second portion.

[Item 7] The cassette according to any one of items 4 to 6, wherein the hood includes an arm that extends on an opposite side of the hood body relative to a fulcrum of the pivot and is connected to the hood body.

[Item 8] A drug injection device, including:

a device housing having a housing space that houses at least a portion of a cassette that houses a drug cartridge having a first temperature sensor and an RF tag, or at least a portion of a drug cartridge having a first temperature sensor and an RF tag and being not housed in the cassette, and a housing opening that communicates with the housing space;
a piston supported so that the piston can move into the housing space;
a motor configured to drive the piston;
a motor driver configured to generate a drive signal for driving the motor;
an antenna arranged adjacent to the housing space;
a transmission/reception circuit configured to transmit electromagnetic waves from the antenna and receive electromagnetic waves received by the antenna;
a display device configured to output information regarding an injection operation; and
a control device configured to control the motor driver, the transmission/reception circuit and the display device, wherein:
with the drug cartridge loaded in the housing space, the control device causes the transmission/reception circuit to receive, via the antenna, drug information including information indicating a type of a drug in the drug cartridge transmitted from an RF tag of the drug cartridge and first temperature information detected by the first temperature sensor, and determines a drive power of the motor based on the first temperature information to control the motor driver so as to output the determined drive power.

[Item 9] A drug injection device, including:

a device housing having a housing space that houses at least a portion of a cassette that houses a drug cartridge having a first temperature sensor and an RF tag, or at least a portion of a drug cartridge having a first temperature sensor and an RF tag and being not housed in the cassette, and a housing opening that communicates with the housing space;
a piston supported so that the piston can move into the housing space;
a motor configured to drive the piston;
a motor driver configured to generate a drive signal for driving the motor;
an antenna arranged adjacent to the housing space;
a transmission/reception circuit configured to transmit electromagnetic waves from the antenna and receive electromagnetic waves received by the antenna;
a memory storing control information for controlling the motor for each of a plurality of drugs, wherein the control information of each drug includes a set of control parameters of the motor for each of a plurality of temperature ranges;
a display device configured to output information regarding an injection operation; and
a control device configured to control the motor driver, the transmission/reception circuit, the memory and the display device.

[Item 10] The drug injection device according to item 9, wherein:

the RF tag stores drug information including information indicating at least a type of the drug;
with the drug cartridge loaded in the housing space, the control device is configured to:

cause the transmission/reception circuit to transmit a control signal via the antenna;
cause the transmission/reception circuit to receive, via the antenna, first temperature information and drug information output from the first temperature sensor and the RF tag of the drug cartridge;
determine one set of control parameters from the control information stored in the memory based on the first temperature information and the drug information; and
control the motor using the determined set of control parameters.

[Item 11] The drug injection device according to item 10, wherein the motor driver generates a drive signal by pulse width modulation and outputs the drive signal to the motor.

[Item 12] The drug injection device according to item 11, wherein in the control information of each drug, a duty ratio of the drive signal by the pulse width modulation of a set of control parameters for the motor is smaller as a lower limit temperature of a temperature range of the set of control parameters for the motor is higher.

[Item 13] The drug injection device according to item 11 or 12, wherein:

in the control information of each drug, the set of control parameters of the motor for each temperature range includes an initial value and an increment of duty ratio of the drive signal by pulse width modulation; and

the control device causes the motor driver to generate the drive signal so that current flowing in the motor increase in steps.

[Item 14] The drug injection device according to any one of items 10 to 13, wherein the control device does not drive the motor when the first temperature information is in a first temperature range that is less than a first temperature or equal to or greater than a second temperature.

[Item 15] The drug injection device according to item 14, wherein when the first temperature information is in a second temperature range that is equal to or greater than a first temperature and less than a third temperature lower than the second temperature, the control device controls the display device to display information indicating to wait before injection.

[Item 16] The drug injection device according to item 15, wherein when the first temperature information is in a third temperature range that is equal to or greater than the third temperature and less than the second temperature, the control device controls the display device to display information indicating that it is possible to perform injection.

[Item 17] The drug injection device according to item 15, further including a second temperature sensor provided in the device housing and configured to output second temperature information indicating a temperature inside the device housing.

[Item 18] The drug injection device according to item 17, wherein when the second temperature information is equal to or greater than a predetermined temperature, the control device successively obtains the first temperature information at predetermined time intervals, successively calculates estimated time until it is possible to perform injection based on the first temperature information and the second temperature information, and controls the display device to display information indicating the calculated estimated time.

[Item 19] The drug injection device according to any one of items 8 to 18, wherein:

the antenna includes a first portion and a second portion, which can independently receive signals from outside; and

the first portion and the second portion are arranged orthogonal to each other adjacent to the housing space.

[Item 20] The drug injection device according to any one of items 8 to 19, further including:

a user interface including an inject button and configured to accept an instruction from an operator; and

a touch sensor of a surface charge transfer type, wherein:

the device housing has a skin-contact surface that comes into contact with skin of the operator during injection; and

the touch sensor is arranged on the skin-contact surface.

[Item 21] The drug injection device according to item 20, wherein:

when a signal resulting from pressing the inject button is received while a detection signal resulting from skin contact is received from the touch sensor, the control device controls the motor driver so as to perform an injection operation; and

when the touch sensor detects separation from skin during the injection operation, the control device causes the display device to display information indicating abnormality.

[Item 22] The drug injection device according to any one of items 8 to 21, further including:

a sensor having a first axis, a second axis and a third axis, which are orthogonal to each other, configured to detect acceleration along the axes or angular acceleration around the axes, and arranged in a housing so that one of the first axis, the second axis and the third axis coincides with a direction of movement of the piston; and

the control device causes the display device to display information regarding an attitude of the drug injection device based on a detection signal of the sensor.

[Item 23] The drug injection device according to any one of items 8 to 22, further including:

a rotary encoder including an encoder plate attached to a rotation axis of the motor and a pulse encoder, wherein:

the encoder plate includes one reference blade section and a plurality of normal blade sections arranged along a circumference, each including a notch and a blade;

the blades of the plurality of normal blade sections have an equal length in a circumferential direction, and the notches of the plurality of normal blade sections have an equal length in the circumferential direction;

a length of the blade of the reference blade section in the circumferential direction and a length of the notch in the circumferential direction are different respectively from the length of the blades of the plurality of normal blade sections in the circumferential direction and the length of the notches in the circumferential direction; and

the pulse encoder includes a light-emitting element and a light-receiving element arranged so as to receive light emitted from the light-emitting element, wherein the reference blade section and the plurality of normal blade sections of the encoder plate, which rotates in sync with the rotation of the motor, crosses an optical path between the light-emitting element and the light-receiving element, thereby generating a pulse signal including a pulse corresponding to the blade of the reference blade section and pulses corresponding to the blades of the plurality of normal blade sections.

[Item 24] The drug injection device according to item 23, wherein the length of the blade of the reference blade section in the circumferential direction is larger than the length of the blades of the normal blade sections in the circumferential direction, and the length of the notch of the reference blade section in the circumferential direction is smaller than the length of the notches of the normal blade sections in the circumferential direction.

[Item 25] The drug injection device according to item 23 or 24, wherein the control device controls the motor driver based on the pulse signal.

[Item 26] The drug injection device according to any one of items 23 to 25, wherein the control device calculates the number of blades of the reference blade section and the plurality of normal blade sections per one revolution of the encoder plate based on the pulse signal, and causes the display device to display information indicating a fault if the calculation result is different from a predetermined value.

[Item 27] The drug injection device according to item 22, wherein the control device reverses an orientation of information displayed on the display device based on a detection signal from the sensor.

[Item 28] The drug injection device according to any one of items 8 to 27, wherein the control device causes the display device to display the drug information.

[Item 29] The drug injection device according to any one of items 8 to 28, wherein the control device displays a theme color corresponding to a type of the drug across a plurality of operation screens.

[Item 30] The drug injection device according to item 8 or 9, wherein:

the device housing has a distal end section including a protruding portion extending in a longitudinal direction, a skin-contact surface located on an upper surface of the protruding portion, a device recess portion adjacent to the skin-contact surface, the housing opening being located at a bottom of the device recess portion, and an injection needle attachment portion that covers the housing opening and has an inner space in which a distal end of the drug cartridge is inserted;

the housing space is adapted to house at least a portion of the drug cartridge that is not housed in the cassette;

the drug injection device further including a hood including a hood body located in the device recess portion and pivotally attached to the device housing, a needle covering supported to be movable relative to the hood body, and a biasing member configured to bias the needle covering in a direction of the projecting position; and

the hood is pivotable between a first position at which the injection needle attachment portion is covered and a second position at which the injection needle attachment portion is exposed.

[Item 31] A drug injection system, including:

the cassette according to any one of items 4 to 7; and
the drug injection device according to any one of items 8 to 29.

[Item 32] The drug injection system according to item 31, further including the drug cartridge according to any one of items 1 to 3, wherein:

the drug cartridge is housed in the cassette columnar space of the cassette.

[Item 33] A drug injection system, including:

the drug cartridge according to any one of items 1 to 3;
a cassette including a cassette columnar space in which at least a portion of the drug cartridge is housed; and
the drug injection device according to any one of items 8 to 29.

[Item 34] The drug injection system according to item 31 or 32, wherein the drug injection device includes:

a secondary battery; and
a charging terminal provided in the device housing, wherein:

the device housing has a distal end section including a protruding portion extending in a longitudinal direction, a skin-contact surface located on an upper surface of the protruding portion, a device recess portion adjacent to the skin-contact surface, the housing opening being located at a bottom of the device recess portion; and with the cassette loaded in a housing space of the drug injection device, a portion of the hood of the cassette is exposed to an outside in the recess portion of the device housing, and another portion of the hood is located in the device housing so that the hood cannot pivot.

[Item 35] The drug injection system according to item 34, further including a charger including a power supply circuit for charging the secondary battery of the drug injection device, and a charger housing that houses the power supply circuit, wherein:

the charger housing includes a charger recess portion having a space in which the distal end portion of the drug injection device can be inserted, a step provided at a bottom of the charger recess portion and having a shape corresponding to the housing recess portion of the drug injection device, and a supply terminal located in the recess portion and connected to the power supply circuit; and the charger housing is configured so that with the cassette not loaded in the drug injection device, the distal end section of the drug injection device can be housed in the charger recess portion so that the charging terminal of the drug injection device is in contact with the supply terminal, and with the cassette loaded in the drug injection device, the cassette interferes with the step of the charger housing, thereby preventing the distal end section of the drug injection device from being housed in the charger recess portion.

(First embodiment)

(Outline of drug injection system)

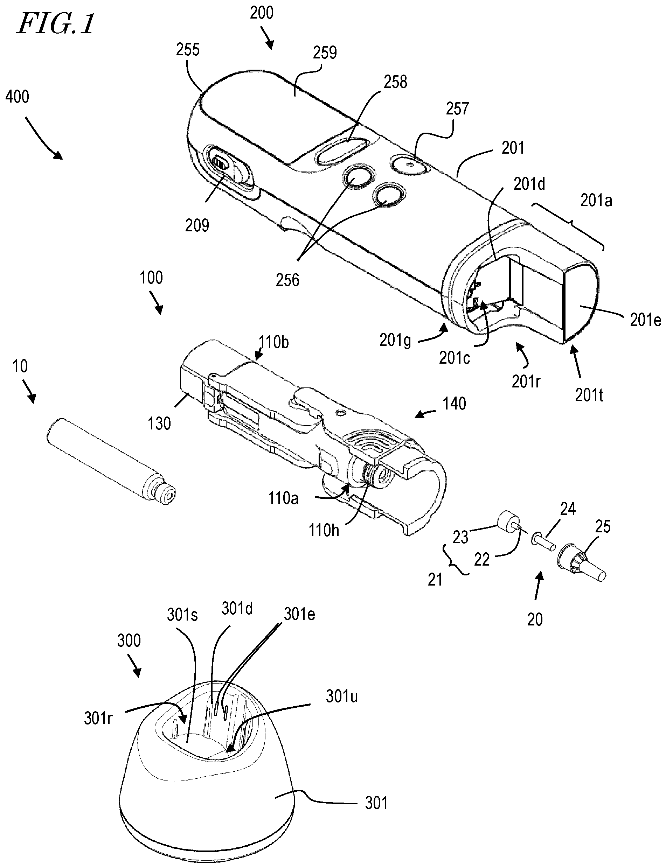

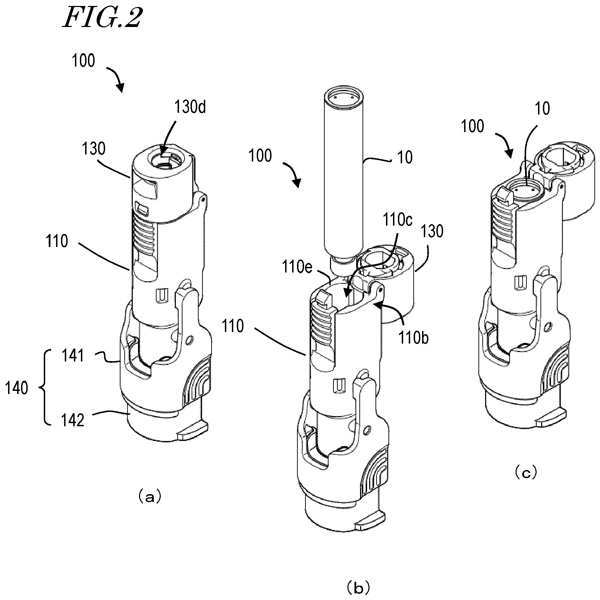

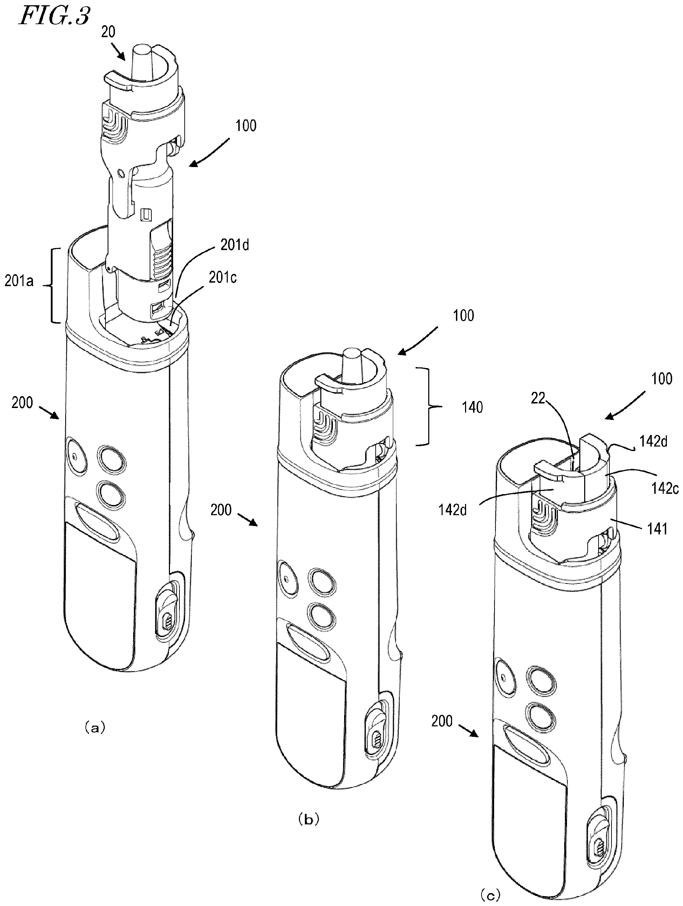

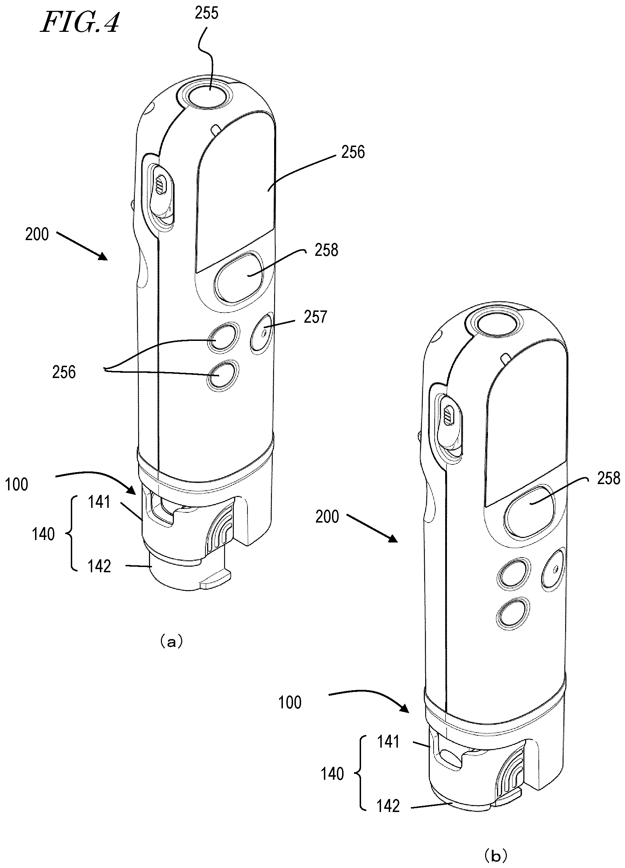

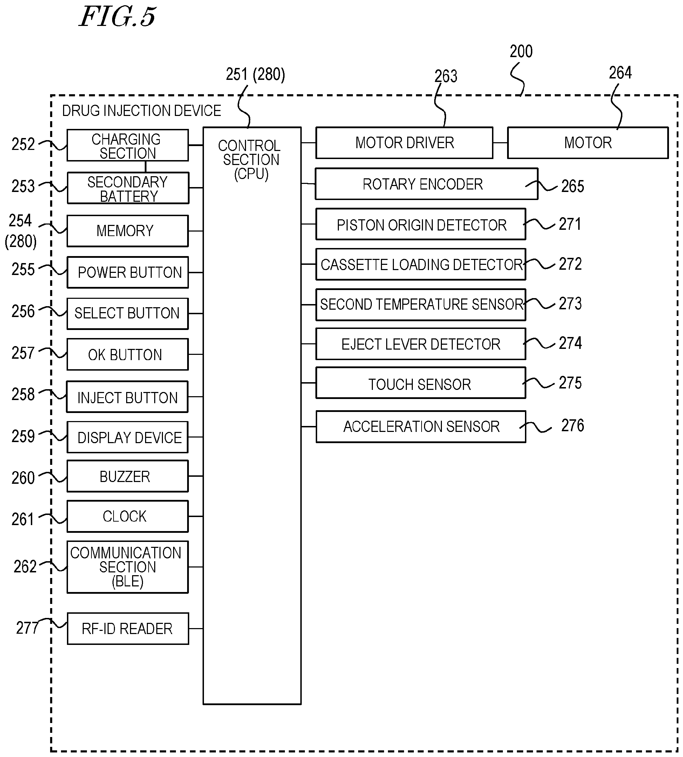

FIG. **1** is a perspective view showing a drug injection system **400** including a cassette **100,** a drug injection device **200** and a charger **300.** The cassette **100** houses the drug cartridge **10.** FIG. **2(a)** to FIG. **2(c)** are perspective views illustrating how the drug cartridge **10** is housed in the cassette **100,** and FIG. **3(a)** to FIG. **3(c)** are perspective views illustrating how the cassette **100** is housed in the drug injection device **200.** FIG. **4(a)** and FIG. **4(b)** are perspective views illustrating the state where injection is performed. FIG. **5** is a block diagram illustrating an example configuration of an electrical circuitry of the drug injection device **200.** With reference to these figures, the drug injection system will be outlined. Note that the drug injection system of the present disclosure is typically used by patients to perform injection by themselves. However, if a patient is young and it is not appropriate for the patient to handle the drug injection system himself/herself, a person other than the patient, such as a guardian, may handle the drug injection system.

The drug cartridge **10** holds multiple doses of drug to be injected into an operator such a as a patient, for example. As shown in FIG. **2(a),** the cassette **100** includes a cassette body **110,** a cassette cap **130** and a hood **140.**

The cassette body **110** has a cassette columnar space **110c** capable of housing at least a portion of the drug cartridge **10,** an injection needle attachment portion **110h** that is located at the distal end **110a** of the cassette columnar space **110c** and to/from which an injection needle can be attached/detached, and a body opening **110e** that is located at the rear end **110b** of the columnar space and accessible to the columnar space. The cassette cap **130** is supported in the vicinity of the rear end **110b** of the cassette body **110** so that the cassette cap **130** can open and close the body opening **110e.** The hood **140** includes a hood body **141** and a needle covering **142.** The needle covering **142** is supported on the hood body **141** so that the needle covering **142** can move between a projecting position in which the needle covering **142** projects relative to the hood body **141** and a stowed position in which the needle covering **142** is at least partially stowed in the hood body **141.**

As shown in FIG. **2(b),** the cassette cap **130** is opened, the drug cartridge **10** is inserted into the cassette columnar space **110c** and the cassette cap **130** is closed, thereby completing the loading of the drug cartridge **10** in the cassette **100.** After starting to use a new drug cartridge **10,** if the drug is still in the drug cartridge **10,** the cassette **100** can be stored in a case (not shown), for example, and stored in a cold place, such as a refrigerator, with the drug cartridge **10** loaded in the cassette **100.**

As will be described in detail below, in the present embodiment, the drug cartridge **10** includes a first temperature sensor and an RF tag. The first temperature information detected by the first temperature sensor **15** is transmitted by the RF tag to the drug injection device **200.**

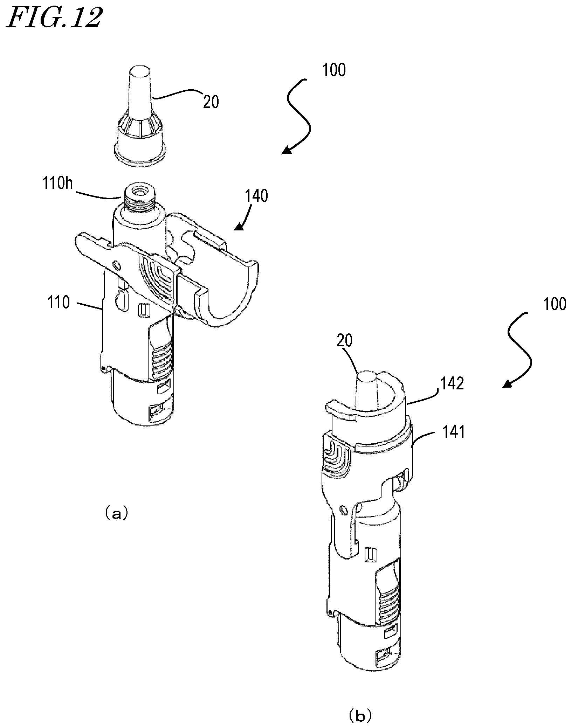

As shown in FIG. **12,** an injection needle **21** can be attached to and detached from the injection needle attachment portion **110h** of the cassette **100.** The injection needle **21** is disposable, and is handled separately from the cassette **100** except when in use as a needle unit **20,** for example. The needle unit **20** includes the injection needle **21,** a needle cap **24** and a needle case **25.** The injection needle **21** includes a needle **22** and a connecting portion **23** that supports the needle **22** and is detachably attached to the injection needle attachment portion **110h** of the cassette **100.** For example, a male thread may be provided on the distal end of the injection needle attachment portion **110h** and a female thread may be provided on a connecting portion **23** of the injection needle **21.** The needle cap **24** has a cylindrical shape that covers the needle **22,** and the needle case **25** houses the injection needle **21** with the needle **22** covered by the needle cap **24.**

Note that, as shown in FIG. **1,** the drug cartridge **10,** the cassette **100** and the drug injection device **200** each have the longitudinal direction L. In the present application, one of the opposite ends of the drug cartridge **10,** the cassette **100** and the drug injection device **200** in the longitudinal direction to which the injection needle **21** is attached is referred to as the distal end, the distal end section or the distal end portion. The end opposite to the distal end in the longitudinal direction of the drug cartridge **10,** the cassette **100** and the drug injection device **200** is referred to as the rear end, the rear end section or the rear end portion.

The drug injection device **200** has a device housing **201.** The device housing **201** has a cylindrical shape having such a thickness that is easy for the operator to hold with one hand, for example. In the present embodiment, the device housing **201** has an oblong circle shape in a cross section perpendicular to the longitudinal direction, making it easy for the operator to hold. However, the shape of the device housing **201** is not limited to this and may be a cylindrical shape or a rectangular cylindrical shape.

A distal end section **201a,** which is one of the opposite ends of the device housing **201** in the longitudinal direction, has a protruding portion **201t** extending in the longitudinal direction and a device recess portion **201r.** The device recess portion **201r** is adjacent to the protruding portion **201t,** and the protruding portion **201t** and the device recess portion **201r** are formed by providing a gap in the cylindrical shape of the device housing **201** that cuts out a portion of the end surface and a portion of the side surface of the cylindrical shape. The upper surface of the protruding portion **201t** is referred to as the skin-contact surface **201e.** A housing opening **201d** into which the cassette **100** can be inserted is located at the bottom surface of the device recess portion **201r.** The drug injection device **200** has a housing space **201c** within the device housing **201** capable of housing at least a portion of the cassette **100,** and the housing space **201c** is connected to the housing opening **201d.**

The drug injection device **200** has a power button **255,** a select button **256,** an OK button **257,** an inject button **258,** an eject lever **209** and a display device **259** on the surface of the device housing **201.** The surface on which these buttons and the display device **259** are located is called the front surface. The power button **255,** the select button **256,** the OK button **257** and the inject button **258** are examples of user interfaces that receive instructions from the operator. Some or all of the user interfaces may be a touch panel provided on the display device **259.** A charging terminal **201g** is arranged on the distal end section **201a** of the device housing **201,** which will be described below.

When the drug injection system **400** is used, when the power button **255** is pressed to start the drug injection device **200,** the display device **259** displays the operating procedure of the drug injection device **200,** and the drug information, the injection history, etc., of the drug cartridge **10** in the loaded cassette **100.**

As shown in FIG. **3(a)** to FIG. **3(c),** the cassette **100,** to which the needle unit **20** is attached, is loaded in the drug injection device **200,** and the needle case **25** and the needle cap **24** are removed. In this state, the needle **22** is located within the space enclosed by the needle covering **142,** and the distal end of the needle **22** does not project from the needle covering **142.**

After pressing the select button **256** and the OK button **257** as appropriate to determine the operation of the drug injection device **200,** a drug injection operation is performed. The drug injection device **200** of the present embodiment is of a semi-automatic type, with needle insertion and needle removal being manual, i.e., performed by the operator. As shown in FIG. **4(a)** and FIG. **4(b),** in the state where the drug injection device **200** is held so that the distal end of the needle covering **142** is in contact with the skin, when the drug injection device **200** is pressed against the skin, the needle covering **142** retracts into the hood body **141.** Accordingly, the distal end of the needle **22** comes into contact with the skin, and the injection needle **22** is inserted into the skin to a predetermined depth. Then, when the inject button **258** is pressed, a predetermined amount of drug is injected from the drug cartridge **10.**

After completion of the drug injection, as the operator moves the drug injection device **200** off the skin, the injection needle **21** is pulled out of the skin. Then, the eject lever **209** is operated to eject the cassette **100** from the drug injection device **200.**

As shown in FIG. **5,** the drug injection device **200** includes a control section **251** including an arithmetic unit such as a CPU, a secondary battery **253** as a power source, a charging section **252** including a charging circuit for charging the secondary battery **253,** a memory **254** storing computer programs, data, etc., and a clock **261.** The control section **251** and the memory **254** together form a control device **280,** and the control section **251** reads a program stored in the memory **254** and controls various components shown in FIG. **5** according to the procedure of the computer program. The procedure of the computer program will be shown by the following description and by the flow charts of the accompanying drawings. The drug injection device **200** may further include a buzzer **260** that informs the operator by means of sound.

The drug injection device **200** further includes a motor driver **263,** a motor **264** and a rotary encoder **265.** The motor driver **263,** the motor **264** and the rotary encoder **265** together form a portion of the piston drive mechanism as will be described below.

The drug injection device **200** also includes various detectors to detect the status of various parts of the drug injection device **200.** Specifically, the drug injection device **200** includes a piston origin detector **271,** a cassette loading detector **272,** an eject lever detector **274,** a touch sensor **275** and an acceleration sensor **276.** The drug injection device **200** may further include a second temperature sensor **273.**

The drug injection device **200** includes an RF-ID reader **277.** The drug injection device **200** may further include an RF-ID writer. The RF-ID reader **277** reads the first temperature information of the first temperature sensor **15** transmitted from an RF tag **16** of the drug cartridge **10,** and drug information including information indicating the type of the drug stored in the memory of the RF tag **16.** The read information is input to the control section **251,** which uses the obtained first temperature information to control the motor and control the operation of the drug injection device **200.**

The drug injection device **200** may further include a communication section **262.** The communication section **262** transmits and receives information to and from the outside by, for example, infrared communication, wireless communication, etc. Specifically, the communication section **262** may be a transmitter/receiver that uses a short-range wireless communication standard, such as BLE (Bluetooth Low Energy, Bluetooth is a registered trademark). For example, the time when the operator uses the drug injection device **200,** the type of drug, the amount of drug injected, and other information may be stored in the memory **254** at the time of use, and at a predetermined timing, these information may be transmitted using the communication section **262** to a portable device such as a smartphone, a tablet device, or to an external device such as a dedicated device for managing the drug injection device **200.** The information may be transmitted from a portable device to a server, or the like, of a hospital, a drug manufacturer, etc., via a cellular phone line or an Internet line.

The charger **300** includes a charger housing **301** and a power supply circuit arranged in the charger housing **301.** The charger housing **301** includes a charger recess portion **301r** with a space into which the distal end section **201a** of the drug injection device **200** can be inserted.

The charger housing **301** includes a step **301s** provided at the bottom of the charger recess portion **301r** and having a shape corresponding to the device recess portion **201r** provided in the distal end section **201a** of the drug injection device **200,** a space **301u** within the charger recess portion **301r** and adjacent to the step **301s,** and a supply terminal **301e** located in the charger recess portion **301r** and connected to the charging circuit. A plurality of ribs **301d** extending in the depth direction of the charger recess portion **301r** are provided on the side surface of the charger recess portion **301r.**

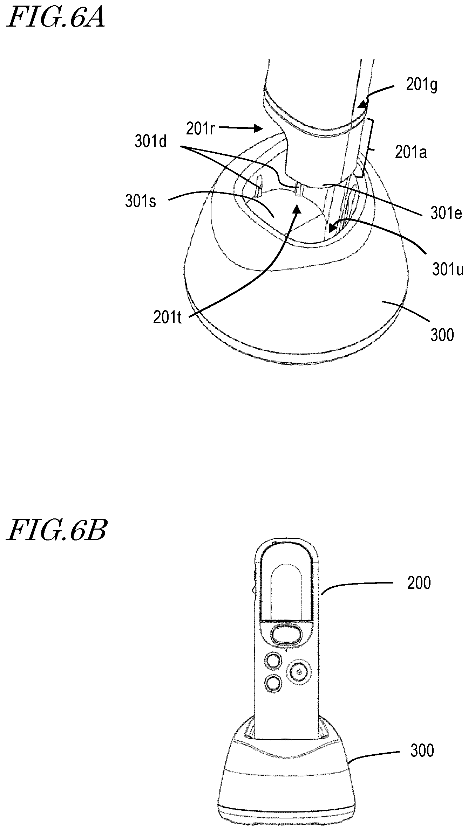

FIG. **6A is** a view illustrating how the drug injection device **200** is set in the charger **300,** and FIG. **6B** is a front view showing the drug injection device **200** set in the charger **300.** In order to charge the drug injection device **200** with the charger **300,** the cassette **100** is removed from the drug injection device **200,** and the distal end section **201a** of the drug injection device **200** is inserted into the charger recess portion **301r** of the charger **300.** At this time, since the device recess portion **201r** of the drug injection device **200** corresponds to the step **301s,** the protruding portion **201t** is inserted into the space **301u** beside the step **301s** without interference, and the entire distal end section **201a** can be inserted into the charger recess portion **301r,** as shown in FIG. **6B.** This allows the distal end section **201a** of the drug injection device **200** to be housed in the charger recess portion **301r** so that the charging terminal **201g** of the drug injection device **200** is in contact with the supply terminal **301e.** At this time, the side surface of the distal end section **201a** of the drug injection device **200** is in contact with the ribs **301d** of the charger **300.** Therefore, a space is formed between the side surface of the charger recess portion **301r** and the side surface of the distal end section **201a** of the drug injection device **200.** This space allows the heat of the secondary battery generated by charging to be released to the outside of the charger recess portion **301r.**

As shown in FIG. **6B,** the charger **300** can hold the drug injection device **200** so that the drug injection device **200** is in the upright position. This allows the charger **300** to function as a storage place for the drug injection device **200** when not in use, and to store the drug injection device **200** in a space-saving and more conspicuous manner than when the drug injection device **200** is stored on its side or the drug injection device **200** is housed and charged inside a case.

On the other hand, when the cassette **100** is loaded in the drug injection device **200,** a portion of the cassette **100** protrudes into the device recess portion **201r.** Therefore, even if one tries to insert the distal end portion **201a** of the drug injection device **200** into the charger recess portion **301r** of the charger **300,** the cassette **100** interferes with the step **301s** of the charger recess portion **301r,** thereby preventing the protruding portion **201t** from being inserted into the space **301u** beside the step **301s,** and the entire distal end section **201a** from being inserted into the charger recess portion **301r.** Therefore, the distal end section **201a** of the drug injection device **200** cannot be housed in the charger recess portion **301r,** and the drug injection device **200** cannot be set in the charger **300.**

Thus, with the drug injection system **400** of the present embodiment, the drug injection device **200** cannot be loaded in the charger **300** with the cassette **100** inserted. This ensures that the cassette **100** be removed during charging, thereby preventing the drug in the cassette **100** from deteriorating due to the heat generated by the drug injection device **200** during charging.

With the drug injection system of the present embodiment, the drug cartridge **10** includes the first temperature sensor **15,** thereby allowing the temperature of the drug stored in the drug cartridge **10** to be measured. This information can be used to estimate the viscosity of the drug so as to drive the motor with a drive force in accordance with the viscosity, and determine whether the temperature is suitable for injection so as to control the operation of the drug injection device **200.** The drug cartridge **10,** the cassette **100** and the drug injection device **200** will now be described in detail.

(Drug cartridge **10**)

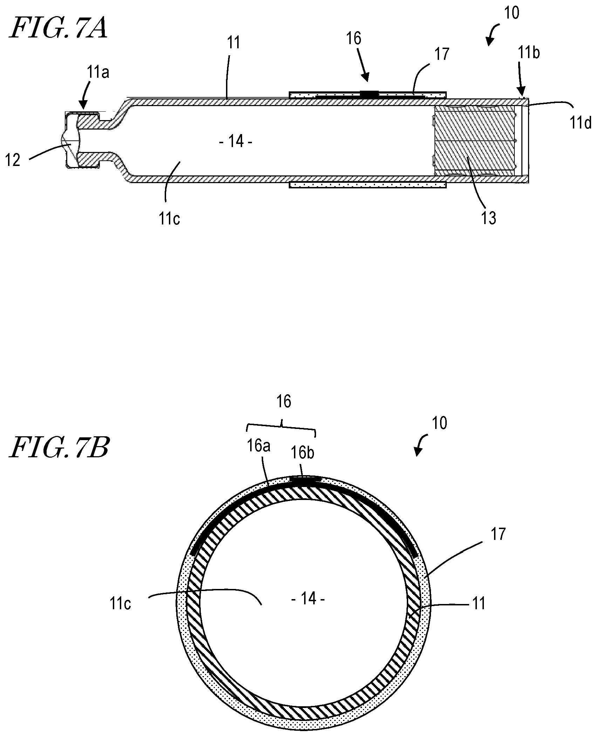

An example of the drug cartridge **10** of the present embodiment will be described. FIG. **7A** is a cross section parallel to the longitudinal direction of the drug cartridge **10,** and FIG. **7B** is a cross section perpendicular to the longitudinal direction of the drug cartridge **10.** The drug cartridge **10** includes a cylinder **11,** a cylinder cap **12,** a gasket **13,** a drug **14** and an RF tag **16.**

The cylinder **11** has a first end **11a** and a second end **11b,** which are spaced apart from each other in the longitudinal direction, and a cylinder columnar space **11c** located between the first end **11a** and the second end **11b.** The needle **22** of the injection needle **21** can be inserted and pulled out through the first end **11a.** For example, the cylinder **11** has an outer shape that tapers on the first end **11a** side so that the cross section perpendicular to the longitudinal direction of the cylinder columnar space **11c** decreases on the first end **11a** side, and the opening of the cylinder columnar space **11c** on the first end **11a** side is sealed with a rubber cylinder cap **12.** The cylinder **11** has a cylinder opening **11d** connected to the cylinder columnar space **11c** at the second end **11b.**

The gasket **13** is inserted into the cylinder columnar space **11c** through the cylinder opening **11d** and is supported on the inner wall of the cylinder **11** so that the gasket **13** can move in the longitudinal direction.

The first end **11a** side and the second end **11b** side of the cylinder columnar space **11c** are closed by the cylinder cap **12** and the gasket **13,** and the drug **14** is sealed in the closed cylinder columnar space **11c.** The drug **14** contains at least a liquid first component and is liquid at room temperature.

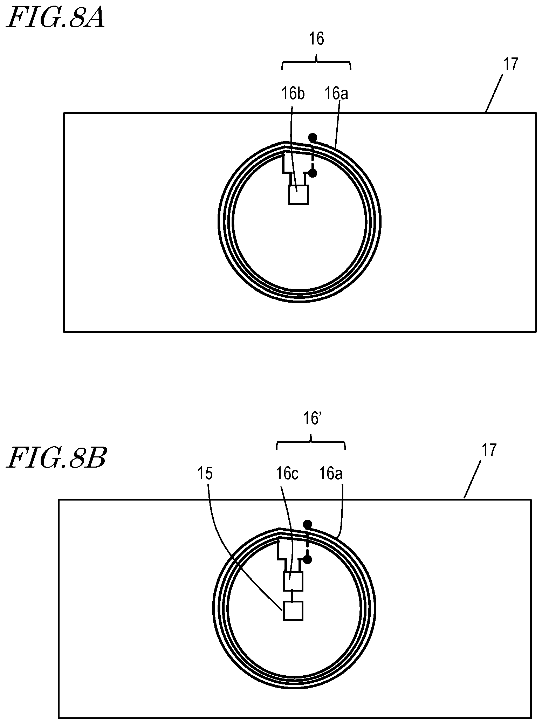

FIG. **8A** and FIG. **8B** are schematic plan views of the RF tag **16.** The RF tag **16** is a device that stores identification information of an object to which the tag is attached, and transmits the identification information wirelessly. In the present embodiment, the RF tag **16** stores drug information including at least information indicating the type of drug in the cylinder columnar space **11c,** and wirelessly transmits the information indicating the type of drug and the first temperature information indicating the temperature detected by the first temperature sensor to the outside in response to an instruction from the outside.

The RF tag **16** may be of an active type or a passive type. In the present embodiment, the RF tag **16** is of a passive type and is an RF tag with a temperature sensor. For example, RF tag **16** includes an antenna **16a** and an IC **16b.** The antenna **16a** transmits and receives electromagnetic waves in the long wave, short wave, or microwave bands. The IC **16b** includes a transmitter section, a receiver section, a storage section and a power rectifier section. The IC **16b** of the present embodiment further includes a first temperature sensor.

As shown in FIG. **8B,** an RF tag **16'** may include an antenna **16a** and an IC **16c** without a temperature sensor. In this case, the drug cartridge **10** further includes a first temperature sensor **15** electrically connected to the IC **16c.** The RF tag **16** or the RF tag **16'** and the first temperature sensor **15** are supported on a label **17** by attaching or laminating, for example. The label **17** is attached to the outer surface of the cylinder **11.** On the outside of the attached label **17,** the name of the drug **14,** or the like, may be written. The drug information to be described below may be written in letters, pictograms, etc.

The first temperature sensor detects the temperature around the second temperature sensor. In the present embodiment, the IC **16b** including the first temperature sensor is attached to the cylinder **11** together with the label, and thus directly detects the temperature of the cylinder **11.** The temperature of the cylinder **11** is the same as the temperature of the drug in the cylinder columnar space **11c.** Therefore, it can be said that the first temperature sensor detects the temperature of the drug in the drug cartridge **10.**

The storage section stores drug information about the drug in the cylinder columnar space **11c.** The drug information includes at least information indicating the type of drug. The drug information may further store information indicating the expiration date of the drug, information indicating the initial amount of drug in an unused state, information about the manufacture of the drug such as the manufacturing lot, unique identification information of the drug cartridge **10,** information about the viscosity of the drug, etc.

With the RF tag **16,** when the antenna **16a** receives a signal transmitted from the drug injection device **200** described below, electromotive force due to resonance is generated, and the power rectifier of the IC **16b** rectifies the electromotive force, thereby generating electric power to drive the RF tag **16.** This activates the IC **16b,** which reads out the drug information stored in the storage section and the first temperature information indicating the temperature detected by the first temperature sensor, and the transmitter section converts the read-out information into electromagnetic waves and transmits the information to the outside through the antenna **16a.** The transmitted information is received by the drug injection device and used to control the drug injection device, as will be described below.

The drug cartridge **10,** which includes the first temperature senso, can detect the temperature of the drug upon request from the drug injection device **200.** Thus, it is possible to realize a drug injection device such that when a cold-stored drug cartridge is used for injection, the temperature of the drug can be used to determine whether the temperature of the drug is suitable for injection, to estimate the viscosity of the drug according to the temperature of the drug, and to inject the drug with an appropriate drive force.

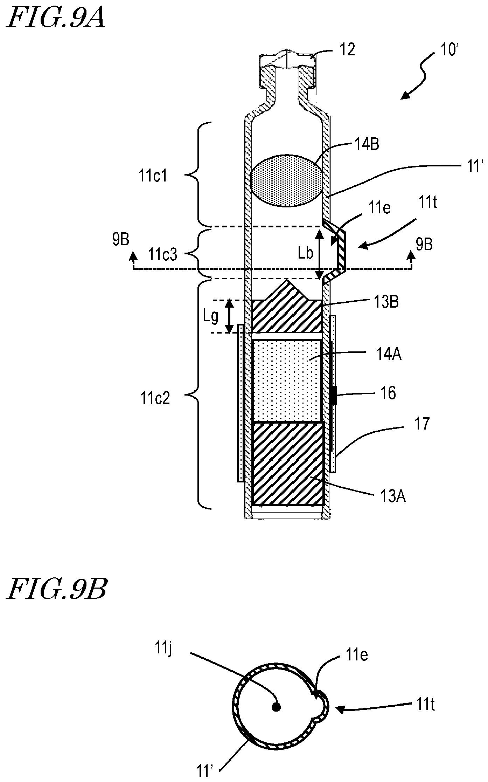

Although the drug cartridge **10** contains only a liquid component, the drug cartridge of the present disclosure may contain a solid component and a liquid component. FIG. **9A** shows a schematic cross section of a drug cartridge **10'** containing a drug in which a liquid component and a solid component are held separate in an unused state, and FIG. **9B** shows a cross section taken along line 9B-9B of FIG. **9A.**

The drug cartridge **10'** includes a cylinder **11',** a first gasket **13A,** a second gasket **13B,** and a liquid component **14A** containing a first component of the drug **14** and a solid component **14B** containing a second component thereof. The liquid component **14A** is liquid at room temperature and the solid component **14B** is solid at room temperature. The solid component **14B** is, for example, in contact with and supported by the inner side surface of the cylinder **11'.** The label **17** and the RF tag **16** are arranged on the side surface of the cylinder **11'.**

The cylinder **11'** has a cylinder columnar space **11c,** and the cylinder columnar space **11c** includes a first region **11c1** located on the first end **11a** side, a second region **11c2** located on the second end **11b** side, and a third region **11c3** sandwiched between the first region **11c1** and the second region **11c2.**

The side surface of the cylinder **11'** includes a projecting portion **11t** projecting outwardly with respect to the axis **11j** in the third region **11c3.** The projecting portion **11t** extends in the longitudinal direction and defines a bypass space **11e** arranged adjacent to the cylinder columnar space **11c** in a cross section perpendicular to the longitudinal direction. The length **Lb** of the bypass space **11e** in the longitudinal direction is longer than the length **Lg** over which the second gasket **13B** is in contact with the inner side surface of the cylinder **11'** (Lb>Lg).

In the initial state where the drug cartridge **10'** is unused, at least a portion of the second gasket **13B** is located in the second region **11c2.** The first gasket **13A** is located on the second end **11b** side of the second region **11c2** in the cylinder columnar space **11c.** The liquid component **14A** of the drug is located in the second region **11c2** and sandwiched between the first gasket **13A** and the second gasket **13B.** On the other hand, the solid component **14B** is located in the first region **11c1** of the cylinder columnar space **11c.**

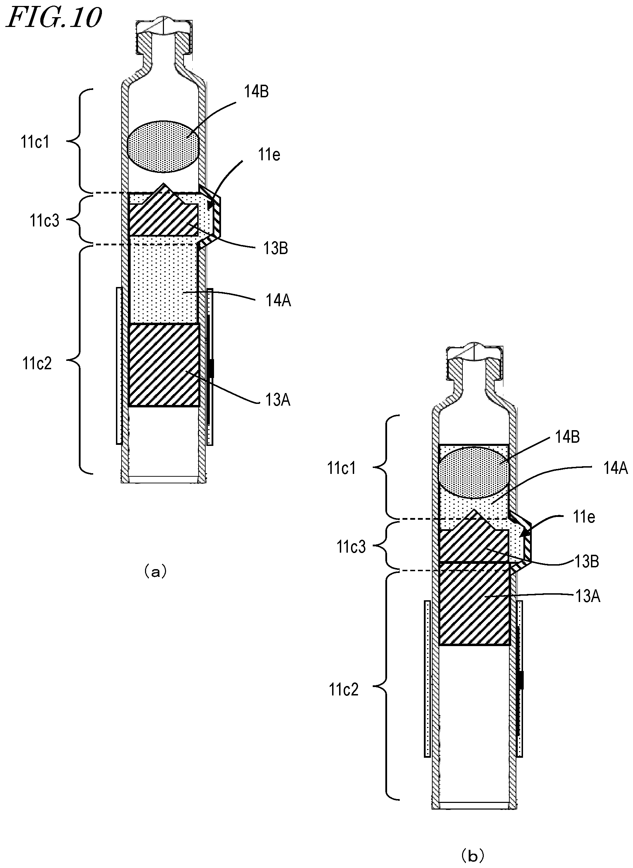

In the drug cartridge **10',** the solid component **14B** is dissolved in the liquid component **14A** before use. FIG. **10(a)** and FIG. **10(b)** are schematic diagrams illustrating the movement of the liquid component **14A** while dissolving the solid component **14B.** When the cassette **100** with the drug cartridge **10'** inserted therein is loaded in the drug injection device **200,** a piston **210** of the drug injection device **200** moves the first gasket **13A** forward. While the rear end of the second gasket **13B** is in the second region **11c2,** the space between the first gasket **13A** and the second gasket **13B** is sealed, so that as the first gasket **13A** moves forward, the second gasket **13B** and the liquid component **14A** moves forward together.

As shown in FIG. **10(a),** when the rear end of the second gasket **13B** reaches the third region **11c3,** the first region **11c1** located forward of the second gasket **13B** and the second region **11c2** located rearward of the second gasket **13B** are connected together by the bypass space **11e,** because the length **Lg** of the second gasket **13B** in the longitudinal direction is shorter than the length **Lb** of the bypass space **11e** in the longitudinal direction. This allows the liquid component **14A** to flow through the bypass space **11e** into the first region **11c1.** In the meantime, the second gasket **13B** does not move even when the first gasket **13A** moves forward, and only the liquid component **14A** moves into the first region **11c1.** Thus, the liquid component **14A** comes into contact with the solid component **14B,** and the solid component **14B** dissolves into the liquid component **14A.**

As shown in FIG. **10(b),** when all the liquid component **14A** moves to the first region **11c1,** the first gasket **13A** contacts the second gasket **13B.** Thus, the first gasket **13A** and the second gasket **13B** thereafter move forward together while being in contact with each other.

As will be described below, the solid component **14B** in contact with the liquid component **14A** dissolves into the liquid component **14A** as the entire drug cartridge **10'** is oscillated by the operator.

Thus, even if the drug held in the drug cartridge contains a solid component and a liquid component, the drug information can be stored in the storage section of the RF tag **16.** Thus, as the stored drug information and the first temperature information of the drug are transmitted to the drug injection device **200,** it is possible to control the drug injection device **200** by a different procedure than a drug cartridge containing only a liquid component, or to perform a dissolution operation depending on the temperature of the drug.

(Cassette **100**)

As described with reference to FIG. **2,** the cassette **100** includes the cassette body **110,** the cassette cap **130** and the hood **140.** The cassette **100** of the present embodiment, having the hood **140,** allows the operator to more safely handle the cassette **100** or the drug injection device **200** with the cassette **100** attached while the injection needle **21** is attached.

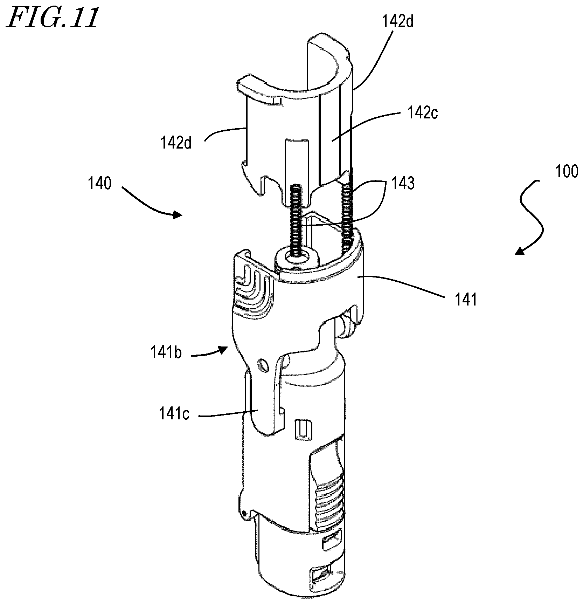

FIG. **11** is an exploded perspective view of the hood **140** of the cassette **100.** The hood **140** includes the hood body **141,** the needle covering **142** and a biasing member **143.** The needle covering **142** has a generally U-letter shape in a cross section perpendicular to the longitudinal direction. Similarly, the hood body **141** also includes a portion having a generally U-letter shape in a cross section perpendicular to the longitudinal direction. The needle covering **142** is movably supported in the longitudinal direction with respect to the hood body **141.** The biasing member **143** biases the needle covering **142** in the direction of projecting from the hood body **141.** While the biasing member **143** is a spring in the present embodiment, it may be any other elastic member.

The needle covering **142** is at least partially transparent. In the present embodiment, the needle covering **142** includes a transparent first portion **142c** and semi-transparent second portions **142d.** More specifically, a first portion **142c** is a region extending in the longitudinal direction, and is located at the bottom of the generally U-letter shape cross section in a cross section perpendicular to the longitudinal direction, for example. The second portions **142d** are located so as to sandwich the first portion **142c.**

The hood body **141** is pivotably supported by the cassette body **110** at the rear end **141b** in the longitudinal direction. Thus, the hood **140** can pivot between the first position at which the injection needle attachment portion is covered and the second position at which the injection needle attachment portion is exposed. The hood body **141** has an arm **141c** connected to the rear end **141b** and extending toward the opposite side of the hood body **141** in the longitudinal direction. The hood body **141** is made of an opaque material, for example. Transparent and opaque may be colorless or colored.

FIG. **12(a)** and FIG. **12(b)** are views illustrating how the needle unit **20** is attached to the cassette **100.** The drug cartridge **10** is inserted into the cassette **100** in advance. As shown in FIG. **12(a),** the hood **140** is first rotated to the second position. In this state, the hood **140** does not cover the injection needle attachment portion **110h** of the cassette **100,** and the injection needle attachment portion **110h** is exposed. Particularly, the hood **140** is located downward (on the rear end side) relative to the injection needle attachment portion **110h.** Thus, for example, when the operator attaches the needle unit **20** to the injection needle attachment portion **110h,** the operator's hand does not come into contact with the hood **140,** making the attachment easy. As shown in FIG. **12(b),** after completion of the attachment of the needle unit **20,** the hood **140** is rotated to the first position. Thus, a portion of the needle unit **20** excluding the distal end section **201a** is covered by the hood **140** from three directions.

As shown in FIG. **3(a),** the cassette **100** is inserted into the housing space **201c** through the housing opening **201d** of the drug injection device **200.** FIG. **3(b)** shows a state where the cassette **100** is completely loaded. In this state, a portion of the hood **140** is exposed in the device recess portion **201r** of the distal end section **201a,** and a portion is located within the housing opening **201d.** Specifically, at least the arms **141c** of the hood body **141** are located within the housing opening **201d.** Therefore, in this state, for example, even if the operator attempts to rotate the hood **140** to the second position, i.e., to open the hood **140** so as to expose the needle unit **20,** the arms **141c** come into contact with the internal housing of the drug injection device **200** in the housing space **201c,** and the hood **140** cannot be rotated. When the cassette **100** is loaded in the drug injection device **200,** a portion of the hood body **141** encloses the device recess portion **201r** of the distal end section **201a.** This allows the drug injection device **200** to be held stable and pressed against the skin during administration.

As shown in FIG. **3(c),** in this state, the operator pulls the needle case **25** of the needle unit **20** to remove the needle cap **24** and the needle case **25** from the needle unit **20,** exposing the injection needle **21.** The exposed injection needle **21** has its distal end located lower than the distal end of the needle covering **142.**

The semi-transparent second portion **142d** of the needle covering **142** faces the same direction as the front surface where the display device **259** of the drug injection device **200,** etc., are arranged. Therefore, when the operator performs injection, the operator visually recognizes the injection needle **21** through the second portion **142d** of the needle covering **142.** With the second portion **142d** being semi-transparent, the operator can recognize that the needle **22** is attached though the operator indistinctly recognizes the shape of the injection needle **21.** Thus, the operator can confirm that the injection needle **21** is correctly attached, and it is possible to reduce the fear caused by not clearly recognizing the needle **22.**

The first portion **142c** of the needle covering **142** is transparent and faces the same direction as the side surface of the drug injection device **200.** Thus, the operator can clearly visually recognize the injection needle **21** through the first portion **142c.** As will be described below, for example, when performing an air-removing operation in the drug cartridge **10,** it is possible to confirm through the first portion **142c** that the drug is seeping out from the distal end of the injection needle **21** and to determine that the air removal is complete.

When performing injection, by pushing down the drug injection device **200** while having the distal end of the needle covering **142** against the skin, the needle covering **142** retracts and is stored in the hood body **141,** causing the injection needle **21** to relatively project from the distal end of the needle covering **142**. This allows the injection needle **21** to be inserted into the skin until the skin-contact surface **201e** of the protruding portion **201t** of the drug injection device **200** comes into contact with the skin.

When the injection is complete and the drug injection device **200** is removed from the skin, the needle covering **142** projects toward the distal end side by the biasing member **143,** thereby covering the injection needle **21** again.

While the cassette **100** housing the drug cartridge **10** has been described above, the cassette of the present embodiment can be similarly configured also when housing the drug cartridge **10'** described above.

(Drug injection device **200**)

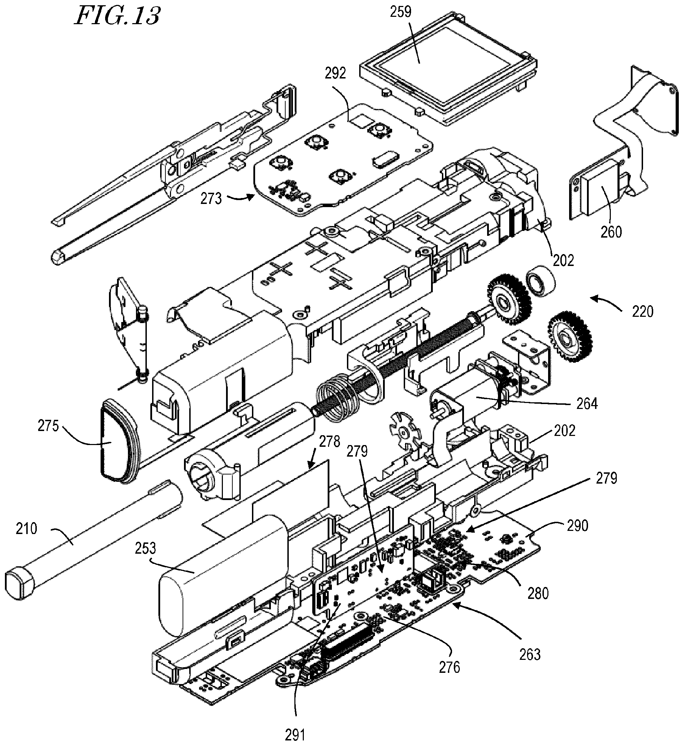

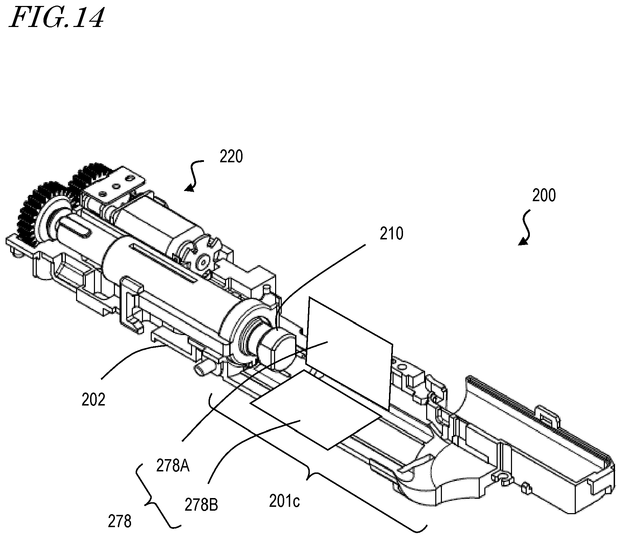

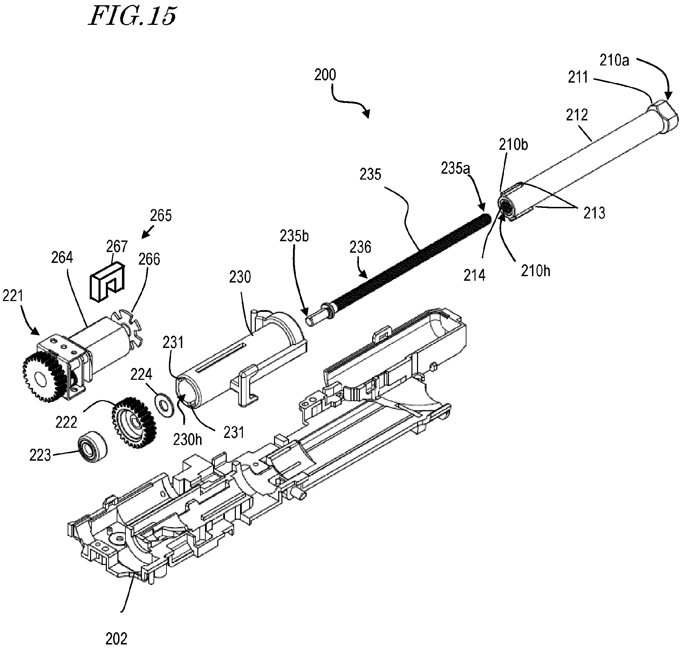

FIG. **13** is an exploded perspective view of the drug injection device **200** with the device housing **201** removed, and FIG. **14** is a perspective view of the drug injection device **200** showing the arrangement of the RF-ID antenna. FIG. **15** is an exploded perspective view of a piston drive mechanism **220** of the drug injection device **200**. In addition to the device housing **201** and the control device **280** described above, the drug injection device **200** includes the piston **210** and the piston drive mechanism **220**. In the present embodiment, the drug injection device **200** further includes an internal housing **202,** a main board **290,** a first sub-board **291** and a second sub-board **292**. The internal housing **202** supports the piston drive mechanism **220**. The control device **280** and the motor driver **263** are formed on the main board **290**. The acceleration sensor **276** is also arranged on the main board **290**.

The RF-ID reader **277** includes an antenna **278** and a transmission/reception circuit **279**. The transmission/reception circuit **279** is formed, for example, on the main board **290**. The select button **256,** the OK button **257,** the inject button **258** and the second temperature sensor **273** are arranged on the second sub-board **292**. The second temperature sensor **273** is preferably arranged at a location where it is less susceptible to heat generated by various components in the device housing **201** during operation.

With the cassette **100,** into which the drug cartridge **10** is inserted, loaded in the drug injection device **200,** the RF-ID reader **277** reads the first temperature information transmitted from the drug cartridge **10** and the drug information including information that indicates the type of drug stored in the memory of the RF tag **16**. The drug injection device **200** injects the drug from the drug cartridge **10** into the operator by moving the piston **210** based on these information. The structure of the RF-ID reader **277** and the piston drive mechanism **220** of the drug injection device **200** will be described primarily below.

<RF-ID reader **277**>

As mentioned above, the RF-ID reader **277** includes the antenna **278** and the transmission/reception circuit **279**. As shown in FIG. **14,** the antenna **278** includes a first portion **278A** and a second portion **278B,** which can independently receive signals from the outside. The first portion **278A** and the second portion **278B** of the antenna **278** are arranged adjacent to the housing space **201c**. The first portion **278A** and the second portion **278B** each include a generally planar radiating conductor and are preferably arranged orthogonal to each other.

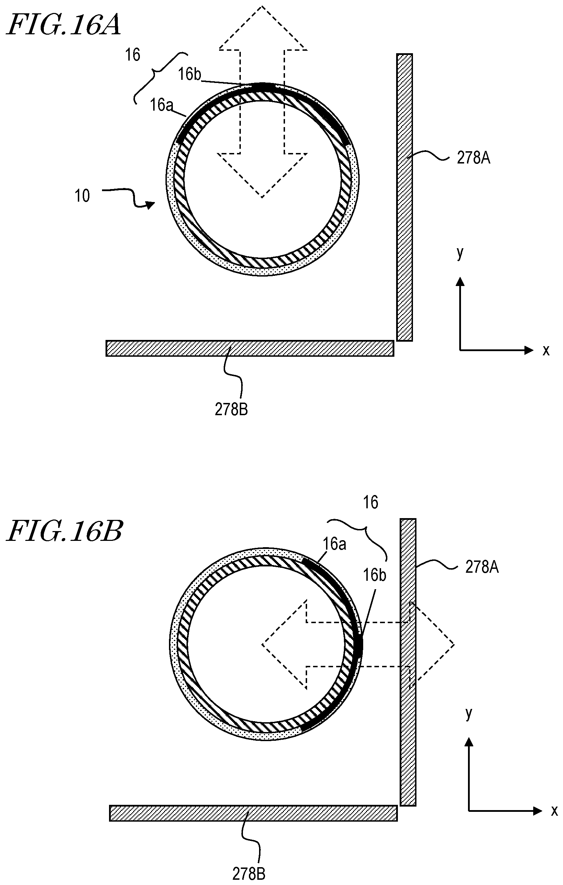

FIG. **16A** and FIG. **16B** schematically illustrate the positional relationship between the RF tag **16** and the first portion **278A** and second portion **278B** of the antenna **278** in the state where the cassette **100** into which the drug cartridge **10** is inserted is loaded in the drug injection device **200**. In FIG. **16A** and FIG. **16B,** the cassette **100** is not shown. In the present embodiment, the drug cartridge **10** has a cylindrical shape, and the outer shape of the cross section perpendicular to the longitudinal direction has a generally circular shape. Therefore, it can be inserted in any orientation (angle) around its axis with respect to the cassette columnar space **110c** of the cassette **100**. Thus, when the cassette **100** is loaded in the drug injection device **200,** the flat RF tag **16** can face any direction.

On the other hand, the antenna **16a** of the RF tag **16** has a generally flat shape and has directionality. Specifically, the antenna **16a** transmits electromagnetic waves with a large field strength distribution in a direction perpendicular to the planar shape. Therefore, if the antenna **278** of the RF-ID reader **277** has high receiving sensitivity only in one direction, the direction in which the receiving sensitivity of the antenna **278** is high and the direction in which the field strength of the electromagnetic wave transmitted from the antenna **16a** of the RF tag **16** is strong are orthogonal or nearly orthogonal to each other, and the electromagnetic waves transmitted from the RF tag **16** may not be correctly received.

In the present embodiment, the first portion **278A** and the second portion **278B** of the antenna **278** are arranged orthogonal to each other, so that the antenna **278** has high sensitivity in the two orthogonal directions. Thus, for example, as shown in FIG. **16A,** when the antenna **16a** of the RF tag **16** is spread generally perpendicular to the y direction, the antenna **278** cannot receive electromagnetic waves from the antenna **16a** of the RF tag **16** with strong sensitivity in the first portion **278A,** but electromagnetic waves from the antenna **16a** of the RF tag **16** can be received with sufficient sensitivity in the second portion **278B**. As shown in FIG. **16B,** when the antenna **16a** of the RF tag **16** is spread generally perpendicular to the x direction, the antenna **278** cannot receive electromagnetic waves from the antenna **16a** of the RF tag **16** with strong sensitivity in the second portion **278B,** but electromagnetic waves from the antenna **16a** of the RF tag **16** can be received with sufficient sensitivity in the first portion **278A.** Even when the antenna **16a** of the RF tag **16** is oriented in a direction other than those shown in FIG. **16a** and FIG. **16b,** the first portion **278A** or the second portion **278B** of the antenna **278** can receive the electromagnetic waves from the antenna **16a** of the RF tag **16** with sufficient sensitivity. Thus, with the drug injection device **200** of the present embodiment, it is possible to more reliably read, from the drug cartridge **10** in the cassette **100,** the first temperature information and drug information including the type of drug stored in the memory of the RF tag **16.**

Information from the RF tag **16** attached to the drug cartridge **10** is read by the RF-ID reader **277** by, for example, the following procedure. First, a signal for activation is generated from the transmission/reception circuit **279** of the RF-ID reader **277** to generate electric power to cause the RF tag **16** to transmit information to the RF tag **16,** and electromagnetic waves are transmitted from the first portion **278A** and/or the second portion **278B** of the antenna **278.** When the antenna **16A** of the RF tag **16** receives electromagnetic waves, the antenna **16a** receives the electromagnetic waves, thereby generating an electromotive force by resonance. The generated electromotive force activates the IC **16b,** which reads the information stored in the storage section and converts it into a signal. The temperature detected by the first temperature sensor is converted into a signal. The generated signal is transmitted from the IC **16b** to the antenna **16a,** and electromagnetic waves are transmitted from the antenna **16a.** The RF-ID reader **277** receives electromagnetic waves from the RF tag **16** by the first portion **278A** or the second portion **278B** of the antenna **278,** and converts the electromagnetic waves into a signal by the transmission/reception circuit **279.** Thus, drug information is obtained, including the first temperature information and information indicating the type of drug stored in the memory of the RF tag **16.** These information are transmitted to the control device **280.**

<Piston drive mechanism **220**>

Reference is made to FIG. **14** and FIG. **15.** The piston **210** includes a distal end section **211** and a body **212** connected to the distal end section **211.** The distal end section **211** is located on the first end **210a** side. In the present embodiment, the distal end section **211** has an I-cut shape which is obtained by cutting a cylinder in two parallel planes along its axis. The distal end section **211** has a shape corresponding to the cap opening **130d** provided in the cassette cap **130** of the cassette **100.**

The piston **210** includes a drive protruding portion **213** located on the side surface of the body **212.** The drive protruding portion **213** engages with a guide **231** provided in the piston drive mechanism **220** as will be described below, thereby regulating rotation based on the shape of the guide **231.** In the present embodiment, the drive protruding portion **213** is a rib provided on the side surface of the piston **210,** and the rib is a ridge-shaped protruding portion that extends parallel to the axis of the piston **210.** In the present embodiment, the piston **210** has two drive protruding portions **213** arranged on the second end **210b** side of the side surface of the body. The body **212** of the piston **210** has a female thread **214** located inside a hole **210h** extending along the axis of the piston **210.**

The piston drive mechanism **220** includes a motor **264,** a gearbox **221,** a drive gear **222,** a drive rod **235** and a piston guide **230.** The motor **264,** the gearbox **221,** the drive gear **222** and the piston guide **230** are supported by the internal housing **202.**

The motor **264** rotates forward or rotates reverse based on the control by the control device **280.** Herein, forward rotation refers to rotation in a direction that moves the piston **210** forward, and reverse rotation refers to rotation in a direction that moves the piston **210** backward.

The rotary encoder **265** is attached to the rotary shaft of the motor **264** as an amount of rotation detector, and the rotary encoder **265** detects the amount of rotation (number of revolutions) of the motor **264.** The rotary encoder **265** includes an encoder plate **266** and a pulse encoder **267** that includes a light-emitting element and a light-receiving element. The rotary encoder **265** will be described in detail below.

The gearbox **221** includes at least one gear attached to the rotary shaft of the motor **264.** The gearbox **221** may include two or more gears to reduce the rotational speed of the motor **264.**

The drive gear **222** meshes with the gear of the gearbox **221** and is rotatably supported by the bearing **223** in the internal housing **202.** A hole is provided in the shaft of the drive gear **222,** into which one end of the drive rod **235** is inserted and fitted.

The drive rod **235** has a rod shape and has a male thread **236** on the side surface. The male thread **236** is configured in terms of thread height, shape, thread pitch, etc., to engage with the female thread **214** provided on the piston **210.**

The piston guide **230** has a hole **230h** into which the piston **210** is inserted. A guide **231** is provided on the inner side surface of the hole **230h.** The guide **231** engages with the drive protruding portion **213** of the piston **210** and guides the piston **210** so that the piston **210** moves forward or backward without rotating around the axis.

In the present embodiment, the guide **231** is a straight groove extending parallel to the axis of the hole **230h,** into which a rib, being the drive protruding portion **213**, provided on the side surface of the piston **210** is inserted. The piston guide **230** includes two guides **231** corresponding to two drive protruding portions **213** of the piston **210**.

When the motor **264** rotates forward by an instruction of the control device **280**, the drive gear **222** rotates via the gearbox **221** and the drive rod **235** rotates. As the drive rod **235** rotates, the female thread **214** of the piston **210**, which meshes with the male thread **236** of the drive rod **235**, receives a rotational force around its axis. Since the drive protruding portion **213** of the piston **210** is inserted into the groove of the guide **231**, the rotation of the piston **210** about its axis is restricted by the guide **231**, and the piston **210** moves forward without rotation as the drive rod **235** rotates.

When motor **264** rotates reverse by an instruction of the control device **280**, the drive gear **222** rotates via the gearbox **221**, causing the drive rod **235** to rotate in the reverse direction. The male thread **236** of the drive rod **235** meshes with the female thread **214** of the piston **210**, regulating rotation around the axis, thereby causing the piston **210** to move backward without rotating around its axis.

<Control of piston drive mechanism **220**>

As described above, the viscosity of the liquid drug **14** varies depending on the temperature. Generally, the higher the temperature of the drug, the smaller the viscosity. Also, the viscosity varies depending on the type of drug. Moreover, even if the same drug is housed in a drug cartridge, the load required to move the gasket **13** may vary with different diameters of the cylinder **11** due to the variation of the capacity of the cylinder **11**. With conventional drug injection devices, in order to ensure smooth injection operation regardless of the type of drug and the capacity of the drug cartridge **10**, a sufficiently high drive voltage is supplied to the motor that can accommodate the most viscous drug among all compatible drugs, and the drug cartridge with the largest load among all compatible drug cartridges. However, this requires the use of a secondary battery with a large maximum output. This also increases the volume of the secondary battery, making the drug injection device larger and heavier. Since the motor is always driven by more electric power than necessary, the power consumption increases.

Conventional drug injection devices addressed the problem that injecting cold drug may cause pain by leaving the drug cartridge in the room for a certain period of time before the operator performs injection. Such a time management as to leave the drug cartridge for a certain period of time is troublesome for the operator, and since the temperature of the room at the time of injection can vary depending on the season, location, etc., the drug cartridge may not necessarily reach the same temperature even if it is left for the same amount of time.

In the present embodiment, the RF-ID reader **277** receives the drug information including information indicating the type of drug in the drug cartridge transmitted from the RF tag **16** of the drug cartridge and the first temperature information detected by the first temperature sensor, and the drive power of the motor based on the first temperature information is determined, and the motor driver is controlled so that the determined drive power is output. If the temperature indicated by the first temperature information is in a certain temperature range, it is determined that the temperature is not suitable for injection, and the injection operation is not started by not driving the motor **264**.

In order to perform such control, control information for controlling the motor **264** for each of the plurality of drugs is stored in the memory **254**. The control information for each drug includes a set of control parameters for the motor for each of the multiple temperature ranges.

Table 1 shows an example of a set of control parameters for the motor stored in the memory **254**. Drug A and Drug B are shown as examples of the plurality of drugs. The viscosity of Drug B is higher than that of Drug A. Also shown are a plurality of temperature ranges: less than 0°C, 0°C or more and less than 10°C, 10°C or more and less than 20°C, 20°C or more and less than 38°C, and 38°C or more. 0°C, 38°C and 10°C are examples of the first temperature, the second temperature and the third temperature, respectively. When the drug cartridge **10** is not inserted in the cassette **100**, the RF-ID reader **277** is unable to obtain either the drug information or the first temperature information. The memory **254** may further include a set of control parameters for the motor in the absence of such a drug cartridge.

[Table 1]

| Type of drug cartridge | Motor control parameters | | | | |
|---|---|---|---|---|---|
| | less than 0°C | 0°C or more less than 10°C | 10°C or more less than 20°C | 20°C or more less than 38°C | 38°C or more |
| Drug A | Drive information C | Drive information A1 | Drive information A2 | Drive information A3 | Drive information C |

(continued)

| Type of drug cartridge | Motor control parameters | | | | |
|---|---|---|---|---|---|
| Drug B | Drive information C | Drive information B1 | Drive information B2 | Drive information B3 | Drive information C |
| No drug cartridge | Drive information C | | | | |

If the first temperature information is less than 0°C, the drug may possibly be frozen. If the first temperature information is 38°C or more, the drug may possibly be altered by being stored at a high temperature. If the first temperature information is in these two temperature ranges, it is not desirable to perform injection. Therefore, drive information C is assigned regardless of whether the drug type of the drug information is A or B. Drive information C may be, for example, information that the motor **264** is not to be driven. If the drug cartridge is not inserted into the cassette **100,** injection cannot be performed with any temperature range, so drive information C is assigned with all temperature ranges. In the temperature ranges of 0°C or more and less than 10°C, 10°C or more and less than 20°C, and 20°C or more and less than 38°C, sets of control parameters of drive information A1, A2, A3 and drive information B1, B2, B3 are assigned for drug A and drug B, respectively.

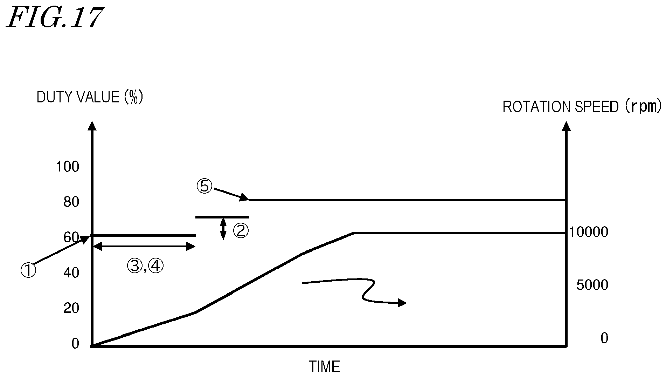

Table 2 shows a specific example of sets of control parameters of drive information A1, A2, A3 and drive information B1, B2, B3. The motor drive power may be adjusted, for example, by adjusting the voltage to be applied or by other means. In the present embodiment, the drive power is adjusted by PWM (pulse width modulation) to control the motor. FIG. **17** shows an example of a drive signal by PWM and the rotation speed of the motor that rotates by the drive signal. Drive information that defines the drive signal by PWM includes, for example, the duty ratio initial value, the duty ratio increment, the increase period, the increase maximum wait time and the target-achieving duty ratio.

For all of the temperature ranges, the viscosity of drug B is higher than that of drug A. Therefore, comparison between the temperature ranges shows that the initial value of the duty ratio initial value is greater for drug B than for drug A. This is because more electric power is needed to drive a drug with higher viscosity. In the present embodiment, the increase period and the increase maximum wait time are shorter for drug B than for drug A, and the target duty ratio is greater for drug B than for drug A for the temperature ranges.

For each drug, the values of the parameters are smaller for higher temperature ranges. That is, the higher the lower limit value of each temperature range, the smaller the duty ratio initial value, the duty ratio increment, the increase period, the increase maximum wait time and the target-achieving duty ratio become. This is because the higher the temperature of the drug, the smaller the viscosity and the lower the electric power required to drive the drug.

[Table 2]

| Type of drug | Drug A | | | Drug B | | |
|---|---|---|---|---|---|---|
| Drive information | A1 | A2 | A3 | B1 | B2 | B3 |
| (1) Duty ratio initial value (%) | 70 | 65 | 60 | 80 | 75 | 70 |
| (2) Duty ratio increment (%) | 5 | 4 | 3 | 4 | 3 | 2 |
| (3) Increase period (Encoder pulse count) | 5 | 4 | 3 | 4 | 3 | 2 |
| (4) Increase maximum wait time (ms) | 30 | 25 | 20 | 25 | 20 | 15 |
| (5) Target duty ratio (%) | 90 | 85 | 80 | 100 | 95 | 90 |

For example, if the drug information is drug A and the first temperature information is 15°C, the control device **280** reads out drive information A2 from the memory **254** and drives the motor driver **263** using the parameters included in drive information A2.

Regardless of which drive information is selected from among A1 to A3 and B1 to B3 as shown in Table 2 and FIG. **17,** the control device **280** causes the motor driver to generate drive signals so that the current flowing to the motor increases in steps as the duty ratio increases in steps. When the duty ratio reaches the target duty ratio, the drive then continues at the target duty ratio. Thus, when starting the motor, the peak value (current overshoot) of the starting (start-up) current can be lowered by performing PWM control with an initial value of duty ratio in accordance with the viscosity of the drug and so that the duty ratio increases in steps.

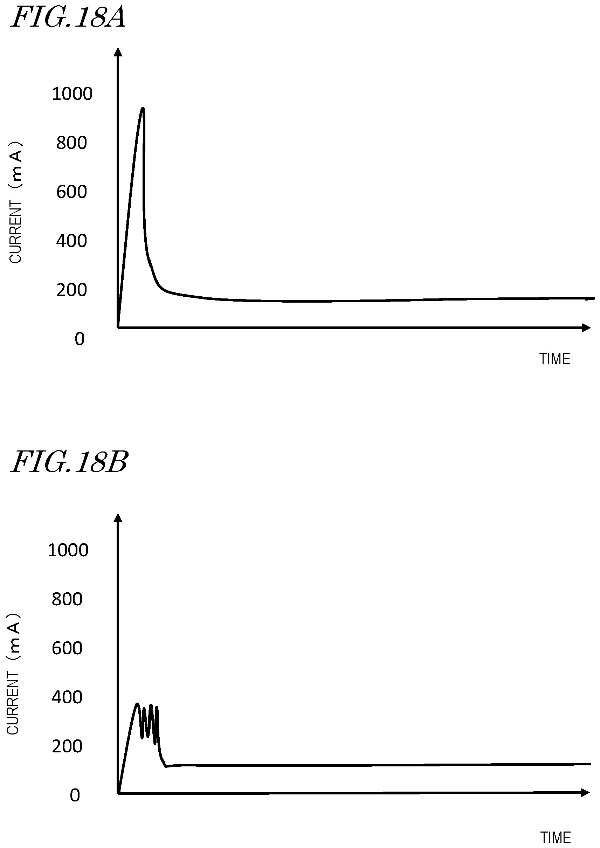

FIG. **18A** shows the starting current that occurs when PWM control is not performed, and FIG. **18B** shows an example of the starting current with PWM control in the present embodiment. As shown in FIG. **18A,** when PWM control is not used, a peak current of about 800 mA at maximum flows. In contrast, according to the present embodiment, the peak current can be suppressed to about 400 mA.

Using an initial value of duty ratio according to the viscosity of the drug contributes particularly to reducing the amount of time until the motor **264** reaches a steady state. Even if the initial value of duty ratio is not changed according to the viscosity of the drug, the overshoot of the current at startup can be suppressed by driving the motor **264** using a pulse signal such that the duty ratio increases in steps. However, if an appropriate initial value of duty ratio according to the viscosity is not used, the amount of time required for the gasket of the drug cartridge **10** to start and move at a constant speed may become very long. This may increase the amount of time required for injection, making it impractical or placing a greater burden on the operator.

In the present embodiment, by setting the parameters of the overall PWM control, such as the duty ratio initial value, etc., taking into account the difference in viscosity due to the type of drug and the difference in viscosity due to temperature, the starting current can be reduced while preventing the amount of time until the motor **264** reaches a steady state from becoming long. This allows the maximum discharge current to be reduced, making it possible to reduce the capacity of the secondary battery. Thus, it is possible to realize a drug injection device that can perform injection with an appropriate drive force even with a smaller-capacity secondary battery. With the drug injection device of the present embodiment, the battery capacity can be reduced by about 20% to 30%, for example, as compared to the case where the drive power is not adjusted. Therefore, it is possible to realize a smaller and lighter drug injection device, which is easier to handle and provides excellent operability.

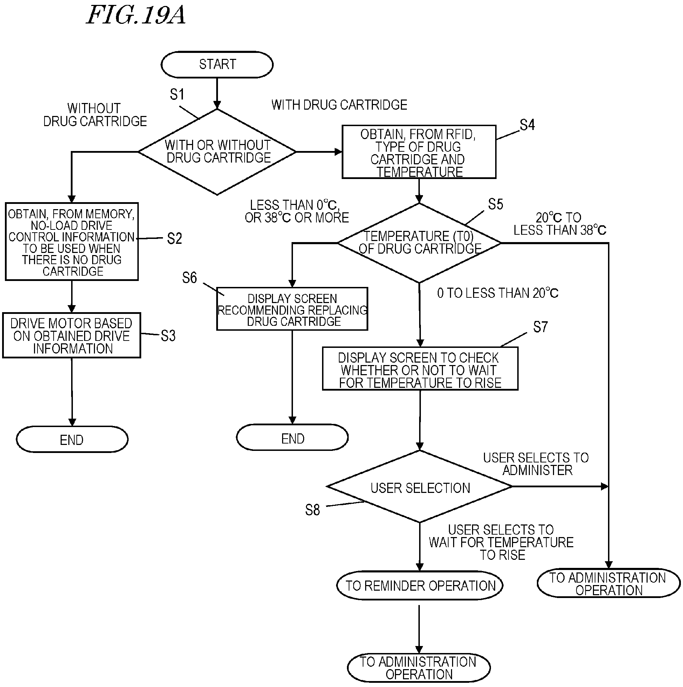

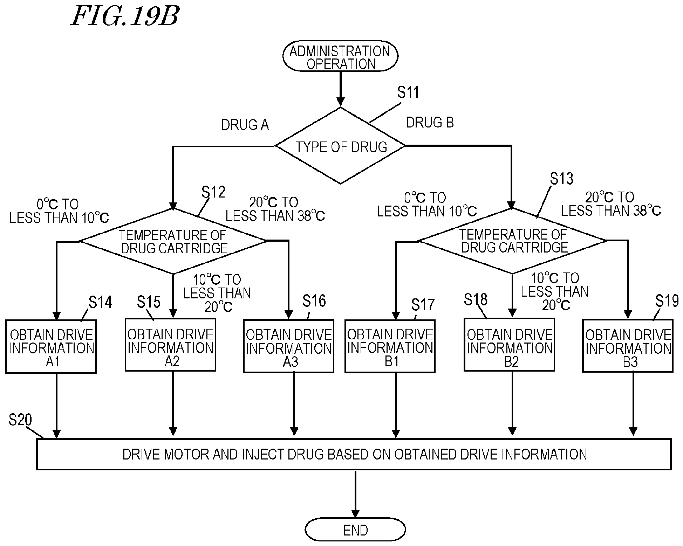

As described above, the first temperature information transmitted from the RF tag **16** of the drug cartridge **10** can be used also to determine whether the temperature is suitable for injection. Referring now to FIG. **19A** and FIG. **19B,** an example of an injection operation in which the first temperature information is used also to determine whether the temperature is suitable for injection will be illustrated. FIG. **19A** and FIG. **19B** are flow charts illustrating the operation of the drug injection device **200** during an injection operation. Referring to FIG. **5** and these figures, the injection operation will be described.

When the operator loads the cassette **100** in the drug injection device **200,** the control device **280** causes the transmission/reception circuit of the RF-ID reader **277** to transmit a signal to generate an electromotive force to cause the RF tag **16** to transmit information (S1). If the RF-ID reader **277** cannot receive the signal from the RF tag **16,** it means that the drug cartridge **10** is not inserted in the cassette **100.** In this case, the control device **280** reads drive information C from the memory **254** (S2), and drives the motor **264** with drive information C (S3). Drive information C is, for example, information that controls the motor driver **263** so as to maintain the state where the motor **264** is stopped.

When the RF-ID reader **277** receives a signal from the RF tag **16** (S4), the control device **280** stores the drug information transmitted from the RF tag **16** of the drug cartridge **10,** including information indicating the type of drug in the drug cartridge **10,** and the first temperature information detected by the first temperature sensor, as T0, in memory **254.** T0 is used also as the initial temperature to estimate the amount of time until the drug reaches an appropriate temperature as will be described below.

The control device **280** compares the temperature indicated by the obtained first temperature information with the reference temperature (S5). The reference temperatures are 0°C (first temperature), 20°C (third temperature) and 38°C (second temperature). If the temperature indicated by the first temperature information is less than 0°C or if it is 38°C or more (first temperature range), the drug in the drug cartridge **10** may be frozen or altered due to the high temperature. Therefore, for example, the control device **280** causes the display device **259** to display a screen prompting to replace the drug cartridge **10** (S6), and ends the drug injection operation.

If the temperature indicated by the first temperature information is 0°C or more and less than 20°C (second temperature range), the temperature of the drug is low and pain may possibly be felt during injection. Therefore, for example, the control device **280** causes the display device **259** to display a screen prompting to wait for the temperature of the drug to rise before injection (S7). In this case, the operator may decide that he/she wants to inject the drug as soon as possible even if he/she may feel some pain, and therefore an input on whether to select injection or to wait is accepted (S8). If the operator selects to wait, the process proceeds to the flow of a reminder operation. On the other hand, if the operator selects injection, the process proceeds to the flow of an administration operation.

If the temperature indicated by the first temperature information is 20°C or more and less than 38°C (third temperature range), injection can be performed. Therefore, the process proceeds to the flow of an administration operation.

As shown in FIG. **19B,** in the flow of the administration operation, the control device **280** first determines whether the drug information is drug A or drug B (S11). If the drug information is drug A, the first temperature information is compared with the reference temperature (S12), and drive information according to the comparison result is read out from the memory **254.** As shown in Tables 1 and 2, drive information A1 is read from the memory **254** if the temperature indicated by the first temperature information is 0°C or more and less than 10°C, drive information A2 is read from the memory **254** if the temperature is 10°C or more and less than 20°C, and drive information A3 is read from the memory **254** if the temperature is 20°C or more and less than 38°C (S14, S15, S16). Similarly, when the drug information is drug B, the first temperature information is compared with the reference temperature (S13), and drive information according to the comparison result is read out from the memory **254**. Drive information B1 is read out from the memory **254** if the temperature indicated by the first temperature information is 0°C or more and less than 10°C, drive information B2 is read out from the memory **254** if the temperature is 10°C or more and less than 20°C, and drive information B3 is read out from the memory **254** if the temperature is 20°C or more and less than 38°C (S17, S18, S19) .

The control device **280** controls the motor driver **263** using the parameters of the selected drive information to rotate the motor **264** by PWM control as described above.

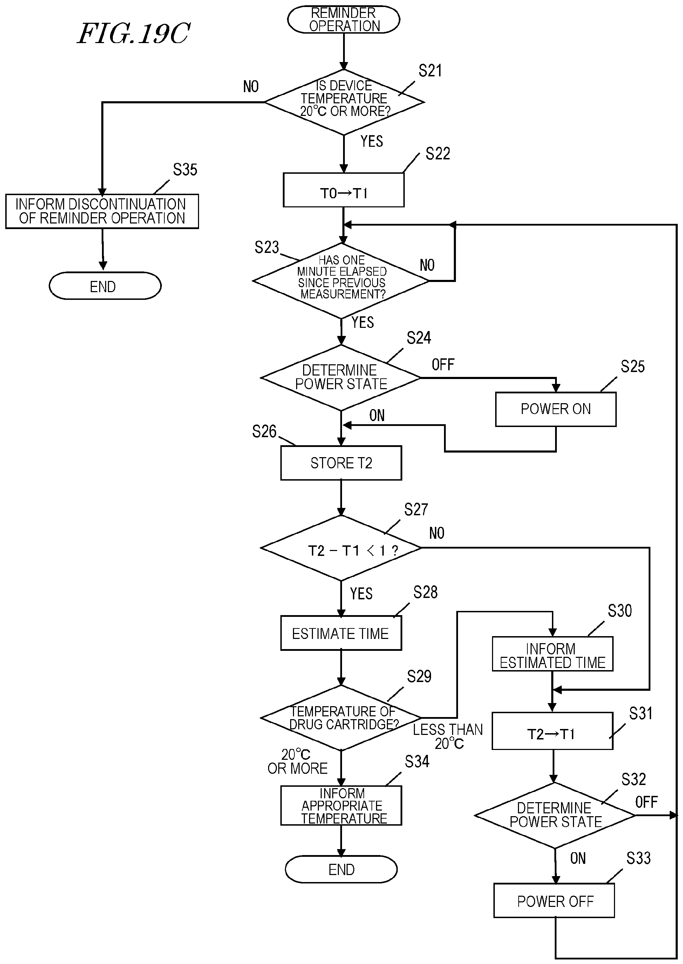

Next, the reminder operation is described. If the temperature of the drug is low and the operator selects to wait for the temperature of the drug to reach the appropriate temperature before injection, the control device **280** of the drug injection device **200** performs the reminder operation. FIG. **19C** is a flow chart showing an example of the reminder operation.

In the reminder operation, the control device **280** successively receives the first temperature information from the first temperature sensor **15** attached to the drug cartridge **10** and informs when the drug temperature reaches the appropriate temperature. The present embodiment further estimates the amount of time required to reach the appropriate temperature and informs of the estimated time. For this, the control device **280** receives the second temperature information from the second temperature sensor **273** and successively obtains the first temperature information at predetermined time intervals when the second temperature information is equal to or greater than a predetermined temperature. Based on the obtained first temperature information and second temperature information, the control device **280** successively calculates the estimated time until it is possible to perform injection, and controls the display device to display information indicating the calculated estimated time.

Specifically, first, the control device **280** receives the second temperature information from the second temperature sensor **273** and determines whether the second temperature information is 20°C or more (S21). The second temperature information output by the second temperature sensor **273** directly indicates the temperature inside the device housing **201.** However, if the second temperature sensor **273** is less susceptible to heat generated by components, etc., in the device housing **201,** the second temperature information is substantially identical to the environmental temperature at which the drug injection device **200** is used. Since the temperature of the drug rises to room temperature by taking the drug cartridge **10** out of a refrigerator, or the like, and keeping it in a room, if the room temperature is less than 20°C, it is difficult for the temperature of the drug to reach 20°C, i.e., the temperature suitable for injection, even when the drug cartridge **10** is kept in the room. Therefore, if the second temperature information is less than 20°C, for example, the control device **280** causes the display device **259** to display a screen informing that the room temperature is too low for injection (S35), and ends the reminder operation.

If the second temperature information is 20°C or more, the control device **280** stores, as T1, temperature T0 of the drug cartridge that is first obtained (S22). The control device **280** adjusts the timing for obtaining the first temperature information. If one minute has elapsed since the first temperature information is obtained in the previous iteration or for the first time, the process proceeds to the next step (S23).

The control device **280** determines the status of the power of the drug injection device **200** (S24). If the power is OFF, e.g., due to auto power-off, the power is turned ON (S25), and the process proceeds to the next step. If the power ON, the process proceeds directly to the next step.

The control device **280** obtains the first temperature information and stores it as the current measured value T2 (S26). Then, the difference between T2 and T1, which is the measured value of the first temperature information in the previous iteration (one time before) is determined (S27). If the difference between T2 and T1 is 1 or more, it means the temperature of the drug is increasing rapidly and it is difficult to appropriately estimate the amount of time required for the drug to reach the appropriate temperature. Therefore, the process waits until a certain amount of time elapses again. Specifically, first, the latest first temperature information T2 is stored as T1 (S31), and the power status is determined (S32). If in auto power mode, the process turns OFF the power (S33) and returns to the step of waiting for one minute (S23). If not in auto power mode, the process does not change the power status and returns to the step of waiting for one minute (S23) .

If the difference between T2 and T1 is less than 1 (S27), the control device **280** calculates how much time is required for the temperature of the drug cartridge **10,** in other words the temperature of the drug, to reach the appropriate temperature.

According to in-depth study by the present inventors, when the drug cartridge **10** that has been stored at a low temperature is held at the environmental temperature at which injection is performed, such as being stored in the room, the rate of temperature rise immediately after can vary depending on the size of the drug cartridge **10** and the amount of drug being held. However, it has been found that once the rate of temperature rise is decreases, the rate of temperature rise thereafter stays approximately the same, regardless of the size of the drug cartridge **10** and the amount of drug.

More specifically, it has been found that when the difference between T2 and T1 is less than 1°C, the rate of temperature rise thereafter stays substantially the same regardless of the size of the drug cartridge **10** and the amount of drug, and the temperature of the drug can be approximated by the time elapsed since the time when the initial temperature TO was obtained.

For example, if the difference between T2 and T1 is less than 1°C, the temperature f(x) of the drug estimated from the elapsed time, where x denotes the elapsed time, is indicated by the following function, depending on the temperature range of the second temperature information, which can be said to be the environmental temperature.

(1) When the second temperature information is 25°C or more and less than 30°C:
[Expression 1]

$$f(x) = 0.00005x^3 0.04x^2 + 1.3x + 1.6 \qquad \text{Equation (1)}$$

(2) When the second temperature information is 20°C or more and less than 25°C:
[Expression 2]

$$f(x) = 0.00002x^3 0.025x^2 + 0.96x + 1.6 \qquad \text{Equation (2)}$$

For example, if the second temperature information is 25°C, the temperature of the drug after the time when the difference between T2 and T1 is less than 1°C using Equation (1) can be estimated using the elapsed time. When the difference between T2 and T1 is less than 1°C, the elapsed time from the time when the initial temperature TO is obtained is denoted as $t_n$. Where $t_{n+1}$, $t_{n+2}$, $t_{n+3}$, ..., $t_{n+i}$ denote every minute elapsed from $t_n$, and $T2_n$ denotes the first temperature information T2 at $t_n$, the estimated drug temperature T(i) at i minutes after $t_n$ is given by the following equation.
[Expression 3]

$$T(i) = T2_n + (f(t_{n+i}) - f(t_n)) \qquad \text{Equation (3)}$$

If the temperature of the drug suitable for injection is, for example, 20°C or more, the control device **280** uses the elapsed time $t_n$ when the difference between T2 and T1 is less than 1 and Equation (1) to determine $f(t_n)$. Also, $f(t_{n+1})$ is determined where i=1. From these two values and the first temperature information $T2_n$, T(1) is determined. If T(1) is less than 20°C, the control device **280** similarly performs the calculation while increasing i by 1 until T(i) is 20°C or more. When T(i) is 20°C or more, the control device **280** uses i at that time as the estimated time.

The control device **280** determines whether the first temperature information $T2_n$ at this time (the present time) is less than 20°C (S29), and if it is less than 20°C, the control device **280** causes the display device **259** to display a screen informing of the estimated time, i.e., that the drug will reach the appropriate temperature after i minutes (S30). How the estimated time is informed is not limited to the display device **259,** but the control device **280** may transmit the estimated time to the operator's portable device, such as a smartphone or a tablet device, using the communication section **262,** and the portable device may perform the informing function such as by displaying.

Then, the latest first temperature information T2 is stored as T1 (S31), and the power status is determined (S32). If in auto power mode, the process turns OFF the power (S33) and returns to the step of waiting for one minute (S23). If not in auto power mode, the process does not change the power status and returns to the step of waiting for one minute (S23) . Moreover, the process repeats steps S23 to S29 to estimate the amount of time required to reach the appropriate temperature. In order to estimate the amount of time required to reach the appropriate temperature for the second and subsequent times, the elapsed time $t_n$ since the time at which the initial temperature TO is obtained and the first temperature information $T2_n$. By successively using the newest measured value, i.e., the first temperature information $T2_n$, for time estimation, it is possible to make more accurate estimation.

For time estimation for the second and subsequent times, the control device **280** determines whether the first temperature information $T2_n$ is less than 20°C (S29), and if it is 20°C or more, the control device **280** causes the display device **259** to display a screen indicating that the appropriate temperature has been reached (S34), and the reminder operation is ended. Then, the process proceeds to the administration operation, as shown in FIG. **19A.**

Table 3 shows an example calculation. Assume that the difference between T2 and T1 becomes less than 1°C after 5 minutes (n=5) from the time when the initial temperature TO is obtained, and the first temperature information $T2_5$ at this time is 13.45°C.

[Table 3]

| t | i | $T2_5$ | $f(t_5)$ | $f(t_{5+i})$ | $T(i)$ |
|---|---|--------|----------|--------------|--------|
| 5 | 0 | 13.45 | 7.1625 | 7.1625 | 13.45 |
| 6 | 1 | 13.45 | 7.1625 | 8.068 | 14.3555 |
| .... | | .... | .... | .... | |
| 14 | 9 | 13.45 | 7.1625 | 13.332 | 19.6195 |
| 15 | 10 | 13.45 | 7.1625 | 13.7875 | 20.075 |

The estimated drug temperature at i=1, i.e., after elapse of one minute since this point in time (after six minutes since obtaining the initial temperature TO), is 14.3555°C. Since the estimated drug temperature is 20.075°C, which is 20°C or more, at i=10, it is determined that the estimated time to reach the appropriate temperature is 10 minutes after. As described above, if the time estimation is calculated again after one minute, the measured value $T2_6$ is used.

Thus, according to the present embodiment, the operator can decide how to use the time until the drug reaches the appropriate temperature, since the operator is informed how many more minutes it will take for the drug to reach the appropriate temperature and be ready for injection.

Note that, in the present embodiment, the estimated time until the drug reaches the appropriate temperature is calculated in the reminder operation, but the estimated time may not need to be calculated and informed. That is, the first temperature information may be obtained at predetermined time intervals, and when the first temperature information becomes equal to or greater than the predetermined temperature, it may be informed that the drug has reached the appropriate temperature and is ready for injection.

<Touch sensor **275**>

As shown in FIG. **1** and FIG. **13,** the touch sensor **275** is arranged on the skin-contact surface **201e** of the protruding portion **201t** of the drug injection device **200.** Thus, it is possible to detect the state where the skin-contact surface **201e** is reliably in contact with the skin, and to detect an inappropriate holding state such as wobbling of the drug injection device **200** before the needle **22** comes completely off the skin during injection. The touch sensor **275** will now be described in detail.

The touch sensor **275** is a touch sensor of a surface charge transfer type. The touch sensor **275** includes a sensor capacitor Cx arranged on the skin-contact surface **201e,** a sampling capacitor Cs and a control circuit built in the control device **280.** The control circuit of the touch sensor **275** first resets the touch sensor **275** by discharging the sensor capacitor Cx and the sampling capacitor Cs. Then, the control circuit of the touch sensor **275** charges the sensor capacitor Cx and transfers the charge charged in the sensor capacitor Cx to the sampling capacitor Cs. This charging and transferring operation is repeated to successively accumulate charge in the sampling capacitor Cs. The control circuit monitors the voltage of the sampling capacitor Cs, and when the voltage exceeds a predetermined value, the control circuit outputs the transfer count N representing the number of repetitions up to that point. The transfer count N is inversely proportional to the capacitance of the sensor capacitor Cx, and as the capacitance of the sensor capacitor Cx increases, the transfer count N decreases. When the skin-contacting surface **201e** of the protruding portion **201t** of the drug injection device **200** is in contact with the skin of the operator, etc., the capacitance of the sensor capacitor Cx increases, so the charge accumulated in the sensor capacitor Cx also increases and the amount of charge transferred to the sampling capacitor Cs in a single transfer operation also increases. Therefore, the transfer count N until the voltage of the sampling capacitor Cs exceeds a predetermined value decreases.

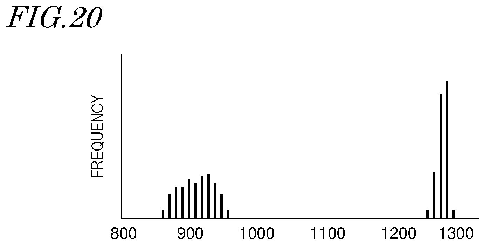

FIG. **20** shows an example of the results of checking the detection sensitivity of the touch sensor **275** using the drug injection device **200.** In FIG. **20,** the result of the value when N is about 900 times indicates the result when the skin-contact surface **201e** is in contact with the skin, and the result of the value when N is about 1300 times indicates the result when the skin-contact surface **201e** is not in contact with the skin. FIG. **20** shows that there is a large difference between the transfer count N when the skin-contact surface **201e** is in contact with the skin and the transfer count N when it is not in contact with the skin, and that it is possible to clearly determine whether the skin is in contact by using the transfer count N. In the example described above, for example, if the transfer count is 1050 or less, it may be determined that it is in contact with the skin, and if the transfer count is 1100 or more, it may be determined that it is off the skin.

The time required for one transfer operation in a touch sensor of a surface charge transfer type is from 0.5 microsecond to 4 microseconds, for example, and the time required for the voltage of the sampling capacitor Cs to exceed a predetermined value is from about 2 ms to about 8 ms. The area of the skin-contact surface **201e** is relatively small, and depending on the area where the injection is to be performed, it may be difficult to stabilize the attitude in which the drug injection device **200** is held during injection, and therefore when skin contact is detected five to eight times in a row, for example, it may be determined that the drug injection device **200** is correctly held and it is possible to perform injection. For example, if the touch sensor **275** detects a transfer count of 1050 or less for 5 consecutive times, it may be determined that the touch sensor **275** is detecting skin contact, and otherwise it may be determined that the touch sensor **275** detects separation from the skin.

A touch sensor of a surface charge transfer type is superior to conventional touch sensors of an oscillation type in that it is less susceptible to noise. Therefore, it is possible to detect that the skin is in contact with the skin-contact surface **201e** more reliably than with conventional methods, and it may be possible to realize safer and more reliable handling of the drug injection device.

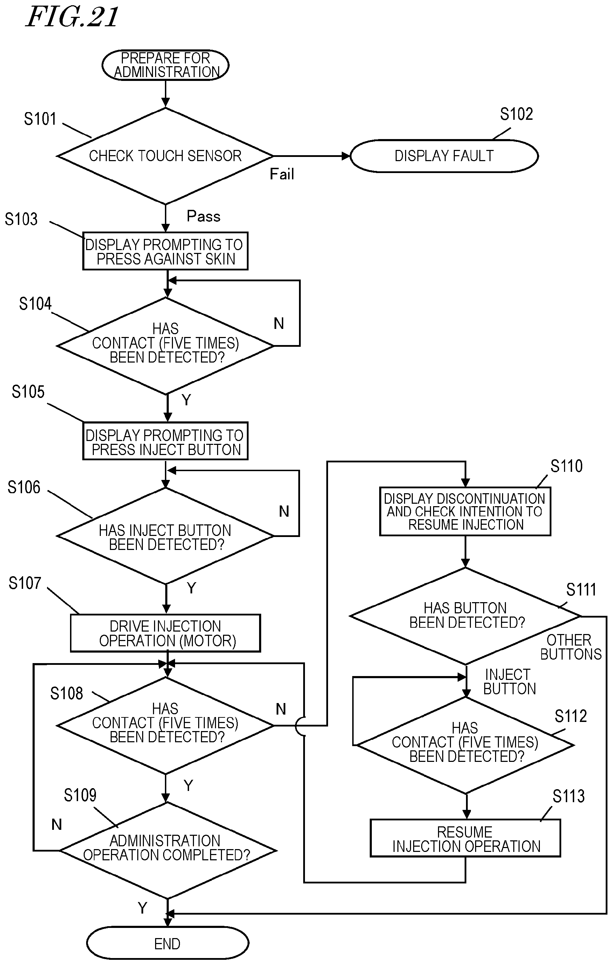

FIG. **21** is a flow chart illustrating the injection operation of the drug injection device **200** using the touch sensor **275.** As shown in FIG. **21,** after the start of the injection preparation operation, the control device **280** checks whether or not the touch sensor **275** is connected, etc., by the control circuit of the touch sensor **275** (S101), and if it is not connected, the control device **280** causes the display device **259** to display a fault or abnormality (S102).

If the touch sensor **275** is correctly connected, the display device **259** displays an image, text or other information that prompts the user to press the skin-contact surface **201e** against the skin and hold the drug injection device **200** ready for injection (S103). The control device **280** successively receives detection signals from the control circuit of the touch sensor **275.** For example, when a detection signal that indicates a transfer count of 1050 or less is received five consecutive times (hereinafter referred to as detecting skin contact, S104), the control device **280** causes the display device **259** to display information that prompts to press the inject button **258** (S105).

When the control device **280** detects a signal that the injection button **258** has been pressed (S106), the control device **280** controls the motor driver **263** so as to perform the injection operation described above, i.e., to inject the drug (S107). During the drug injection operation, the control device **280** receives successive detection signals from the control circuit of the touch sensor **275** in parallel. If skin contact cannot be detected, that is, if the touch sensor **275** detects separation from skin (S108), the control device **280** interrupts the injection operation and causes the display device **259** to display information indicating that the drug injection device **200** has come off the injection site and information that prompts to enter whether to discontinue or continue the injection (S110).

When the control device **280** detects a signal that the injection button **258** has been pressed (Sill), the control device **280** confirms that the touch sensor **275** has detected skin contact (S112) and returns to injection operation (S113). If the operator makes an input to discontinue the injection (e.g., an input of the OK button **257**), the control device **280** ends the injection operation.

The end of the injection operation is determined by the control device **280** based on the number of pulses or the number of revolutions detected by, for example, the rotary encoder **265** (S109), ending the injection operation.

<Acceleration sensor **276**>

The drug injection device **200** of the present embodiment includes the acceleration sensor **276.** Thus, it is possible to detect the attitude of the drug injection device **200** and to prompt the operator to change the attitude of the drug injection device **200.**

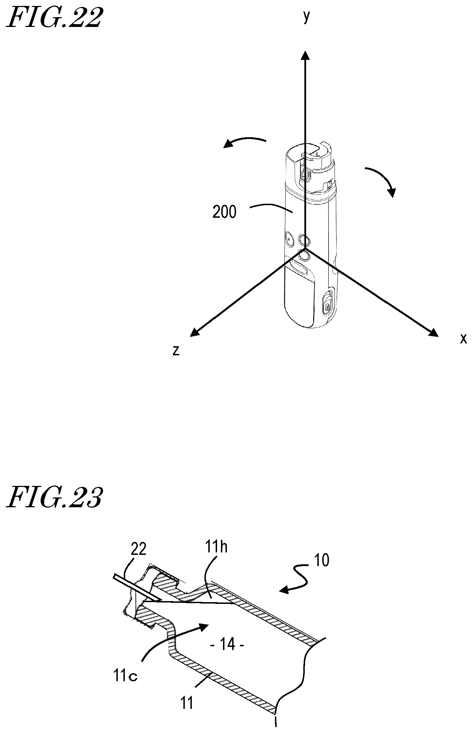

As shown in FIG. **13,** the acceleration sensor **276** can be arranged on the main board **290,** for example. Preferably, the acceleration sensor **276** has first, second and third axes that are orthogonal to each other, and can detect acceleration along the axes or angular acceleration around the axes. It is also preferred that one of the first, second and third axes is arranged in the housing so that it coincides with the direction of movement of the piston. In the present embodiment, the acceleration sensor **276** detects acceleration along the first, second and third axes. For example, as shown in FIG. **22,** in the drug injection device **200,** the positive direction of the y axis coincides with the direction of movement of the piston **210** and the forward direction of the piston, and the direction of the normal vector of the plane on which the display device **259** is arranged is the z axis. Then, the x axis coincides with the direction of the normal vector of the right side surface of the drug injection device **200** when held in the upward orientation. The x axis, the y axis and the z axis are fixed to the drug injection device **200.** Since the acceleration sensor **276** can detect gravitational acceleration along the x axis, the y axis and the z axis, it is possible to detect the attitude of the drug injection device **200,** e.g., the tilt relative to the vertical direction of the y axis.

The result of such an attitude detection can be used for various operations and movements of the drug injection device **200.** For example, when performing injection, it is preferred to perform air removal (also known as emptying) to remove air from inside the drug cartridge **10** in advance. This operation is performed by moving the piston **210** with the needle **22** pointing upward to expel air staying in an upper portion the drug cartridge **10** through the needle **22.** However, if the distal end of the drug injection device **200** is tilted instead of pointing straight up, as shown in FIG. **23,** air may stay in a corner **11h** of the cylinder columnar space **11c,** where air is difficult to escape, depending on the shape of the cylinder columnar space **11c** of the drug cartridge **10.** In such a case, the acceleration sensor **276** can be used to guide the operator to hold the drug injection device **200** in such an attitude that is suitable for air removal.

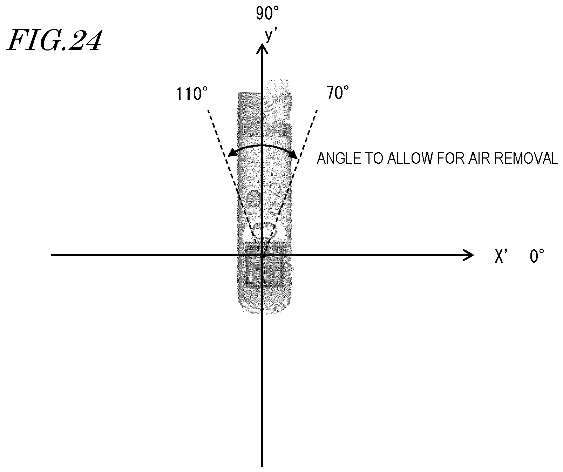

How much the drug injection device **200** should be held in an upright position without tilting when performing air removal depends on the cylinder columnar space **11c** of the drug cartridge **10.** As generally shown in FIG. **24,** in coordinates where the vertical direction is fixed as the y' axis, if the y axis of the drug injection device **200** is within ±70° from the vertical direction, i.e., in the range of 70° or more and 110° or less relative to the x' axis, it is considered that the air can substantially be correctly expelled without the operator feeling difficulty in adjusting the attitude (adjusting the direction).

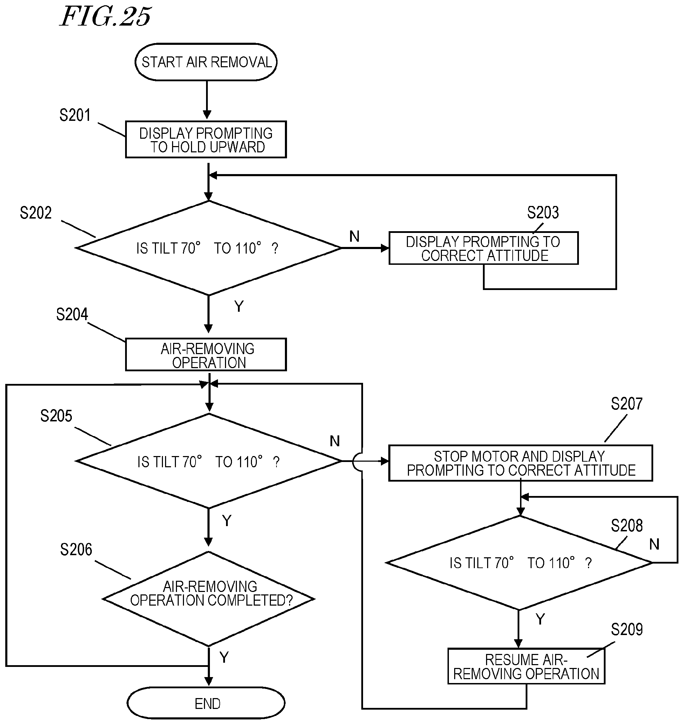

FIG. **25** shows a flow chart of an air-removing operation using the acceleration sensor **276.** When the operator selects the air-removing operation, or when the control device **280** performs the air-removing operation after the operator inputs an instruction to perform injection, the control device **280** causes the display device **259** to display information that prompts the operator to hold the drug injection device **200** upward (information about the attitude of the drug injection device) (S201). The control device **280** receives the detection signal from the acceleration sensor **276,** specifically, the acceleration information in the x-, y- and z-axis directions, calculates the angle of tilt of the y axis from the horizontal direction, and makes a determination regarding the tilt (S202). If the angle of tilt is an angle other than 70° to 110°, the drug injection device **200** is tilted, and the control device **280** causes the display device **259** to display information that indicates that the drug injection device **200** is tilted and prompts the operator to correctly hold the drug injection device **200** upward (S203).

If the calculated angle is 70° to 110°, the control device **280** controls the motor driver **263** to drive the motor **264** to move the piston **210** forward to start the air-removing operation (S204).

While the piston is moving, the control device **280** successively receives the detection signal from the acceleration sensor **276** and makes a determination regarding the tilt (S205). If the angle of tilt becomes an angle other than 70° to 110°, the control device **280** stops driving the motor **264** and causes the display device **259** to display information that indicates that the drug injection device **200** is tilted and prompts the operator to correctly hold the drug injection device **200** upward (S207). Moreover, when the angle of tilt becomes 70° to 110° (S208), the drive of the motor **264** is restarted (S208).

The end of the air-removing operation is determined by the control device **280,** for example, based on the number of pulses or the number of revolutions detected by the rotary encoder **265** (S206), ending the air-removing operation.

Thus, by using the acceleration sensor **276** to detect the attitude of the drug injection device **200,** information regarding the more appropriate handling of the drug injection device **200** can be presented to the operator.

As described above, the drug injection device **200** of the present embodiment is also compatible with the drug cartridges **10'** containing drug in which the liquid component and the solid component are held separately in an unused state. When using the drug cartridge **10',** the liquid component **14A** and the solid component **14B** are brought into contact with each other before use, as described above with reference to FIG. **9A,** FIG. **9B,** FIG. **10(a)** and FIG. **10(b).** However, simply bringing the liquid component **14A** and the solid component **14B** into contact may not cause the solid component **14B** to dissolve immediately into the liquid component **14A.** Therefore, it is preferred for the operator to move the drug injection device **200** to mix (stir) the liquid component **14A** and the solid component **14B.** The detection of the attitude of the drug injection device **200** by the acceleration sensor **276** is also suitable for performing such a mixing operation. The drug mixing operation using the acceleration sensor **276** will now be described.

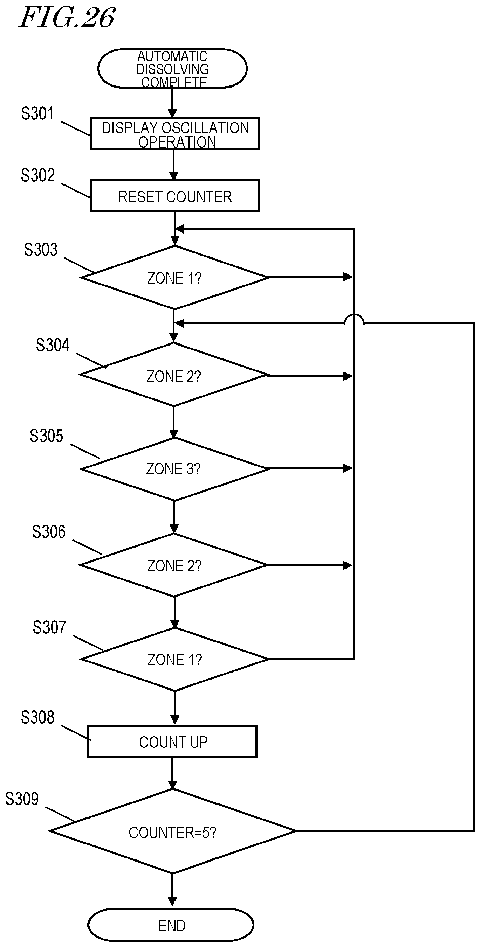

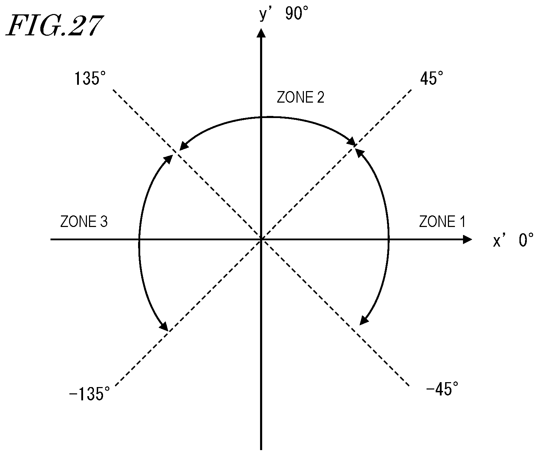

FIG. **26** is a flow chart of the mixing operation. To move the drug, one may consider oscillating the drug injection device **200** up and down, for example. However, such an operation may cause the liquid component **14A** to foam easily, making it impossible to perform injection immediately after mixing. In addition, moving the drug injection device **200** up and down may possibly cause the drug injection device **200** to hit furniture such as a desk, which is undesirable. In the present embodiment, in order to slowly mix the drug without foaming, the drug injection device **200** is tilted left and right as indicated by arrows in FIG. **22.** The attitude of the drug injection device **200** is indicated by the angle of the y axis of the drug injection device **200** shown in FIG. **22** on the x'y' coordinates shown in FIG. **27.** The x'y' coordinates are fixed regardless of the attitude of the drug injection device **200.** As shown in FIG. **27,** it is represented by ±180° with 0° being the positive direction of the x' axis. As shown in FIG. **27,** the range from -45° to 45° is defined as Zone 1, the range from 45° to 135° as Zone 2, and the range from -180° to -135° or from 135° to 180° as Zone 3.

The control device **280** determine that the operator is correctly oscillating the drug injection device **200** and mixing the drug by determining that the angle of the y axis of the drug injection device **200,** as calculated using the acceleration sensor **276,** falls within the range of Zone 1, the range of Zone 2 and the range of Zone 3 in a predetermined order.

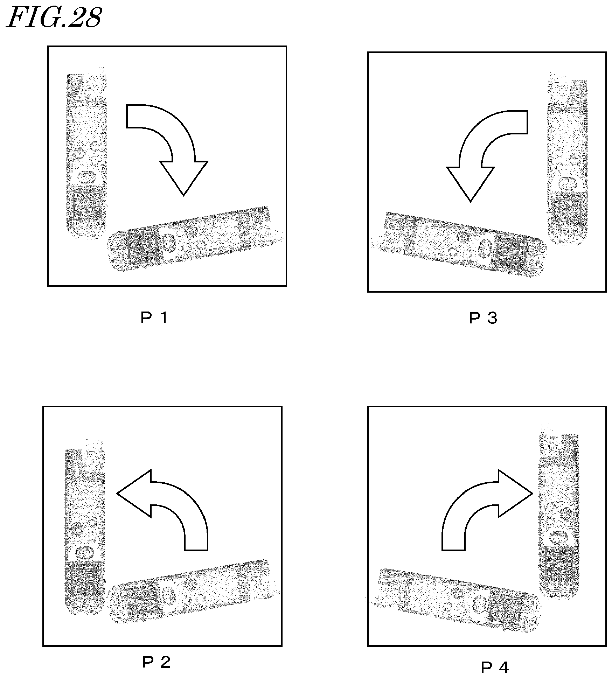

As shown in FIG. **26,** first, after bringing the liquid component **14A** and the solid component **14B** into contact before use (automatic dissolving operation), the control device **280** causes the display device **259** to display information that prompts the operator to perform the oscillating operation described above (S301). Then, the counter is reset to count the number of oscillations (S302). The operator tilts the drug injection device **200** left and right in the order of P1, P2, P3, P4 and P1, as shown in FIG. **28**. In this operation, the control device **280** receives a detection signal from the acceleration sensor **276** and successively determines that the y-axis tilt is in the following ranges: Zone 1 (-45° to 45°) (S303), Zone 2 (45° to 135°) (S304), Zone 3 (-180° to -135° or 135° to 180°) (S305), Zone 2 and Zone 1 in this order.

Then, the control device **280** increments the count by one and determines whether the number has reached a specified value (S309). For example, the specified value is five, and if the count is less than or equal to the specified value, the detection operation described above is repeated (S303 to S308).

If it does not fall within the zones, it is determined that correct oscillation is not being performed, and the detection of the attitude is redone from the state where the attitude has returned to Zone 1. When the count reaches the specified value (S309), the mixing operation is ended.

By performing such an operation, the control device **280** can detect that the operator has performed the mixing operation correctly.

Note that according to the control of the mixing operation described above, one reciprocal oscillation is not counted unless the attitude of the drug injection device **200** is not detected to enter Zone 1 and Zone 3, particularly. Therefore, if the operator repeats improper oscillation operation, it may possibly become difficult to complete the oscillation operation. In such a case, images corresponding to the operations denoted as P1 to P4 in FIG. **28** may be sequentially displayed on the display device **259**. For example, images showing the operations from P1 to P4 may be displayed before determination in S303, S304, S305, S306 and S307 in the flow chart. Such a display makes it easier for the operator to move the drug injection device **200** in accordance with the display, thereby preventing improper operations such as small tilt angles. By receiving specific operation instructions, the operator can confirm that the operation is not incorrect, that the movement is appropriate, etc., and can use the drug injection device **200** with less worry.

<Rotary encoder **265**>

The rotary encoder **265** detects the number of revolutions (the amount of driving) of the motor **264**. Since the amount of drug to be injected is set by the number of revolutions of the motor **264,** it is important that the number of revolutions of the motor **264** be accurately measured, and it is therefore important that the rotary encoder **265** be capable of accurate measurement. The rotary encoder **265** of the present embodiment has a structure with which it is possible to correctly detect a fault when the rotary encoder **265** itself fails.

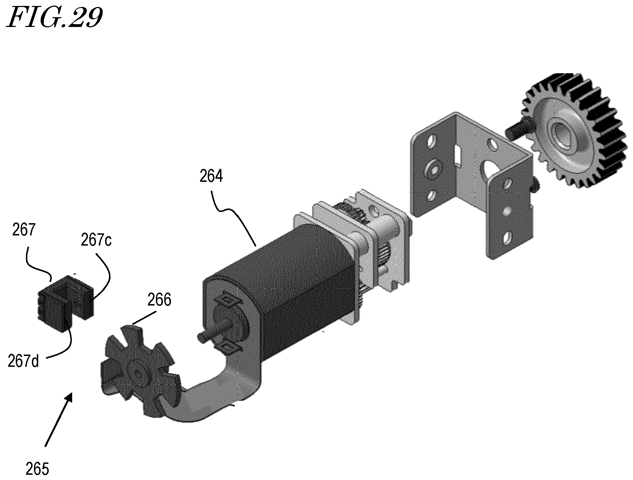

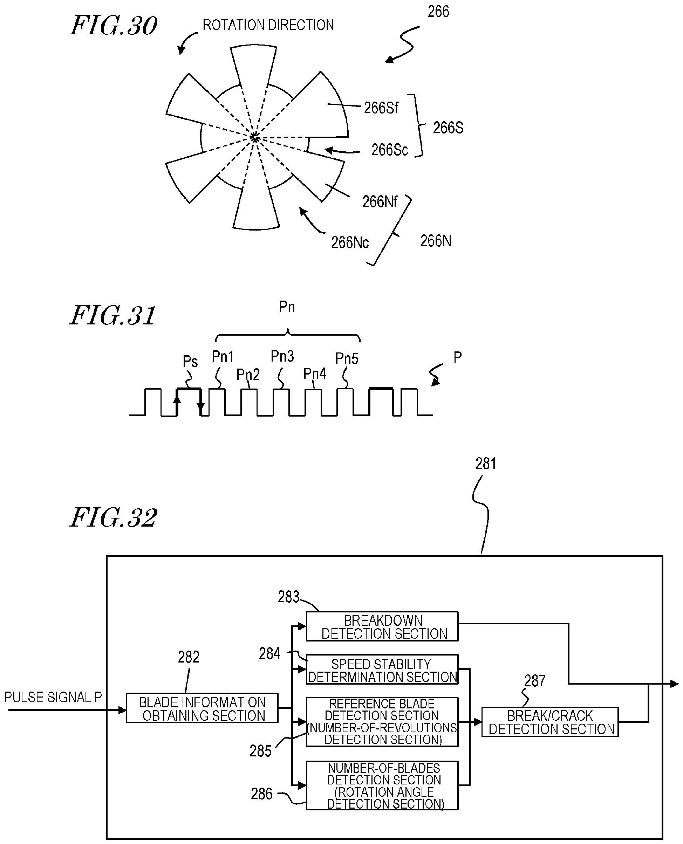

FIG. **29** is an exploded perspective view of a portion of the piston drive mechanism **220** including the rotary encoder **265**. As described above, the rotary encoder **265** includes the encoder plate **266** attached to the rotary shaft of the motor and the pulse encoder **267** including a light-emitting element **267c** and a light-receiving element **267d**. FIG. **30** is a plan view of the encoder plate **266**. The encoder plate **266** includes one reference blade section **266S** and a plurality of normal blade sections **266N** arranged along the circumference. In the present embodiment, the encoder plate **266** includes five normal blade sections **266N**. The reference blade section **266S** includes a blade **266Sf** and a notch **266Sc.** Each normal blade section **266N** includes a blade **266Nf** and a notch **266Nc.**

The lengths of the blades **266Nf** of the normal blade sections **266N** in the circumferential direction are equal to each other. The lengths of the notches **266Nc** of the normal blade sections **266N** in the circumferential direction are equal to each other. In contrast, the length of the blade **266Sf** of the reference blade section **266S** in the circumferential direction and the length of the notch **266Sc** in the circumferential direction are different from the length of the blade **266Nf** of the normal blade section **266N** in the circumferential direction and the length of the notch **266Nc** of the normal blade section **266N** in the circumferential direction, respectively.

In the present embodiment, the center angles of the blade **266Nf** and the notch **266Nc** of the normal blade section **266N** are 30°. On the other hand, the center angle of the blade **266Sf** of the reference blade section **266S** is 45°, and the center angle of the notch **266Sc** is 15°. By making the center angle of the blade **266Sf** of the reference blade section **266S** about 1.5 times the center angle of the blade **266Nf** of the normal blade section **266N,** it is easier to detect the difference between the reference blade section **266S** and the normal blade section **266N**. The larger the blade **266Sf** is, the smaller the notch **266Sc** becomes, but the notch **266Sc** can be detected with sufficient accuracy as long as the difference is about 1.5 times.

The encoder plate **266** is attached to the rotary shaft of the motor **264,** and when the motor makes one revolution, the encoder plate **266** also makes one revolution.

The light-receiving element **267d** of the pulse encoder **267** is arranged in a position at which light emitted from the light-emitting element **267c** enters. The pulse encoder **267** generates a pulse signal by detecting changes in the amount of light as the reference blade section **266S** and the normal blade sections **266N** of the encoder plate **266,** which rotates in sync with the rotation of the motor **264,** cross the optical path between the light-emitting element **267c** and the light-receiving element **267d**. FIG. **31** shows an example of a pulse signal. The example shown in FIG. **31** shows a signal obtained when the encoder plate **266** shown in FIG. **30** rotates counterclockwise. The pulse signal P includes a pulse Ps corresponding to the blade **266Sf** of the reference blade section **266S** and pulses Pn corresponding to the blades **266Nf** of multiple normal blade sections **266N**. The rising edge of each pulse is called the control edge and the falling edge is called the check edge. Control edges and check edges can be distinguished based on whether the light intensity detected by the photodetector increases or decreases.

The control device **280** receives the pulse signal P from the rotary encoder **265** and controls the motor driver **263** based on the pulse signal P. Specifically, the control device **280** controls the number of revolutions of the motor **264** based on the pulse signal P. It also detects a fault of the rotary encoder **265** and stops the rotation of the motor **264** when a fault is detected. One of the main faults of the rotary encoder **265** that should be detected is breaking or cracking of a blade of the encoder plate **266.** A broken blade means that the blade breaks near the base and drops off. When a blade breaks, the number of pulses generated per revolution of the encoder plate **266** decreases. Conversely, when a blade is cracked, the blade generates two or more pulses, so the number of pulses generated per revolution of the encoder plate **266** increases. In either case, these faults should be detected early because they cause errors in the measurement of the number of revolutions and the angle of rotation of the motor **264** and affect the amount of drug administered.

For this, the control device **280** includes a fault detector **281,** as shown in FIG. **32.** The fault detector **281** includes a blade information obtaining section **282,** a breakdown detection section **283,** a speed stability determination section **284,** a reference blade detection section **285,** a number-of-blades detection section **286,** and a break/crack detection section **287.**

The blade information obtaining section **282** receives the pulse signal from the pulse encoder **267** to measure the times of the control edge and the check edge of the pulses Ps and the pulses Pn in the pulse signal P based on the reference clock of the control device **280,** for example.

The breakdown detection section **283** successively calculates the blade ratio and outputs a detection signal indicating an abnormality when the blade ratio becomes less than or equal to a reference value. Specifically, the breakdown detection section **283** detects an abnormality in which one or more blades **266Sf** or **266Nf** are broken down and missing the encoder plate **266.** If one or more blades are completely missing, the number of pulses per revolution is more definitely reduced than if a part of a blade is broken and missing, and there will be a greater error in the amount of drug administered. The blade ratio is the ratio of the width of a pulse (Ps, Pn) in the pulse signal P with respect to the period of time from the control edge of the pulse to the control edge of the next pulse, i.e., the ratio of the time width from the control edge to the check edge of the pulse. As shown in Table 4, the blade ratio of the pulse Ps is 75% (75/100) and the blade ratio of the pulse Pn is 50% (50/100). If one blade adjacent to the normal blade section **266N** (e.g., the blade **266Nf** corresponding to the pulse Pn2 adjacent to the pulse Pn1) is missing, the blade ratio is 25% (50/200), and if one blade adjacent to the reference blade section **266S** (the blade **266Nf** corresponding to pulse Pn1) is missing, the blade ratio is 37.5% (75/200). If two blades adjacent to the normal blade section **266N** are missing, the blade ratio is 16.7% (50/300), and if two blades adjacent to the reference blade section **266S** are missing, the blade ratio is 25% (75/300). Thus, where 37.5% is used as the reference value and the blade ratio is less than or equal to 37.5%, for example, it may be determined that the blades of one or more blade portions are broken down.

The reference value used in the determination by the breakdown detection section **283** to may be another value. If the reference value is set to a value smaller than 250, e.g., if the reference value of 24%, it may be determined that two or more blades are consecutively missing. If two or more blades are consecutively missing, the number of pulses per revolution of the encoder plate **266** will be even smaller, and the error in the amount of drug administered will be even larger. The reference value to be set is not limited to the values shown in Table 4, and the reference value may be determined by taking into consideration the margin due to variations in the rotational speed of the motor **264.**

[Table 4]

| | No breakdown | Where adjacent blades are broken down | |
| --- | --- | --- | --- |
| | | Number of blades broken down | |
| | | 1 | 2 |
| Blade ratio of reference blade section | 75% | 37.5% | 25% |
| Blade ratio of normal blade section | 50% | 25% | 16% |

Each time the control edge of a pulse Ps, Pn is detected, the breakdown detection section **283** calculates the blade ratio using the time of the control edge and the time of the check edge of the immediately preceding pulse, and if it is a value in the range described above, the breakdown detection section **283** outputs a signal indicating a fault. As described above, a breakdown of a blade is likely to leads to a decrease in the number of pulses and gives a large error in the measurement of the number of revolutions and the angle of rotation of the motor **264.** Therefore, if the breakdown detection section **283** outputs a signal indicating an abnormality, the control device **280** directly controls the motor driver **263** to stop the motor **264** immediately, without using the determination result of the speed stability determination section **284** to be described below.

The speed stability determination section **284** determines whether the rotational speed of the encoder plate **266** is stable or not. This can be determined, for example, by measuring the length of the pulse interval (the time interval between the control edges of two adjacent pulses). However, if blades are broken or cracked, the pulse interval will vary. Specifically, if a part of a blade is missing, the pulse width may be shorter and the pulse interval may be longer. If a blade is broken, i.e., the blade is completely broken down and missing, the pulse interval will also be longer. If a blade is cracked, a single pulse turns into two pulses, thereby shortening the pulse interval in two places. That is, the pulse interval obtained in one revolution of the encoder plate **266** may possibly include those that have become longer or shorter due to chipping, breaking or cracking of a blade. Therefore, the speed stability determination section **284** excludes these and successively obtains and outputs the pulse interval average time.

If the number of blades of the encoder plate **266** is n and the blades are cracked, the number of pulses generated in one revolution is n+1 and the pulse interval between two pulses is short. When pulses are detected for r revolutions of the encoder plate **266,** the number of short pulse intervals is $(2/(n+1))\times(nr)$ . Similarly, when a blade is chipped, the number of long pulse intervals is $(1/(n-1))\times(nr)$.

Therefore, if the number of blades n is 6 and a pulse signal for 18 pulses (3 revolutions) is obtained, $(2/(6+1))\times(18) = 5.14$, which includes 5 to 6 short pulse intervals. Similarly, $(1/(6-1))\times(18) = 3.6$, which includes 3 to 4 long pulses.

Therefore, in order to obtain the correct rotation speed even when blades are cracked or broken, the pulse intervals for 18 pulses obtained are arranged in the order of length so as to exclude data for the four highest (long) pulse intervals and the six lowest (short) pulse intervals to determine the stability from the eight pulse intervals. For example, the standard deviation of the eight pulse intervals is determined, and if the standard deviation value is greater than a predetermined value, a signal indicating that the rotation speed is not stable is output. Alternatively, if the standard deviation value is smaller than a predetermined value, a signal indicating that the rotation speed is stable is output.

The reference blade detection section **285** detects a pulse Ps based on the reference blade section **266S** in the pulse signal P to output a detection signal. The blade ratio described above can be used for the detection.

The number-of-blades detection section **286** detects pulses Ps and Pn in the pulse signal to output a detection signal.

The break/crack detection section **287** receives signals from the speed stability determination section **284,** the reference blade detection section **285,** and the number-of-blades detection section **286** to determine whether a blade is broken or cracked. For example, when a signal indicating that the rotation speed is stable is received from the speed stability determination section **284,** the number of pulses per revolution is determined based on the detection signal output from the reference blade detection section **285** and the detection signal from the number-of-blades detection section **286.** If the number of pulses is not six, it is determined that there is a blade that is broken or cracked (hereafter, a broken or cracked blade is referred to as a fault) to output a signal indicating a fault. If a signal indicating that the rotation speed is stable is not received from the speed stability determination section **284,** or if a signal indicating that the rotation speed is not stable is received from the speed stability determination section **284,** the detection signal from the number-of-blades detection section **286** may possibly be not accurate, and therefore the detection result is not output. Alternatively, the break/crack detection section **287** outputs a signal indicating that correct measurement is impossible. This can prevent false detection of a broken or cracked blade of the encoder plate **266** due to an unstable rotation speed of the motor **264.** When the control device **280** receives a signal indicating a fault from the break/crack detection section **287,** the control device **280** controls the motor driver **263** so as to stop the motor **264.**

Note that the determination result of the speed stability determination section **284** is used for the determination of a fault in the number-of-blades detection section **286,** but even if a signal indicating that the rotation speed is stable is output from the speed stability determination section **284,** a fault may be erroneously detected due to the number of revolutions being stable. That is, there is some possibility of an erroneous fault detection in a single fault determination by the number-of-blades detection section **286.** In such a case, the probability of erroneous fault detection can be made smaller by using the detection results of the break/crack detection section **287** a plurality of times. For example, one may consider setting the number of fault detections used in one determination to a plurality of times, and accumulating the number of times a fault is determined.

For example, N consecutive detection results may be used for one determination of the break/crack detection section **287,** and the determination of the break/crack detection section **287** is accumulated M times.

Where it is demanded that the stability of the rotational speed of the motor **264** be high and the accuracy of the amount of drug administered be strict, the possibility of false detection of the break/crack detection section **287** is small to begin with. If there is a possibility that the amount of drug administered may not be accurate, it is preferable not to administer the drug. For this, it is preferred to determine a fault with N=1 and M=1. That is, if the break/crack detection section **287** detects a fault even once, it is preferred to stop the motor **264** or suspend the injection operation.

On the other hand, if there is a margin of accuracy in the amount of drug administered, and there is little possibility of adversely influencing the operator even if the amount administered is a little less or a little more, it is preferred to set N and M to values greater than 1 to further reduce the probability of false detection. For example, if N=2 and M=2, then a false detection is determined based on detection results of four iterations. Table 5 shows one such example. In Table 5, a fault (denoted as F in Table 5) is detected in the second and fourth iterations, but it is not determined as a fault (M is not counted) because a fault is not detected twice in a row (N=1). In the fifth and eighth iterations, a fault is detected twice consecutively, and it is determined that there have been two faults by accumulation in the eighth inspection. Therefore, in the eighth detection, a signal is output indicating that the break/crack detection section 287 has determined a fault.

[Table 5]

| Number of fault detections performed | 1 | 2 | 3 | 4 | 5 | 6 | 7 | 8 | 9 | 10 |
|---|---|---|---|---|---|---|---|---|---|---|
| Detection result | | F | | F | F | | F | F | F | F |
| N | 0 | 1 | 0 | 1 | 2 | 0 | 1 | 2 | 1 | 2 |
| M | 0 | 0 | 0 | 0 | 1 | 1 | 1 | 2 | 2 | 3 |

The fault detection of the rotary encoder 265 described above may be set to be performed whenever the drug injection device 200 is in operation and the motor 264 rotates. Alternatively, if it is undesirable for the injection to be interrupted or ended halfway through by detecting a fault and stopping the motor 264 while an injection is being performed, the fault detection may be not performed during the injection operation. Alternatively, the fault detection may be performed during the injection operation, but the motor 264 may be stopped after the injection is completed.

With either control, when the control device 280 detects a fault, the control device 280 causes the display device 259 to display information indicating the fault. This allows the operator to recognize the abnormality.

<User interface>

Information obtained from the various detection devices described above that are included in the drug injection device 200 can be displayed on the display device 259 or information for prompting the operator to operate the drug injection device 200 using the detection devices can be displayed on the display device 259. Example images that the control device 280 causes the display device 259 to display will now be described.

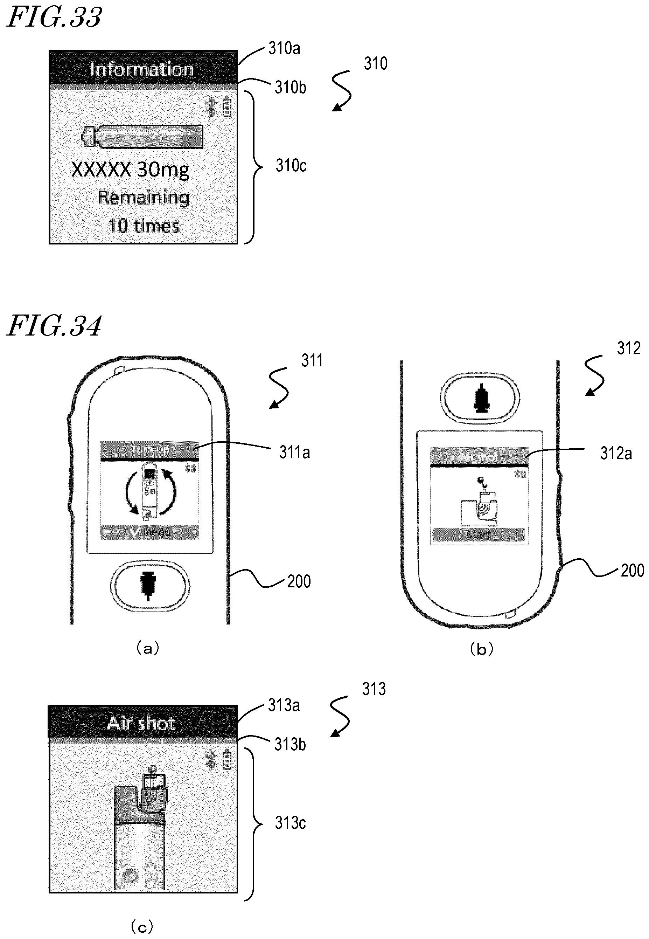

FIG. 33 shows an example of information of the RF tag 16 of the drug cartridge 10 to be displayed on the display device 259. As shown in FIG. 33, an image 310 includes a first region 310a, a second region 310b and a third region 310c. The first region 310a displays text information indicating the operation to be instructed to the operator or the status of the drug injection device 200. In the example shown in FIG. 33, the word "Information" is displayed because drug information stored in the RF tag 16 is being displayed. The second area 310b is colored with a theme color that corresponds to the type of the drug, for example. The third area 310c displays specific drug information.

In the present embodiment, for example, the drug is "XXXXX" and the second region 310b is colored in blue. The name of the drug, the dose per injection, and the amount of drug remaining (the number of times the drug can be injected) are displayed in the third area 310c, along with an image of the drug cartridge 10. The amount of drug remaining may be stored in the RF tag 16 or stored in the memory of the drug injection device 200. As the image of the drug cartridge 10 is displayed together, it is easier for the operator to recognize that the text information relates to the drug cartridge 10. The drug information shown in FIG. 33 is displayed, for example, after the cassette 100 is loaded and after performing the injection operation.

By using the format of the image 310 in other operations as well, it is easier for the operator to recognize what information is displayed where on the screen of the display device 259. In this case, the second area 310b should be colored in the theme color of the drug on the screen during any operation. Thus, the operator can recognize the type of drug being injected in any operation state.

FIG. 34(a) to FIG. 34(c) show example images displayed in the air-removing operation using the acceleration sensor 276. FIG. 34(a) shows an image 311, which prompts the operator to invert and hold the drug injection device 200 before the air-removing operation. FIG. 34(b) shows an image 312 just before the start of the air-removing operation, with the operator holding the drug injection device 200 inverted and with the distal end facing up. In FIG. 34(a), the first area 311a is displayed on the rear end side of the drug injection device 200 on the display device 259, while in FIG. 34(b), the first area 312a is displayed on the distal end side of the drug injection device 200 on the display device 259. That is, when the drug injection device 200 is held inverted, the control device 280 reverses the orientation of the image 312 displayed on the display device 259 based on the detection signal from the acceleration sensor 276. By matching the orientation of the image 312 displayed on the display device 259 to the attitude of the drug injection device 200, it is possible to present information that is easily recognized by the operator.

Then, while the control device **280** is performing the air-removing operation, the control device **280** may display an image **313** with an animation showing the air removal being performed in the third area **313c** as shown in FIG. **34(c).**

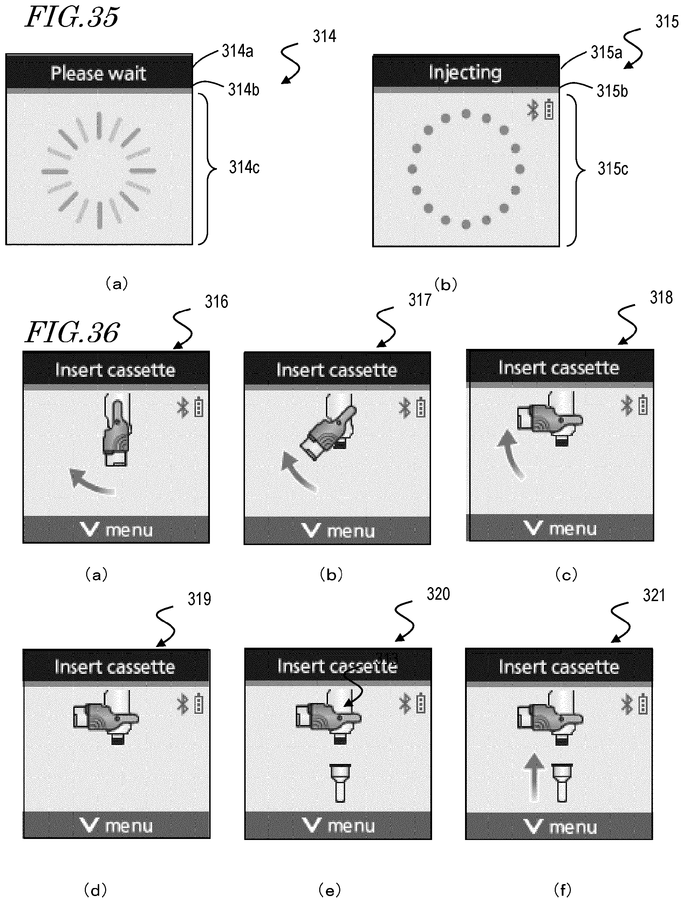

FIG. **35(a)** and FIG. **35(b)** are example images **314** and **315** that prompt the operator to remain in the same state until the operation of the drug injection device **200** is complete. Since the length of time to wait is not constant, the waiting time is not displayed, but an animation is shown in which designs arranged in a circular arrangement appear to be moving by blinking the designs. By thus displaying the animation, the operator can recognize that the operation of the drug injection device **200** is normal.

FIG. **36(a)** to FIG. **36(f)** show example images **316** to **321** that prompt the operator to attach the needle unit **20** and remove the needle case **25** after attachment. By sequentially repeatedly displaying the images shown in FIG. **36(a)** to FIG. **36(f)** until the operator removes the needle case **25,** the operator can easily understand how to operate and how far the operator should proceed in the operation.

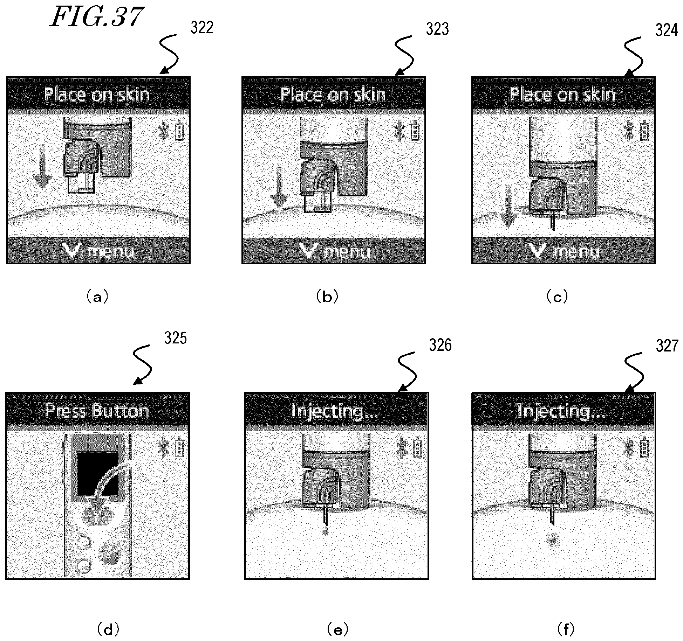

FIG. **37(a)** to FIG. **37(f)** show examples images **322** to **327** that prompt the operator to press the drug injection device **200** against the skin and press the inject button **258** for injection. The images of FIG. **37(a)** to FIG. **37(c)** are repeatedly displayed until the touch sensor **275** detects skin contact. If the touch sensor **275** then detects skin contact, the image in FIG. **37(d)** is displayed, which prompts the user to press the inject button **258.** While the drug is being injected, the images of FIG. **37(e)** and FIG. **37(f)** are repeated in sequence. If the touch sensor **275** detects separation from skin while the drug is being injected, the images of FIG. **37(a)** to FIG. **37(c)** may be repeatedly displayed again.

Thus, with the drug injection device **200** of the present embodiment, it is possible to provide a more user-friendly operation to the operator by using the information of the first temperature sensor, the information of the RF tag **16,** and the detection signals of the acceleration sensor **276** and the touch sensor **275.**

(Second embodiment)

The drug injection device of the present disclosure may be configured to directly house the drug cartridge **10** without using the cassette **100.** With reference to the drawings, an embodiment of a drug injection system including a drug injection device **500** that does not use the cassette **100** will now be described.

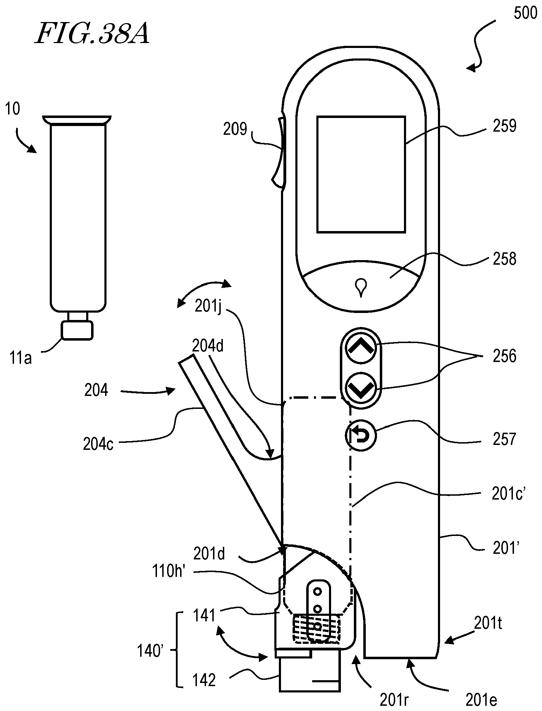

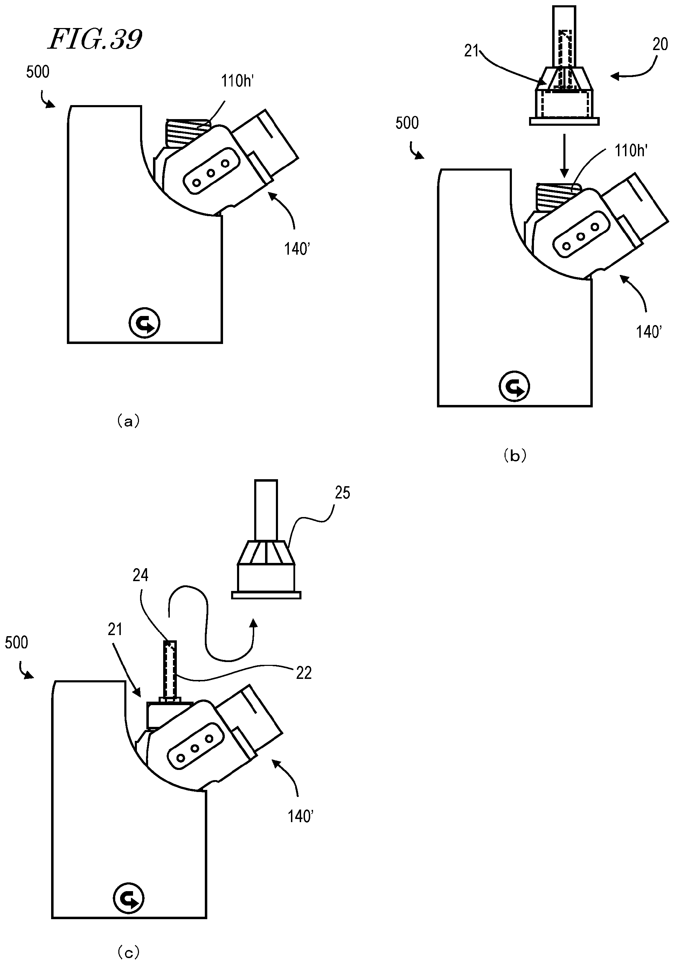

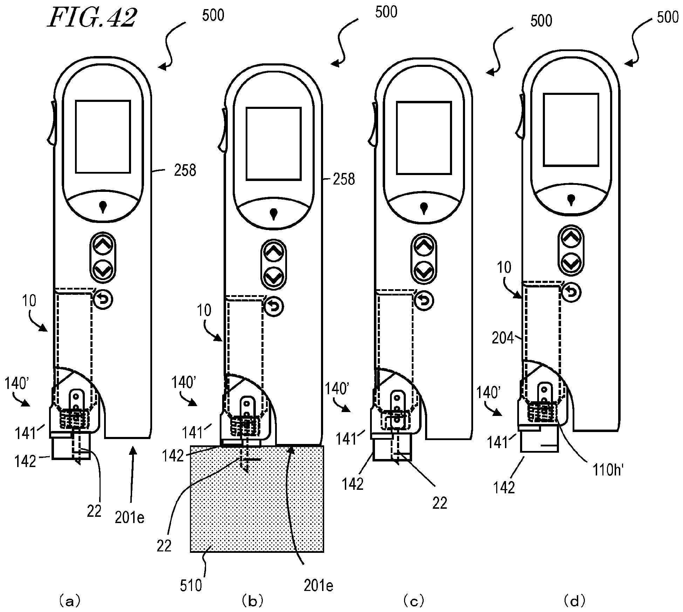

FIG. **38A** to FIG. **38C** are front views showing how the drug cartridge **10** is loaded in the drug injection device **500.** FIG. **39** to FIG. **41** are views illustrating how a needle unit **20** is attached to, or a used injection needle **21** is removed from, a drug injection device **500** with the drug cartridge loaded. FIG. **42** is views illustrating how an injection is performed using the drug injection device **500.** The structure and operation of a drug injection device **500** will be described with reference to these figures.

With the drug injection device **500,** the cassette **100** with the drug cartridge **10** housed therein is not loaded in the drug injection device **500,** but the drug cartridge **10** is directly loaded in the drug injection device **500.** The drug injection system does not include the cassette **100.** Otherwise, a drug injection device **500** has the same structure and function as the drug injection device **200** of the first embodiment. That is, a drug injection device **500** includes the RF-ID reader **277,** the piston drive mechanism **220,** the touch sensor **275,** the acceleration sensor **276,** the rotary encoder **265,** etc., performs a control using these components as described above in the first embodiment, and realizes a user interface using the display device **259;** these aspects remain the same also with the drug injection device **500.** Therefore, the following description mainly focuses on points that differ from the first embodiment.

As shown in FIG. **38A,** the drug injection device **500** includes a cartridge holder **204,** a hood **140',** and an injection needle attachment portion **110h'.** The drug injection device **500** also includes a housing space **201c'** in a device housing **201'** that can hold at least a portion of the drug cartridge **10** not stored in a cassette. The housing space **201c'** is adapted to house a portion of the drug cartridge **10** supported by the cartridge holder **204** described below. The device housing **201'** has a holder opening **201j** connected to the housing space **201c'** . The holder opening **201j** is a housing opening provided on the side surface of the device housing **201'.** With the drug injection device **500,** the drug cartridge **10,** which is not housed in a cassette, is supported by the cartridge holder **204** and loaded through the holder opening **201j.**

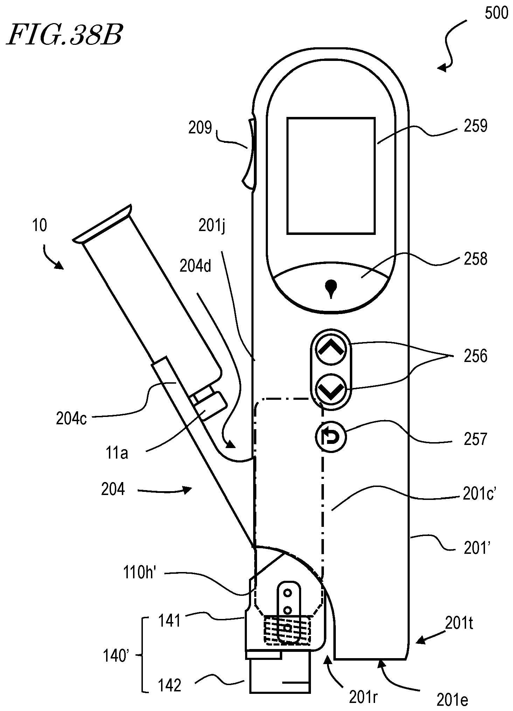

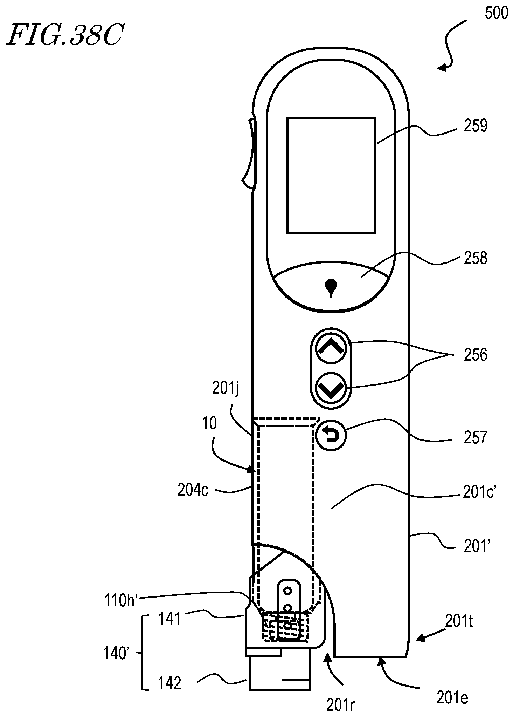

The cartridge holder **204** includes a door portion **204c** and a holder portion **204d.** The door portion **204c** has a shape corresponding to the holder opening **201j** so that the holder opening **201j** can be blocked. The holder portion **204d** has an inner space that supports the drug cartridge **10.** The cartridge holder **204** is pivotally attached to the device housing **201'** so that the door portion **204c** can assume a state where the holder opening **201j** is open and a state where the holder opening **201j** is closed. As shown in FIG. **38B,** when the door portion **204c** is in a state where the holder opening **201j** is open, the drug cartridge **10** can be supported in the cartridge holder **204** by inserting the drug cartridge **10** into the holder portion **204d** from the first end **11a.** It is also possible to pull out the drug cartridge **10** supported in the holder section **204D.** With the drug cartridge **10** inserted into the holder portion **204d,** the cartridge holder **204** is rotated to close the holder opening **201j** with the door portion **204C.** As shown in FIG. **38C,** with the holder opening **201j** closed by the door portion **204c,** a portion of the drug cartridge **10** is arranged in the housing space **201c'.** The distal end portion of the drug cartridge **10,** including the first end **11a,** is housed in the injection needle attachment portion **110h.**

The injection needle attachment portion **110h'** includes an internal space for storing the distal end portion of the drug cartridge **10,** and is arranged so as to cover the housing opening **201d** located at the bottom of the device recess portion **201r** of the device housing **201'**. The injection needle attachment portion **110h'** includes a structure similar to the injection needle attachment portion **110h** of the cassette **100.**

The hood **140'** has a structure similar to the hood **140** of the cassette **100** of the first embodiment, except that it does not include the arms **141c** (shown in FIG. **11). The** hood **140'** is located in the device recess portion **201r** of the housing **201'** and is pivotally attached to the housing **201'.** The hood **140'** covers the injection needle attachment portion **110h'** and can pivot with respect to the device housing **201'** in the direction indicated by the arrow in FIG. **38A.** Specifically, it can pivot between the first position in which the injection needle attachment portion **110h'** is covered and the second position in which the injection needle attachment portion **110h'** is exposed. With the injection needle attachment portion **110h'** exposed from the hood **140',** the operator can easily attach the needle unit **20** to the injection needle attachment portion **110h'** and remove the needle unit **20** engaged with the injection needle attachment portion **110h'.** The needle concealment **142** may be at least partially transparent as described with reference to FIG. **11.**

Next, the operation of attaching the needle unit **20** to the drug injection device **500** in which the drug cartridge **10** is loaded in order to perform injection will be described. As shown in FIG. **39(a),** first, the hood **140'** is pivoted to expose the injection needle attachment portion **110h'** from the hood **140'.** As shown in FIG. **39(b),** the needle unit **20** that houses the injection needle **21** is attached to the injection needle attachment portion **110h'.** Then, as shown in FIG. **39(c),** the needle case **25** is removed.

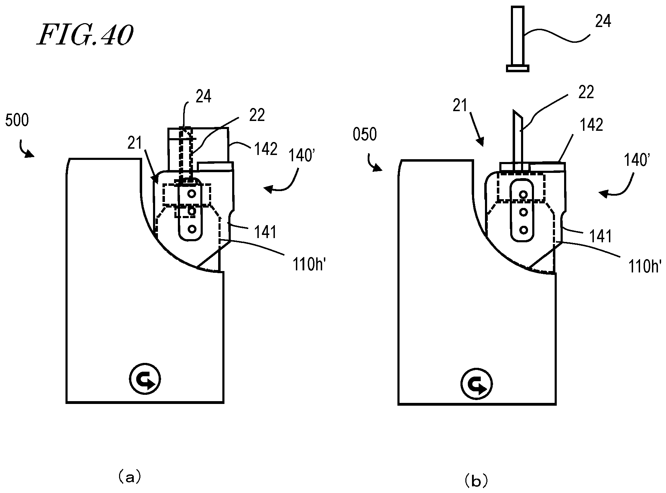

The hood **140'** is returned to its original position as shown in FIG. **40(a),** and the needle covering **142** is pushed down as shown in FIG. **40(b).** Then, the needle cap **24** is removed to expose the needle **22** of the injection needle **21.** Then, injection can be performed.

As described in the first embodiment, when performing injection, the drug injection device **500** is held so that the distal end of the needle **22** faces down, as shown in FIG. **42(a).** The needle **22** is covered by the hood body **141** and the needle cover **142.** As shown in FIG. **42(b),** in this state, when the skin-contact surface **201e** is brought into contact with the skin **510** and the drug injection device **500** is further pressed against the skin, the needle **22** is inserted into the skin **510** while the needle covering **142** retracts. Then, the inject button **258** is pressed to inject the drug.

After completion of injection, the drug injection device **500** is pulled off the skin **510,** and the needle covering **142** moves forward and covers the needle **22** as shown in FIG. **42 (c) .**

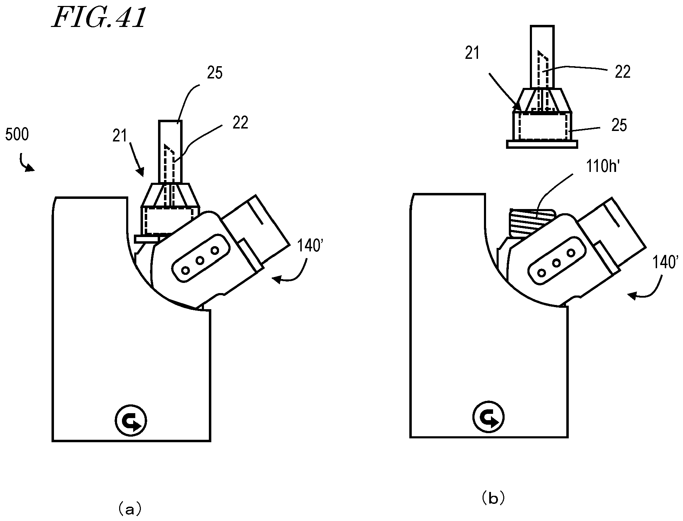

When injection is complete, the needle case **25** is placed over the injection needle **21** with the hood **140'** covering the needle **22.** Then, as shown in FIG. **41(a),** with the hood **140'** rotated, the needle case **25** is pulled while being rotated, so the injection needle **21,** together with the needle case **25,** is pulled out of the injection needle attachment portion **110h'** as shown in FIG. **41(b).**

By returning the hood **140'** to the original position, the injection needle attachment portion **110h'** is covered by the hood **140'** as shown in FIG. **42(d).** Then, the cartridge holder **204** is rotated and the drug cartridge **10** is taken out, thereby completing injection.

As described above, the drug injection device **500** can manage the temperature of the drug using the RF-ID reader **277,** control the motor according to the type of drug using the piston drive mechanism **220,** determine skin contact using the touch sensor **275,** control the attitude of the drug injection device **500** using the acceleration sensor **276** and determine a fault using the rotary encoder **265** as in the first embodiment.

A charger compatible with the drug injection device **500** of the present embodiment may, for example, have a space into which the distal end of the drug injection device **500** cannot be inserted with the drug cartridge **10** loaded in the drug injection device **500.** In this case, although not shown in the figures, some mechanism can be added such that the attachment of the drug cartridge **10** causes a change in the external appearance and external shape of the device housing **201',** thereby inhibiting the attachment to the charger. By inhibiting the attachment to the charger, deterioration of the drug due to heat generated during charging can be avoided.

Alternatively, the charger may have a space in which the distal end of the drug injection device **500** cannot be inserted, for example, with the needle unit **20** attached to the first end **11a** of the drug cartridge **10.** In addition to avoiding deterioration of the drug due to heat generated during charging, this configuration is also effective in reducing the size of the drug injection device and the charger, and in preventing infection, etc., caused by reuse of injection needles.

With the drug injection device **500** of the present embodiment, it is possible to provide operability that is easy for the operator to use as described in the first embodiment without the use of the cassette **100.**

The drug injection device **500** of the present embodiment is suitable, for example, when the drug cartridge **10** houses a single dose of drug to be injected into an operator such as a patient. Since the used drug cartridge **10** is disposed of, there is no need to store it in a refrigerator or other cold place using the cassette **100.** Thus, the cost of injection can be reduced. However, the drug cartridge **10** may store multiple doses of drug. In this case, a separate storage case may be provided and the drug cartridge **10** containing the remaining drug may be stored in a refrigerator, or the like, or the entire drug injection device **500** to which the drug cartridge **10** is attached may be stored in a refrigerator, or the like. However, if the entire drug injection device **500** is stored in a cold place, the reminder operation described in the first embodiment above will be difficult to perform because the drug injection device **500** itself is also cooled.

(Other embodiments)

The embodiment described above is one example of the drug injection device of the present disclosure, and various modifications can be made thereto. The shapes of the drug cartridge **10,** the cassette **100,** the drug injection device **200** and the charger **300** illustrated in the figures and their constituent parts are examples, and they may have other shapes. The drug injection device **200** does not need to include all of the various sensors. The injection operation, the air-removing operation, the mixing operation, etc., according to the temperature of the drug described with reference to the flow charts are examples, and some of the steps may be omitted or performed in a different order, or other steps may be included.

The estimation of the time at which the drug reaches the appropriate temperature may be performed based on a calculation other than Equations (1) to (3), or the time at which the drug reaches the appropriate temperature may be determined directly as a function of elapsed time. The fault detector **281** may include other functional blocks or may perform signal processing different from the embodiments described above to determine the fault.

**INDUSTRIAL APPLICABILITY**

The cassette, the drug injection device and the drug injection system of the present disclosure can suitably be used for devices for injecting various drugs.

**REFERENCE SIGNS LIST**

| | |
|---|---|
| 10, 10' | Drug cartridge |
| 11 | Cylinder |
| 11' | Cylinder |
| 11a | First end |
| 11b | Second end |
| 11c | Cylinder columnar space |
| 11c1 | First region |
| 11c2 | Second region |
| 11c3 | Third region |
| 11d | Cylinder opening |
| 11e | Bypass space |
| 11h | Corner |
| 11j | Shaft |
| 11t | Projecting portion |
| 12 | Cylinder cap |
| 13 | Gasket |
| 13A | First gasket |
| 13B | Second gasket |
| 14 | Drug |
| 14A | Liquid component |
| 14B | Solid component |
| 15 | First temperature sensor |
| 16, 16' | RF tag |
| 16a | Antenna |
| 17 | Label |
| 20 | Needle unit |
| 20a | Distal end |
| 20c | Housing space |
| 20t | Protruding portion |
| 21 | Injection needle |
| 22 | Needle |
| 23 | Connecting portion |
| 24 | Needle cap |

| | |
|---|---|
| 25 | Needle case |
| 31 | Charger housing |
| 100 | Cassette |
| 110 | Cassette body |
| 110a | Distal end |
| 110b | Rear end |
| 110c | Cassette columnar space |
| 110e | Body opening |
| 110h, 110h' | Injection needle attachment portion |
| 130 | Cassette cap |
| 130d | Cap opening |
| 140, 140' | Hood |
| 141 | Hood body |
| 141b | Rear end |
| 141c | Arm |
| 142 | Needle covering |
| 142c | First portion |
| 142d | Second portion |
| 143 | Biasing member |
| 200 | Drug injection device |
| 200a | Distal end section |
| 201 | Device housing |
| 201a | Distal end section |
| 201c, 201c' | Housing space |
| 201d | Housing opening |
| 201e | Skin-contact surface |
| 201g | Charging terminal |
| 201r | Recess portion |
| 201t | Protruding portion |
| 202 | Internal housing |
| 202d | Gear region |
| 202f | Piston guide region |
| 202g | Protruding portion |
| 202h | Cassette region |
| 203 | First guide |
| 204 | Cartridge holder |
| 204c | Door portion |
| 204d | Holder portion |
| 209 | Eject lever |
| 210 | Piston |
| 210a | First end |
| 210b | Second end |
| 210h | Hole |
| 211 | Distal end section |
| 212 | Body |
| 213 | Drive protruding portion |
| 214 | Female thread |
| 220 | Piston drive mechanism |
| 221 | Gearbox |
| 222 | Drive gear |
| 223 | Bearing |
| 230 | Piston guide |
| 230g | Groove |
| 230h | Hole |
| 231 | Guide |
| 235 | Drive rod |
| 236 | Male thread |
| 251 | Control section |

| 252 | Charging section |
| 253 | Secondary battery |
| 254 | Memory |
| 255 | Power button |
| 256 | Select button |
| 257 | OK button |
| 258 | Inject button |
| 259 | Display device |
| 260 | Buzzer |
| 261 | Clock |
| 262 | Communication section |
| 263 | Motor driver |
| 264 | Motor |
| 265 | Rotary encoder |
| 266 | Encoder plate |
| 266N | Normal blade section |
| 266Nc | Notch |
| 266Nf | Blade |
| 266S | Reference blade section |
| 266Sc | Notch |
| 266Sf | Blade |
| 267 | Pulse encoder |
| 267c | Light-emitting element |
| 267d | Light-receiving element |
| 270C | Transmission/reception circuit |
| 271 | Piston origin detector |
| 272 | Cassette loading detector |
| 273 | Second temperature sensor |
| 274 | Eject lever detector |
| 275 | Touch sensor |
| 276 | Acceleration sensor |
| 277 | RF-ID reader |
| 278 | Antenna |
| 278A | First portion |
| 278B | Second portion |
| 279 | Transmission/reception circuit |
| 280 | Control device |
| 281 | Fault detector |
| 282 | Blade information obtaining section |
| 283 | Breakdown detection section |
| 284 | Speed stability determination section |
| 285 | Reference blade detection section |
| 286 | Number-of-blades detection section |
| 287 | Detection section |
| 290 | Main board |
| 291 | First sub-board |
| 292 | Second sub-board |
| 300 | Charger |
| 301 | Charger housing |
| 301d | Rib |
| 301e | Supply terminal |
| 301r | Charger recess portion |
| 301s | Step |
| 301u | Space |
| 310 | Image |
| 310a, 311a, 312a | First region |
| 310b | Second region |
| 310c | Third region |

| 311, 312, 313 | Image |
| 400 | Drug injection system |

**Claims**

1. A drug cartridge, comprising:

   a cylinder having a cylindrical cylinder columnar space extending in a longitudinal direction;
   a gasket supported in the cylinder columnar space so that the gasket is movable in the longitudinal direction;
   a drug held in the cylinder columnar space and at least including a liquid first component; and
   a first temperature sensor and an RF tag arranged on a side surface of the cylinder, wherein:
   the RF tag stores drug information that includes at least information indicating a type of the drug, and wirelessly transmits at least the information indicating the type of the drug and first temperature information indicating a temperature detected by the first temperature sensor in response to an instruction from outside.

2. The drug cartridge according to claim 1, wherein the RF tag is of a passive type.

3. The drug cartridge according to claim 1, wherein the RF tag further stores at least one of information indicating an expiration date of the drug and information indicating an initial amount of the drug.

4. A cassette housing a drug cartridge that is loaded in a drug injection device, the cassette comprising:

   a cassette body having a cassette columnar space capable of housing at least a portion of the drug cartridge, an injection needle attachment portion that is located at a distal end of the cassette columnar space and to/from which an injection needle can be attached/detached, and a body opening located at a rear end of the cassette columnar space and accessible to the cassette columnar space;
   a cassette cap supported in the vicinity of the rear end of the cassette body so as to open/close the body opening; and
   a hood including a hood body supported by the cassette body so that the hood body can pivot between a first position at which the injection needle attachment portion of the cassette body is covered and a second position at which the injection needle attachment portion is exposed, a needle covering supported by the hood body so that the needle covering can move between a projecting position at which the needle covering projects relative to the hood body and a stowed position at which the needle covering is at least partially stowed, and a biasing member configured to bias the needle covering in a direction of the projecting position.

5. The cassette according to claim 4, wherein a portion of the needle covering is transparent.

6. The cassette according to claim 5, wherein the needle covering includes a transparent first portion and a semitransparent second portion.

7. The cassette according to any one of claims 4 to 6, wherein the hood includes an arm that extends on an opposite side of the hood body relative to a fulcrum of the pivot and is connected to the hood body.

8. A drug injection device, comprising:

   a device housing having a housing space that houses at least a portion of a cassette that houses a drug cartridge having a first temperature sensor and an RF tag, or at least a portion of a drug cartridge having a first temperature sensor and an RF tag and being not housed in the cassette, and a housing opening that communicates with the housing space;
   a piston supported so that the piston can move into the housing space;
   a motor configured to drive the piston;
   a motor driver configured to generate a drive signal for driving the motor;
   an antenna arranged adjacent to the housing space;
   a transmission/reception circuit configured to transmit electromagnetic waves from the antenna and receive electromagnetic waves received by the antenna;
   a display device configured to output information regarding an injection operation; and
   a control device configured to control the motor driver, the transmission/reception circuit and the display device, wherein:

with the drug cartridge loaded in the housing space, the control device causes the transmission/reception circuit to receive, via the antenna, drug information including information indicating a type of a drug in the drug cartridge transmitted from an RF tag of the drug cartridge and first temperature information detected by the first temperature sensor, and determines a drive power of the motor based on the first temperature information to control the motor driver so as to output the determined drive power.

9. A drug injection device, comprising:

a device housing having a housing space that houses at least a portion of a cassette that houses a drug cartridge having a first temperature sensor and an RF tag, or at least a portion of a drug cartridge having a first temperature sensor and an RF tag and being not housed in the cassette, and a housing opening that communicates with the housing space;
a piston supported so that the piston can move into the housing space;
a motor configured to drive the piston;
a motor driver configured to generate a drive signal for driving the motor;
an antenna arranged adjacent to the housing space;
a transmission/reception circuit configured to transmit electromagnetic waves from the antenna and receive electromagnetic waves received by the antenna;
a memory storing control information for controlling the motor for each of a plurality of drugs, wherein the control information of each drug includes a set of control parameters of the motor for each of a plurality of temperature ranges;
a display device configured to output information regarding an injection operation; and
a control device configured to control the motor driver, the transmission/reception circuit, the memory and the display device.

10. The drug injection device according to claim 9, wherein:

the RF tag stores drug information including information indicating at least a type of the drug;
with the drug cartridge loaded in the housing space, the control device is configured to:

cause the transmission/reception circuit to transmit a control signal via the antenna;
cause the transmission/reception circuit to receive, via the antenna, first temperature information and drug information output from the first temperature sensor and the RF tag of the drug cartridge;
determine one set of control parameters from the control information stored in the memory based on the first temperature information and the drug information; and
control the motor using the determined set of control parameters.

11. The drug injection device according to claim 10, wherein the motor driver generates a drive signal by pulse width modulation and outputs the drive signal to the motor.

12. The drug injection device according to claim 11, wherein in the control information of each drug, a duty ratio of the drive signal by the pulse width modulation of a set of control parameters for the motor is smaller as a lower limit temperature of a temperature range of the set of control parameters for the motor is higher.

13. The drug injection device according to claim 11 or 12, wherein:

in the control information of each drug, the set of control parameters of the motor for each temperature range includes an initial value and an increment of duty ratio of the drive signal by pulse width modulation; and
the control device causes the motor driver to generate the drive signal so that current flowing in the motor increase in steps.

14. The drug injection device according to any one of claims 10 to 13, wherein the control device does not drive the motor when the first temperature information is in a first temperature range that is less than a first temperature or equal to or greater than a second temperature.

15. The drug injection device according to claim 14, wherein when the first temperature information is in a second temperature range that is equal to or greater than the first temperature and less than a third temperature lower than the second temperature, the control device controls the display device to display information indicating to wait before injection.

16. The drug injection device according to claim 15, wherein when the first temperature information is in a third temperature range that is equal to or greater than the third temperature and less than the second temperature, the control device controls the display device to display information indicating that it is possible to perform injection.

17. The drug injection device according to claim 15, further comprising a second temperature sensor provided in the device housing and configured to output second temperature information indicating a temperature inside the device housing.

18. The drug injection device according to claim 17, wherein when the second temperature information is equal to or greater than a predetermined temperature, the control device successively obtains the first temperature information at predetermined time intervals, successively calculates estimated time until it is possible to perform injection based on the first temperature information and the second temperature information, and controls the display device to display information indicating the calculated estimated time.

19. The drug injection device according to any one of claims 8 to 18, wherein:

the antenna includes a first portion and a second portion, which can independently receive signals from outside; and
the first portion and the second portion are arranged orthogonal to each other adjacent to the housing space.

20. The drug injection device according to any one of claims 8 to 19, further including:

a user interface including an inject button and configured to accept an instruction from an operator; and
a touch sensor of a surface charge transfer type, wherein:

the device housing has a skin-contact surface that comes into contact with skin of the operator during injection; and
the touch sensor is arranged on the skin-contact surface.

21. The drug injection device according to claim 20, wherein:

when a signal resulting from pressing the inject button is received while a detection signal resulting from skin contact is received from the touch sensor, the control device controls the motor driver so as to perform an injection operation; and
when the touch sensor detects separation from skin during the injection operation, the control device causes the display device to display information indicating abnormality.

22. The drug injection device according to any one of claims 8 to 21, further comprising:

a sensor having a first axis, a second axis and a third axis, which are orthogonal to each other, configured to detect acceleration along the axes or angular acceleration around the axes, and arranged in a housing so that one of the first axis, the second axis and the third axis coincides with a direction of movement of the piston; and
the control device causes the display device to display information regarding an attitude of the drug injection device based on a detection signal of the sensor.

23. The drug injection device according to any one of claims 8 to 22, further comprising:
a rotary encoder including an encoder plate attached to a rotation axis of the motor and a pulse encoder, wherein:

the encoder plate includes one reference blade section and a plurality of normal blade sections arranged along a circumference, each including a notch and a blade;
the blades of the plurality of normal blade sections have an equal length in a circumferential direction, and the notches of the plurality of normal blade sections have an equal length in the circumferential direction;
a length of the blade of the reference blade section in the circumferential direction and a length of the notch in the circumferential direction are different respectively from the length of the blades of the plurality of normal blade sections in the circumferential direction and the length of the notches in the circumferential direction; and

the pulse encoder includes a light-emitting element and a light-receiving element arranged so as to receive light emitted from the light-emitting element, wherein the reference blade section and the plurality of normal blade sections of the encoder plate, which rotates in sync with the rotation of the motor, crosses an optical path between the light-emitting element and the light-receiving element, thereby generating a pulse signal including a pulse corresponding to the blade of the reference blade section and pulses corresponding to the blades of the plurality of normal blade sections.

24. The drug injection device according to claim 23, wherein the length of the blade of the reference blade section in the circumferential direction is larger than the length of the blades of the normal blade sections in the circumferential direction, and the length of the notch of the reference blade section in the circumferential direction is smaller than the length of the notches of the normal blade sections in the circumferential direction.

25. The drug injection device according to claim 23 or 24, wherein the control device controls the motor driver based on the pulse signal.

26. The drug injection device according to any one of claims 23 to 25, wherein the control device calculates the number of blades of the reference blade section and the plurality of normal blade sections per one revolution of the encoder plate based on the pulse signal, and causes the display device to display information indicating a fault if the calculation result is different from a predetermined value.

27. The drug injection device according to claim 22, wherein the control device reverses an orientation of information displayed on the display device based on a detection signal from the sensor.

28. The drug injection device according to any one of claims 8 to 27, wherein the control device causes the display device to display the drug information.

29. The drug injection device according to any one of claims 8 to 28, wherein the control device displays a theme color corresponding to a type of the drug across a plurality of operation screens.

30. The drug injection device according to claim 8 or 9, wherein:

the device housing has a distal end section including a protruding portion extending in a longitudinal direction, a skin-contact surface located on an upper surface of the protruding portion, a device recess portion adjacent to the skin-contact surface, the housing opening being located at a bottom of the device recess portion, and an injection needle attachment portion that covers the housing opening and has an inner space in which a distal end of the drug cartridge is inserted;
the housing space is adapted to house at least a portion of the drug cartridge that is not housed in the cassette;
the drug injection device further comprising a hood including a hood body located in the device recess portion and pivotally attached to the device housing, a needle covering supported to be movable relative to the hood body, and a biasing member configured to bias the needle covering in a direction of the projecting position; and
the hood is pivotable between a first position at which the injection needle attachment portion is covered and a second position at which the injection needle attachment portion is exposed.

31. A drug injection system, comprising:

the cassette according to any one of claims 4 to 7; and
the drug injection device according to any one of claims 8 to 29.

32. The drug injection system according to claim 31, further comprising the drug cartridge according to any one of claims 1 to 3, wherein:
the drug cartridge is housed in the cassette columnar space of the cassette.

33. A drug injection system, comprising:

the drug cartridge according to any one of claims 1 to 3;
a cassette including a cassette columnar space in which at least a portion of the drug cartridge is housed; and
the drug injection device according to any one of claims 8 to 29.

**34.** The drug injection system according to claim 31 or 32, wherein the drug injection device comprises:

a secondary battery; and
a charging terminal provided in the device housing, wherein:

the device housing has a distal end section including a protruding portion extending in a longitudinal direction, a skin-contact surface located on an upper surface of the protruding portion, a device recess portion adjacent to the skin-contact surface, the housing opening being located at a bottom of the device recess portion; and with the cassette loaded in a housing space of the drug injection device, a portion of the hood of the cassette is exposed to an outside in the recess portion of the device housing, and another portion of the hood is located in the device housing so that the hood cannot pivot.

**35.** The drug injection system according to claim 34, further comprising a charger including a power supply circuit for charging the secondary battery of the drug injection device, and a charger housing that houses the power supply circuit, wherein:

the charger housing includes a charger recess portion having a space in which the distal end portion of the drug injection device can be inserted, a step provided at a bottom of the charger recess portion and having a shape corresponding to the housing recess portion of the drug injection device, and a supply terminal located in the recess portion and connected to the power supply circuit; and
the charger housing is configured so that with the cassette not loaded in the drug injection device, the distal end section of the drug injection device can be housed in the charger recess portion so that the charging terminal of the drug injection device is in contact with the supply terminal, and with the cassette loaded in the drug injection device, the cassette interferes with the step of the charger housing, thereby preventing the distal end section of the drug injection device from being housed in the charger recess portion.

FIG.1

*FIG.2*

100

130d

130

110

140 { 141
142

(a)

100

10

110c

130

110e

110

110b

(b)

100

10

(c)

*FIG.3*

(a)

(b)

(c)

FIG.4

(a)

(b)

*FIG.5*

DRUG INJECTION DEVICE

- 252 — CHARGING SECTION
- 253 — SECONDARY BATTERY
- 254 (280) — MEMORY
- 255 — POWER BUTTON
- 256 — SELECT BUTTON
- 257 — OK BUTTON
- 258 — INJECT BUTTON
- 259 — DISPLAY DEVICE
- 260 — BUZZER
- 261 — CLOCK
- 262 — COMMUNICATION SECTION (BLE)
- 277 — RF-ID READER

251 (280) — CONTROL SECTION (CPU)

200

- 263 — MOTOR DRIVER
- 264 — MOTOR
- 265 — ROTARY ENCODER
- 271 — PISTON ORIGIN DETECTOR
- 272 — CASSETTE LOADING DETECTOR
- 273 — SECOND TEMPERATURE SENSOR
- 274 — EJECT LEVER DETECTOR
- 275 — TOUCH SENSOR
- 276 — ACCELERATION SENSOR

## FIG.6A

## FIG.6B

*FIG.7A*

*FIG.7B*

*FIG.8A*

16

16b  16a

17

*FIG.8B*

16'

15  16c  16a

17

*FIG.9A*

*FIG.9B*

FIG.10

(a)

(b)

## FIG.11

# FIG.12

(a)

(b)

FIG.13

FIG.14

FIG.15

*FIG.16A*

16
16b
16a
10
278A
y
x
278B

*FIG.16B*

16
16a
16b
278A
y
x
278B

## FIG.17

FIG.18A

FIG.18B

## FIG.19A

```
                              START
                                │
                                ▼ S1
WITHOUT                                WITH DRUG CARTRIDGE
DRUG CARTRIDGE                                              OBTAIN, FROM RFID,     S4
              ◇ WITH OR WITHOUT                            TYPE OF DRUG
                DRUG CARTRIDGE ──────────────────────▶    CARTRIDGE AND
                                                          TEMPERATURE
                                                                │
OBTAIN, FROM MEMORY,          LESS THAN 0°C,                    ▼ S5          20°C TO
NO-LOAD DRIVE                 OR 38°C OR MORE                              LESS THAN 38°C
CONTROL INFORMATION    S2                   ◇ TEMPERATURE (T0)
TO BE USED WHEN                               OF DRUG CARTRIDGE
THERE IS NO DRUG          S6
CARTRIDGE                                               0 TO LESS THAN 20°C
                         DISPLAY SCREEN
DRIVE MOTOR BASED        RECOMMENDING REPLACING
ON OBTAINED DRIVE   S3   DRUG CARTRIDGE              DISPLAY SCREEN TO CHECK   S7
INFORMATION                                         WHETHER OR NOT TO WAIT
                                                    FOR TEMPERATURE TO RISE
                                                                │
      END                        END                            ▼
                                                                           USER SELECTS TO
                                                                            ADMINISTER
                                                    ◇ USER SELECTION ──────────────────┐
                                                   S8                                    │
                                                         USER SELECTS TO                 │
                                                      WAIT FOR TEMPERATURE               │
                                                           TO RISE                       ▼
                                                                │              TO ADMINISTRATION
                                                    TO REMINDER OPERATION         OPERATION
                                                                │
                                                    TO ADMINISTRATION
                                                       OPERATION
```

## FIG.19B

*FIG.19C*

REMINDER
OPERATION

S21
IS DEVICE
TEMPERATURE
20°C OR MORE?

NO

YES

S22
T0→T1

S35
INFORM DISCONTINUATION
OF REMINDER OPERATION

END

S23
HAS ONE
MINUTE ELAPSED
SINCE PREVIOUS
MEASUREMENT?

NO

YES

S24
DETERMINE
POWER STATE

OFF

S25
POWER ON

ON

S26
STORE T2

S27
$T2 - T1 < 1$ ?

NO

YES

S28
ESTIMATE TIME

S30
INFORM
ESTIMATED TIME

S29
TEMPERATURE OF
DRUG CARTRIDGE?

LESS THAN
20°C

20°C
OR MORE

S31
T2→T1

S34
INFORM
APPROPRIATE
TEMPERATURE

S32
DETERMINE
POWER STATE

OFF

END

ON

S33
POWER OFF

## FIG.20

## FIG.21

```
                    ┌─────────────────┐
                    │  PREPARE FOR    │
                    │ ADMINISTRATION  │
                    └────────┬────────┘
                             │
              S101           ▼
                  ╱────────────────────╲         ┌──────────────────────┐
                 ╱  CHECK TOUCH SENSOR  ╲───Fail──▶│    DISPLAY FAULT     │
                 ╲                      ╱         └──────────────────────┘
                  ╲────────┬───────────╱
                       Pass│
              S103         ▼
              ┌─────────────────────────┐
              │ DISPLAY PROMPTING TO    │
              │  PRESS AGAINST SKIN     │
              └────────────┬────────────┘
                           ▼  ◀──────────────┐
              S104                           │
                  ╱────────────────────╲     │
                 ╱         HAS          ╲  N  │
                ╱ CONTACT (FIVE TIMES)   ╲────┘
                ╲   BEEN DETECTED?       ╱
                 ╲──────────┬───────────╱
              S105        Y │
              ┌─────────────▼───────────┐
              │ DISPLAY PROMPTING TO    │
              │ PRESS INJECT BUTTON     │
              └─────────────┬───────────┘
                            ▼  ◀─────────────┐
              S106                           │
                  ╱────────────────────╲     │
                 ╱    HAS INJECT BUTTON  ╲ N  │
                 ╲    BEEN DETECTED?     ╱────┘
                  ╲─────────┬──────────╱
              S107        Y │
              ┌─────────────▼───────────┐
              │    DRIVE INJECTION      │
              │   OPERATION (MOTOR)     │
              └─────────────┬───────────┘
                            ▼
              S108
                  ╱────────────────────╲
                 ╱         HAS          ╲  N
                ╱ CONTACT (FIVE TIMES)   ╲────▶
                ╲   BEEN DETECTED?       ╱
                 ╲──────────┬───────────╱
              S109        Y │
               N  ╱────────────────────╲
              ◀──╱    ADMINISTRATION     ╲
                 ╲ OPERATION COMPLETED?  ╱
                  ╲─────────┬──────────╱
                          Y │
                            ▼
                    ┌───────────┐
                    │    END    │
                    └───────────┘
```

S110 — DISPLAY DISCONTINUATION AND CHECK INTENTION TO RESUME INJECTION

S111 — HAS BUTTON BEEN DETECTED?  OTHER BUTTONS / INJECT BUTTON

S112 — HAS CONTACT (FIVE TIMES) BEEN DETECTED?

S113 — RESUME INJECTION OPERATION

*FIG.22*

*FIG.23*

*FIG.24*

90°
y'

110°    70°

ANGLE TO ALLOW FOR AIR REMOVAL

X'    0°

*FIG.25*

START AIR REMOVAL

S201 — DISPLAY PROMPTING TO HOLD UPWARD

S202 — IS TILT 70° TO 110° ?

N → S203 — DISPLAY PROMPTING TO CORRECT ATTITUDE

Y

S204 — AIR-REMOVING OPERATION

S205 — IS TILT 70° TO 110° ?

N → S207 — STOP MOTOR AND DISPLAY PROMPTING TO CORRECT ATTITUDE

Y

S206 — AIR-REMOVING OPERATION COMPLETED?

S208 — IS TILT 70° TO 110° ?

N

Y

S209 — RESUME AIR-REMOVING OPERATION

Y

END

## FIG.26

FIG.27

*FIG.28*

P 1

P 3

P 2

P 4

FIG.29

264

267

267c

266

267d

265

*FIG.30*

ROTATION DIRECTION

266

266Sf

266S

266Sc

266Nf

266N

266Nc

*FIG.31*

Pn

Ps    Pn1    Pn3    Pn5    P

Pn2    Pn4

*FIG.32*

281

PULSE SIGNAL P

282
BLADE INFORMATION
OBTAINING SECTION

283
BREAKDOWN
DETECTION SECTION

284
SPEED STABILITY
DETERMINATION SECTION

285
REFERENCE BLADE
DETECTION SECTION
(NUMBER-OF-REVOLUTIONS
DETECTION SECTION)

286
NUMBER-OF-BLADES
DETECTION SECTION
(ROTATION ANGLE
DETECTION SECTION)

287
BREAK/CRACK
DETECTION SECTION

*FIG.33*

*FIG.34*

(a)

(b)

(c)

FIG.35

(a)

(b)

FIG.36

(a)      (b)      (c)

(d)      (e)      (f)

*FIG.37*

322

Place on skin

V menu

(a)

323

Place on skin

V menu

(b)

324

Place on skin

V menu

(c)

325

Press Button

(d)

326

Injecting...

(e)

327

Injecting...

(f)

*FIG.38A*

*FIG.38B*

FIG.38C

# FIG.39

(a)

(b)

(c)

*FIG.40*

(a)

(b)

*FIG.41*

(a)

(b)

*FIG.42*

(a)　　　　　(b)　　　　　(c)　　　　　(d)

## INTERNATIONAL SEARCH REPORT

| International application No. |
| --- |
| **PCT/JP2021/043006** |

**A.    CLASSIFICATION OF SUBJECT MATTER**

    *A61M 5/20*(2006.01)i; *A61M 5/24*(2006.01)i; *A61M 5/28*(2006.01)i
    FI:    A61M5/28; A61M5/20; A61M5/24 500

According to International Patent Classification (IPC) or to both national classification and IPC

**B.    FIELDS SEARCHED**

Minimum documentation searched (classification system followed by classification symbols)

    A61M5/20; A61M5/24; A61M5/28

Documentation searched other than minimum documentation to the extent that such documents are included in the fields searched

    Published examined utility model applications of Japan 1922-1996
    Published unexamined utility model applications of Japan 1971-2022
    Registered utility model specifications of Japan 1996-2022
    Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2015-514524 A (SANOFI-AVENTIS DEUTSCHLAND GMBH) 21 May 2015 (2015-05-21) paragraphs [0014], [0025]-[0031], fig. 1 | 1, 3 |
| Y | | 2, 8-29, 33 |
| A | | 4-7, 30-32, 34-35 |
| Y | JP 2020-506760 A (SANOFI-AVENTIS DEUTSCHLAND GMBH) 05 March 2020 (2020-03-05) paragraphs [0015]-[0053], fig. 1A-1D | 1-3, 8-29, 33 |
| A | | 4-7, 30-32, 34-35 |
| Y | WO 03/024385 A1 (TERUMO CORP.) 27 March 2003 (2003-03-27) p. 9, lines 18-26, p. 18, lines 9, 10 | 1-3, 33 |
| Y | JP 2017-531459 A (UNITRACT SYRINGE PROPRIETARY LTD.) 26 October 2017 (2017-10-26) paragraphs [0061], [0064], [0067]-[0071], [0107]-[0215], fig. 1-10 | 1-3, 8-29, 33 |
| A | | 4-7, 30-32, 34-35 |

☑ Further documents are listed in the continuation of Box C.　　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **17 January 2022** | **01 February 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
|---|
| **PCT/JP2021/043006** |

### C.    DOCUMENTS CONSIDERED TO BE RELEVANT

| Category* | Citation of document, with indication, where appropriate, of the relevant passages | Relevant to claim No. |
|---|---|---|
| Y | JP 2016-534788 A (SANOFI-AVENTIS DEUTSCHLAND GMBH) 10 November 2016 (2016-11-10)<br>    paragraph [0028] | 9-29, 33 |
| Y | WO 2020/072299 A1 (ELI LILLY AND COMPANY) 09 April 2020 (2020-04-09)<br>    paragraphs [0094], [0114]-[0116], [0131], fig. 9A-9B, 21A-22B | 20-21 |
| Y | JP 2014-533539 A (SANOFI-AVENTIS DEUTSCHLAND GMBH) 15 December 2014 (2014-12-15)<br>    paragraphs [0020]-[0025], [0086] | 20-21 |
| Y | JP 2020-525100 A (ASCENDIS PHARMA A/S) 27 August 2020 (2020-08-27)<br>    paragraphs [0072]-[0077] | 22, 27 |
| Y | JP 9-154944 A (TERUMO CORP.) 17 June 1997 (1997-06-17)<br>    paragraph [0034] | 23-26 |
| Y | JP 6-227062 A (SEIKO EPSON CORP.) 16 August 1994 (1994-08-16)<br>    paragraph [0020], fig. 2 | 23-26 |
| Y | JP 10-198972 A (NIPPON COLUMBIA CO., LTD.) 31 July 1998 (1998-07-31)<br>    paragraph [0036], fig. 2 | 23-26 |
| A | JP 2019-98013 A (PHC HOLDINGS CORP.) 24 June 2019 (2019-06-24)<br>    paragraphs [0037]-[0070], fig. 4-10 | 1-34 |
| A | WO 2017/110590 A1 (PANASONIC HEALTHCARE HOLDINGS CO., LTD.) 29 June 2017 (2017-06-29)<br>    entire text, all drawings | 1-34 |

Form PCT/ISA/210 (second sheet) (January 2015)

**INTERNATIONAL SEARCH REPORT**
Information on patent family members

| International application No. |
| --- |
| **PCT/JP2021/043006** |

| Patent document cited in search report | | | Publication date (day/month/year) | Patent family member(s) | Publication date (day/month/year) |
| --- | --- | --- | --- | --- | --- |
| JP | 2015-514524 | A | 21 May 2015 | US 2015/0080810 A1 paragraphs [0015], [0028]-[0034], fig. 1<br>WO 2013/160152 A1<br>EP 2656865 A1<br>CN 104379195 A<br>KR 10-2015-0010730 A | |
| JP | 2020-506760 | A | 05 March 2020 | US 2019/0388619 A1 paragraphs [0019]-[0058], fig. 1A-1D<br>WO 2018/138192 A1<br>CN 110430909 A | |
| WO | 03/024385 | A1 | 27 March 2003 | US 2005/0029277 A1 paragraphs [0048], [0078]<br>EP 1433456 A1 | |
| JP | 2017-531459 | A | 26 October 2017 | US 2018/0236181 A1 paragraphs [0075], [0078], [0081]-[0085], [0120]-[0228], fig. 1-10<br>WO 2016/033507 A2<br>EP 3733228 A1<br>CN 107073216 A | |
| JP | 2016-534788 | A | 10 November 2016 | US 2016/0263331 A1 paragraph [0028]<br>WO 2015/063193 A1<br>CN 105873627 A | |
| WO | 2020/072299 | A1 | 09 April 2020 | JP 2021-521899 A paragraphs [0052], [0073]-[0075], [0090], fig. 9A-9B, 21A-22B<br>US 2021/0093784 A1<br>CN 112867521 A | |
| JP | 2014-533539 | A | 15 December 2014 | US 2014/0330243 A1 paragraphs [0028]-[0032], [0156]<br>WO 2013/076026 A1<br>CN 104093438 A | |
| JP | 2020-525100 | A | 27 August 2020 | WO 2019/002534 A1 p. 16, line 15 to p. 17, line 34<br>CN 110809484 A<br>KR 10-2020-0023363 A | |
| JP | 9-154944 | A | 17 June 1997 | (Family: none) | |
| JP | 6-227062 | A | 16 August 1994 | (Family: none) | |
| JP | 10-198972 | A | 31 July 1998 | (Family: none) | |
| JP | 2019-98013 | A | 24 June 2019 | (Family: none) | |
| WO | 2017/110590 | A1 | 29 June 2017 | US 2018/0339107 A1 entire text, all drawings<br>US 2019/0091406 A1<br>US 2021/0085875 A1<br>EP 3395385 A1 | |

Form PCT/ISA/210 (patent family annex) (January 2015)

**REFERENCES CITED IN THE DESCRIPTION**

**Patent documents cited in the description**

- JP 2014516634 A